(19) Europäisches Patentamt
European Patent Office
Office européen des brevets

(11) Publication number : **0 265 519 B1**

(12) # EUROPEAN PATENT SPECIFICATION

(45) Date of publication of patent specification : **13.09.95 Bulletin 95/37**

(51) Int. Cl.$^6$ : **G01N 33/532,** G01N 33/569, G01N 33/76

(21) Application number : **87904151.5**

(22) Date of filing : **30.04.87**

(86) International application number : **PCT/US87/00987**

(87) International publication number : **WO 87/06706 05.11.87 Gazette 87/24**

(54) **ELECTROCHEMILUMINESCENT ASSAYS.**

Divisional application 94120516.3 filed on 30/04/87.

(30) Priority : **30.04.86 US 858354**

(43) Date of publication of application : **04.05.88 Bulletin 88/18**

(45) Publication of the grant of the patent : **13.09.95 Bulletin 95/37**

(84) Designated Contracting States : **AT BE CH DE FR GB IT LI LU NL SE**

(56) References cited :
**EP-A- 0 178 450**
**WO-A-81/01883**
**WO-A-86/02734**
**US-A- 4 205 952**
**US-A- 4 233 144**
**US-A- 4 280 815**
**US-A- 4 293 310**
**CLINICAL CHEMISTRY, Diagnosis & Treatment, Zilva & Parnell (ed.), 3rd Ed., Lloyd Luke Ltd. (publ.); pp. 472-473/**

(73) Proprietor : **IGEN, INC. (a California corporation)**
**16020 Industrial Drive**
**Gaithersburg Maryland 20877 (US)**

(72) Inventor : **MASSEY, Richard, J.**
**5 Valerian Court**
**Rockville, MD 20852 (US)**
Inventor : **POWELL, Michael, J.**
**5 War Admiral Way**
**Gaithersburg, MD 20878 (US)**
Inventor : **MIED, Paul, A.**
**3713 Buffalo Road**
**New Windsor, MD 21776 (US)**
Inventor : **FENG, Peter**
**245 Rollins Avenue**
**Rockville, MD 20852 (US)**
Inventor : **DELLA CIANA, Leopoldo**
**5511 Halpine Place**
**Rockville, MD 20851 (US)**
Inventor : **DRESSICK, Walter, J.**
**12905 Crookston Lane**
**Rockville, MD 20851 (US)**
Inventor : **POONIAN, Mohindar, S.**
**224 High Timber Court**
**Gaithersburg, MD 20879 (US)**

(74) Representative : **Cropp, John Anthony David et al**
**MATHYS & SQUIRE**
**100 Grays Inn Road**
**London, WC1X 8AL (GB)**

EP 0 265 519 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the detection of an analyte of interest in a sample by means of electrochemiluminescence.

Within this application several publications are referenced by Arabic numerals within parentheses. Full citations for these references may be found at the end of the specification immediately preceding the claims. The disclosures of these publications in their entirety are hereby incorporated by reference into this application in order to more fully describe the state of the art to which this invention pertains.

There is a continuous and expanding need for rapid, highly specific methods of detecting and quantifying chemical, biochemical and biological substances. Of particular value are methods for measuring small quantities of pharmaceuticals, metabolites, microorganisms and other materials of diagnostic value. Examples of such materials include narcotics and poisons, drugs administered for therapeutic purposes, hormones, pathogenic microorganisms and viruses, antibodies, metabolites, enzymes and nucleic acids.

The presence of these materials can often be determined by binding methods which exploit the high degree of specificity which characterizes many biochemical and biological systems. Frequently used methods are based on, for example, antigen-antibody systems, nucleic acid hybridization techniques, and protein-ligand systems. In these methods, the existence of a complex of diagnostic value is typically indicated by the presence or absence of an observable "label" which has been attached to one or more of the complexing materials.

The specific labeling method chosen often dictates the usefulness and versatility of a particular system for detecting a matarial of interest. A preferred label should be inexpensive, safe, and capable of being attached efficiently to a wide variety of chemical, biochemical, and biological materials without changing the important binding characteristics of those materials. The label should give a highly characteristic signal, and should be rarely, and preferably never, found in nature. The label should be stable and detectable in aqueous systems over periods of time ranging up to months. Detection of the label should be rapid, sensitive, and reproducible without the need for expensive, specialized facilities or personnel. Quantification of the label should be relatively independent of variables such as temperature and the composition of the mixture to be assayed. Most advantageous are labels which can be used in homogeneous systems, i.e., systems in which separation of the complexed and uncomplexed labeled material is not necessary. This is possible if the detectability of the label is modulated when the labeled material is incorporated into a specific complex.

A wide variety of labels have been developed, each with particular advantages and disadvantages. For example, radioactive labels are quite versatile, and can be detected at very low concentrations. However, they are expensive, hazardous, and their use requires sophisticated equipment and trained personnel. Furthermore, the sensitivity of radioactive labels is limited by the fact that the detectable event can, in its essential nature, occur only once per radioactive atom in the labeled material. Moreover, radioactive labels cannot be used in homogeneous methods.

Thus, there is wide interest in non-radioactive labels. These include molecules observable by spectrophotometric, spin resonance, and luminescence techniques, as well as enzymes which produce such molecules. Among the useful non-radioactive labeling materials are organometallic compounds. Because of the rarity of some metals in biological systems, methods which specifically assay the metal component of the organometallic compounds can be successfully exploited. For example, Cais, U.S. Patent No. 4,205,952 (1980) discloses the use of immunochemically active materials labeled with certain organometallic compounds for use in quantitating specific antigens. Any general method of detecting the chosen metals can be used with these labels, including emission, absorption and fluorescence spectroscopy, atomic absorption, and neutron activation. These methods often suffer from lack of sensitivity, can seldom be adapted to a homogeneous system, and as with atomic absorption, sometimes entail destruction of the sample.

Of particular interest are labels which can be made to luminesce through photochemical, chemical, and electrochemical means. "Photoluminescence" is the process whereby a material is induced to luminesce when it absorbs electromagnetic radiation. Fluorescence and phosphorescence are types of photoluminescence. "Chemiluminescent" processes entail the creation of the luminescent species by a chemical transfer of energy. "Electrochemiluminescence" entails the creation of the luminescent species electrochemically.

These luminescent systems are of increasing importance. For example, Mandle, U.S. Patent No. 4,372,745 (1983) discloses the use of chemiluminescent labels in immunochemical applications. In the disclosed systems, the labels are excited into a luminescent state by chemical means such as by reaction of the label with $H_2O_2$ and an oxalate. In these systems, $H_2O_2$ oxidatively converts the oxalate into a high energy derivative, which then excites the label. This system will, in principle, work with any luminescent material that is stable in the oxidizing conditions of the assay and can be excited by the high energy oxalate derivative. Unfortunately, this very versatility is the source of a major limitation of the technique: typical biological fluids containing the analyte of interest also contain a large number of potentially luminescent substances that can cause high background levels of luminescence.

Another example of the immunochemical use of chemiluminescence which suffers from the same disadvantages is Oberhardt et al., U.S. Patent No. 4,280,815, (1981) who disclose the in situ electrochemical generation of an oxidant (e.g., $H_2O_2$) in close proximity to an immunoreactant labeled with a chemiluminescent species. The electrogenerated oxidant diffuses to the chemiluminescent species and chemically oxidizes it, resulting in the net transfer of one or more electrons to the electrogenerated oxidant. Upon oxidation, the chemiluminescent species emits a photon. In contrast, the subject invention requires the direct transfer of electrons from a source of electrochemical energy to a chemiluminescent species which is capable of repeatedly emitting photons.

Neither US-A-4372745 nor US-A-4280815 employs electrochemiluminescence.

The present invention is concerned with electrochemiluminescent labels. Suitable labels comprise electrochemiluminescent organometallic compounds. Electrochemiluminescent methods of determining the presence of labeled materials are preferred over other methods for many reasons. They are highly diagnostic of the presence of a particular label, sensitive, nonhazardous, inexpensive, and can be used in a wide variety of applications. Many organometallic compounds are suitable electrochemical labels, but of particular use are Ru-containing and Os-containing compounds.

Thus, in one embodiment, the present invention is concerned with the use of Ru-containing and Os-containing labels which can be detected by a wide variety of methods. These labels are advantageous for many reasons that will be discussed herein.

Ru-containing and Os-containing organometallic compounds have been discussed in the literature. Cais discloses that any metal element or combination of metal elements, including noble metals from group VIII such as Ru, would be suitable components of organmetallic labels detectable by atomic absorption methods. (Cais, column 11, line 20). However, ruthenium is not a preferred metal in Cais, osmium is not specifically mentioned, no data are presented on the efficiency of using Ru or Os in any of the methods disclosed, and the preferred method of detection, atomic absorption, entails destruction of the sample.

Weber, U.S. Patent No. 4,293,310 (1981), discloses the use of Ru-containing and Os-containing complexes as electrochemical labels for analytes in immunoassays. The disclosed complexes are linked to amino groups on the analytes through a thiourea linkage. Weber also suggests the possibility of forming carboxylate esters between the labels and hydroxy groups on other analytes.

According to Weber, the presence of the labeled materials can be determined with an apparatus and method which comprises a quencher and an electrochemical flow cell with light means. The photoelectrochemically active label upon photoexcitation transfers an electron to a quencher molecule; the oxidized molecule is subsequently reduced with an electron from an electrode of the flow cell which is held at suitable potential. This electron is measured as photocurrent. The amount of free labelled analyte in the system is determined by the photocurrent signal. Note that this method is the reverse of electrochemiluminescent detection of luminescent materials.

In subsequent reports, Weber et al. discussed the problems associated with the use of this method to detect Ru-containing labels (1). In Table 2 of Weber et al. (1), the extrapolated detection limit for tris(bipyridyl)ruthenium(II) is $1.1 \times 10^{-10}$ moles/L under optimal conditions. In anticipating that the actual use of these labels would entail measurements in the presence of complex mixtures, Weber et al. tested for potential interferents in their system. Table 3 of Weber et al. lists dimethylalkyl amines, EDTA, N-methylmorpholine, N,N′(-dimethylpiperazine, hydroxide, oxalate, ascorbate, uric acid, and serum as interferents which would presumably raise the practical detection limit substantially above $1.1 \times 10^{-10}$ moles/L.

These studies were performed with a simple Ru-containing compound. No studies were reported in Weber or Weber et al. regarding the limits of detection of complex substances labelled with Ru-containing labels, or whether the thiourea linkage between the labeled material and label is stable under conditions of the assay.

The particular labels with which the present invention is concerned are electrochemiluminescent. They can often be excited to a luminescent state without their oxidation or reduction by exposing the compounds to electromagnetic radiation or to a chemical energy source such as that created by typical oxalate-$H_2O_2$ systems. In addition, luminescence of these compounds can be induced by electrochemical methods which do entail their oxidation and reduction.

Extensive work has been reported on methods for detecting $Ru(2,2'\text{-bipyridine})_3^{2+}$ using photoluminescent, chemiluminescent, and electrochemiluminescent means (2, 3). This work demonstrates that bright orange chemiluminescence can be based on the aqueous reaction of chemically generated or electrogenerated $Ru(bpy)_3^{3+}$ (where "bpy" represents a bipyridyl ligand) with strong reductants produced as intermediates in the oxidation of oxalate ions or other organic acids. Luminescence also can be achieved in organic solvent $H_2O$ solutions by the reaction of electrogenerated, or chemically generated, $Ru(bpy)_3^{1+}$ with strong oxidants generated during reduction of peroxydisulfate. A third mechanism for production of electrochemiluminescence from

$Ru(bpy)_3^{2+}$ involves the oscillation of an electrode potential between a potential sufficiently negative to produce $Ru(bpy)_3^{1+}$ and sufficiently positive to produce $Ru(bpy)_3^{3+}$. These three methods are called, respectively, "oxidative-reduction," "reductive-oxidation," and "the $Ru(bpy)_3^{3+/+}$ regenerative system".

The oxidative-reduction method can be performed in water, and produces an intense, efficient, stable luminescence, which is relatively insensitive to the presence of oxygen or impurities. This luminescence from $Ru(bpy)_3^{2+}$ depends upon the presence of oxalate or other organic acids such as pyruvate, lactate, malonate, tartrate and citrate, and means of oxidatively producing $Ru(bpy)_3^{3+}$ species. This oxidation can be performed chemically by performed strong oxidants as $PbO_2$ or a Ce(IV) salt. It can be performed electrochemically by a sufficiently positive potential applied either continuously or intermittently. Suitable electrodes for the electrochemical oxidation of $Ru(bpy)_3^{2+}$ are, for example, Pt, pyrolytic graphite, and glassy carbon. Although the oxalate or other organic acid is consumed during chemiluminescence, a strong, constant chemiluminescence for many hours can be achieved by the presence of an excess of the consumed material, or by a continuous supply of the consumed material to the reaction chamber.

The reductive-oxidation method can be performed in partially aqueous solutions containing an organic cosolvent such as, for example, acetonitrile. This luminescence depends upon the presence of peroxydisulfate and a means of reductively presence $Ru(bpy)_3^{1+}$ species. The reduction can be performed chemically by strong reductants such as, for example, magnesium or other metals. It can be performed electrochemically by a sufficiently negative potential applied either continuously or intermittently. A suitable electrode for the electrochemical reduction of $Ru(bpy)_3^{2+}$ is, for example, a polished glassy-carbon electrode. As with the oxidative-reduction method, continuous, intense luminescence can by achieved for many hours by inclusion of excess reagents, or by continous addition of the consumed reagents to the reaction mixture.

The $Ru(bpy)_3^{3+/+}$ regenerative system can be performed in organic solvents such as acetonitrile or in partially aqueous systems, by pulsing an electrode potential between a potential sufficiently negative to reduce $Ru(bpy)_3^{2+}$ and a potential sufficiently positive to oxidize $Ru(bpy)_3^{2+}$. A suitable electrode for such a regenerative system is, for example, a Pt electrode. This system does not consume chemical reagents and can proceed, in principle, for an unlimited duration.

These three methods of producing luminescent Ru-containing compounds have in common the repetitive oxidation-reduction or reduction-oxidation of the Ru-containing compound. The luminescence of solutions containing these compounds is therefore highly dependent on the electric potential of the applied energy source, and is therefore highly diagnostic of the presence of the Ru-containing compound.

Mandle cites Curtis et al. (4) as a possible label in chemiluminescent applications. Curtis et al. reports only unpublished observations that Ru complexes can be induced to emit light when chemically excited by an oxalate/$H_2O_2$ system (Curtis et al. p. 350).

Neither Mandle nor Curtis recognized the exceptional utility of ruthenium and osmium complexes in chemiluminescent applications or the utility of electrochemiluminescent systems. Sprintschnik, G. et al. (5) have described complexes of tris (2,2′-bipyridine)ruthenium(II) esterified with octadecanol or dehydrocholesterol, and have created monolayer films of these surfactant complexes. The complexes were photoluminescent. But when the films were exposed to water, and then to light, the Ru-complexes failed to photoluminesce. This was attributed to photohydrolysis of ester groups in the presence of light.

It has been discovered, and is disclosed herein, that a wide variety of analytes of interest and chemical moieties that bind to analytes of interest may be conveniently attached to Ru-containing or Os-containing labels through amide or amine linkages. The labeled materials may then be determined by any of a wide variety of means, but by far the most efficient, reliable, and sensitive means are photoluminescent, chemiluminescent, and electrochemiluminescent means. It is also disclosed herein that electrochemiluminescent labels, including Ru-containing and Os-containing labels and organic molecules such as rubrene and 9,10-diphenyl anthracene, are particularly versatile and advantageous. The great advantages of the use of these novel labeled materials, and of the methods of detecting them, are further discussed hereinbelow.

For many years the food industry has been concerned with the presence of biological and chemical contaminants in raw food components and processed foods. While technological advances have been made in reducing the occurrence of food contamination and food borne disease outbreaks resulting therefrom, little progress has been reported in developing rapid and sensitive methods for the detection and identification of food contaminants. Existing standard methods for the detection of harmful contaminants in foods are generally very time consuming, labor intensive, and technically difficult. While the analytical methods themselves for the most part are of adequate sensitivity, the lengthy sample preparation procedures prior to the performance of the detection method often result in low yield of the contaminant in question so that false negatives are frequently encountered.

Two examples which serve to illustrate these problems are the currently recognized standard methods for detecting the presence of Salmonella and Staphylococcal enterotoxins in foods. The detection of Salmonella

in foods involves several enrichment stages due to the fact that these bacteria, when present in foods, are usually found in low numbers and are often sublethally injured. Therefore, detection methods for Salmonella must be sensitive and allow for the resuscitation and growth of injured cells.

Two methods for Salmonella detection are currently recommended by the U.S. Food and Drug Administration. These methods appear in The Bacteriological Analytical Manual for Foods (1984), 6th ed., Association of Official Analytical Chemists, Washington, DC. One method is a pure culture technique involving preenrichment, selective enrichment and selective plating, a procedure which requires 4 days to obtain presumptive results and 5 to 7 days to obtain complete results. The other method involves immunofluorescence after selective enrichment. This procedure is more rapid, however it can result in a high incidence of false-positive results due to problems of cross-reactivity of the polyvalent antisera used in the test (6, 7).

The procedure recommended by the U.S. Food and Drug Administration for the detection of Staphylococcal enterotoxins in foods also appears in The Bacteriological Analytical Manual for Foods (1984), 6th ed. Association of Official Analytical Chemists, Washington, DC. This method involves the concentration of an extract of a large food sample, e.g. approximately 100 grams, to a small volume, e.g. approximately 0.2 ml,by several dialysis concentration steps and an ion exchange column purification of the sample extract in order to prepare the sample for the microslide double-immunodiffusion technique. This procedure generally requires more than a week to perform.

Tests which are more rapid have recently been developed for the detection of a variety of contaminants such as bacteria, toxins, antibiotics and pesticide residues in foods. In many cases however, sample preparation prior to running the assay continues to be laborious and time consuming. Radioimmunoassays (RIA) and enzyme-linked immunosorbent assays (ELISA) have shortened the hands-on time for the analytical method itself, however these methods are still labor intensive and far from simple to perform. In addition, these methods are usually based on the use of polyclonal antisera, which is variable in specifity and sensitivity, and is generally in short supply for testing for a given food contaminant. ELISA methods have been developed for the analysis of food samples which employ monoclonal antibodies rather than polyclonal antisera. The use of monoclonal antibodies in an assay system for a food contaminant assures the constant supply of a reagent which imparts unchanging specificity and sensitivity to the test itself. Monoclonal antibodies have been used in ELISA systems to test for food contaminants such as Salmonella (8) and Staphylococcal enterotoxins (9). Commercially available products for Salmonella detection which employ EIA methodology (Bio-Enzabead Screen Kit, Litton Bionetics) and DNA probe technology (Gene-Trak, Integrated Genetics) are time consuming and labor intensive. Commercially available tests for detection of Staphylococcal enterotoxin in foods which employ reversed passive latex agglutination (SET-RPLA, Denka Seiken Co.) and EIA methodology (SET-EIA, Dr. W. Bommeli Laboratories) suffer from the same limitations.

For the past 100 years the bacterium *Escherichia coli* and the coliform group have been commonly used as indicators to monitor water quality and incidences of sewage contamination.

Current detection methodologies for *E. coli* and/or coliforms are based on the properties of acid or gas production from the fermentation of lactose. The most widely used methods are: the Most Probable Number (MPN) assay and the Membrane Filtration (MF) test. Both techniques are approved by the Environmental Protection Agency (EPA) and the American Public Health Association (APHA) for the microbiological examination of water and waste water (10), and also by the Food and Drug Administration (FDA) for the bacteriological examination of milk and foods (11).

The MPN method is actually comprised of three (12) separate assays (10). In the Presumptive test, a nonselective medium such as Lauryl Sulfate Tryptose (LST) broth or Lactose broth is used to check for gas production from the fermentation of lactose. Gas positive tubes are then subcultured into a more selective medium, Brillant Green Lactose Bile (BGLB) broth for coliforms and *E. coli* (EC) broth for fecal coliforms, and again checked for gas production (confirmed test). Samples from positive Confirmatory tests are required to be tested further by plating on a selective and differential medium like Eosin Methylene Blue (EMB) agar or Endos agar, followed by Gram stain and some biochemical tests to firmly establish the presence of the indicator bacteria (Completed test). The entire MPN assay may require up to five (5) days for completion; therefore, for routine water analysis, most laboratories use only the Presumptive and the Confirmed portions of the MPN assay, which still requires 48 hours to 72 hours to complete. In addition to being time consuming and cost ineffective in terms of materials, incidences of both false positive and false negative reactions have also been commonly encountered in the MPN assays (15, 16, 20).

The MF technique for the bacteriological examination of water was introduced in the early 1950's (12). Unlike the MPN assay, which was tedious and time consuming, MF analysis could be completed in 24 hours without the need for further confirmations. The basic MF procedure is as follows: A volume of water sample, usually 100 ml is filtered through a 0.45 um pore diameter filter, and then incubated on a sterile pad saturated with selective medium. The two media most often used are the mEndo broth, selective for coliforms at 35°C,

and the mFC broth, selective for fecal coliforms at 44.5°C (10). Since the introduction of the media, numerous authors have reported that both the mEndo and the mFC broths tend to underestimate the actual numbers of indicator bacteria present, due either to the selectivity of the medium or the high temperature used for incubation (44.5°C) (21, 22) . Such incidences of false negatives have been especially prevalent when the organisms in the sample have been sublethally injured by environmental factors and/or chemicals (17, 18). Recently, modifications have been proposed by the EPA to follow up the MF test by a confirmatory procedure, whereby at least ten colonies on each filter need to be checked for gas production using the LST broth followed by BGLB broth as in the MPN assay (14). Such modifications although would reduce the incidences of both false negative and false positive reactions, it would also increase material cost as well as triple the MF assay time from 24 hours to 72 hours.

In 1982, Feng and Hartman introduced a fluorogenic assay for the detection of E. coli using the substrate 4-methyl umbelliferone glucuronide (MUG) (13). E. coli cells produced the enzyme beta-glucuronidase which would cleave the substrate releasing the fluorogenic 4-methylumbelliferone radical (19). By incorporating the compound MUG into the Presumptive LST medium, a single tube of LST-MUG medium provided both the Presumptive data (gas production) and the Confirmed data (fluorescence) for fecal coliforms within 24 hours. Although the MUG assay was rapid and simple, only 85% to 95% of the E. coli (depending on source) produced this enzyme, hence the test was not 100% reliable. Also the system was not applicable to the coliform group.

Currently, no suitable assay exists for the detection and enumeration of coliforms and fecal coliforms in a sample. The development of a simple, rapid, and reliable detection assay would not only decrease cost and time, but also greatly increase the efficiency of monitoring water sanitation and food processing and handling.

WO 81/01883 was published after the priority date of the present patent application but has an earlier priority date. This publication is directed to a method of detecting an analyte of interest in a sample by contacting the sample with a reagent comprising a metal-containing organic compound conjugate under conditions such that the analyte and reagent bind, or the analyte and reagent each combine with a complementary material in a competitive format, and then inducing the conjugate to emit electromagnetic radiation. However, it does not disclose the use of electrochemiluminescence to detect an analyte of interest at a concentration below $10^{-3}$ molar.

According to the present invention, there is provided a method for detecting in a predetermined volume of a multicomponent, liquid sample the presence or absence of an analyte of interest which, if present in the sample, is at a concentration below $10^{-3}$ molar, said method comprising:

a. contacting the sample with a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of combining with the analyte of interest, the contact being effected under conditions such that the analyte and conjugate bind;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. detecting emitted luminescence to determine whether the analyte of interest is present in the sample.

The invention also provides a method for quantitatively determining in a predetermined volume of a multicomponent, liquid sample the amount present in the sample of an analyte of interest which, if present in the sample, is at a concentration which is below $10^{-3}$ molar, said method comprising:

a. contacting the sample with a known amount of a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of combining with the analyte of interest, the contact being effected under conditions such that the analyte and conjugate bind;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. quantitatively determining the amount of emitted luminescence to determine whether and in what amount the analyte of interest is present in the sample.

The invention further provides a method for detecting in a predetermined volume of a multicomponent, liquid sample the presence or absence of an analyte of interest which, if present in the sample, is at a concentration below $10^{-3}$ molar, said method comprising:

a. contacting the sample with a complementary material not normally present in the sample and with a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of competing with the analyte of interest for binding sites on the complementary material not normally present in the sample, the contact being effected under appropriate conditions such that the analyte of interest and the conjugate competitively bind to the complementary material;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. detecting emitted luminescence to determine whether the analyte of interest is present in the sample.

Still further, there is provided a method for quantitatively determining in a predetermined volume of a multicomponent, liquid sample the amount present in the sample of an analyte of interest which, if present in the sample, is at a concentration which is below $10^{-3}$ molar, said method comprising:

a. contacting the sample with a known amount of a complementary material and a known amount of reagent (i) comprising a metal-containing organic compound conjugated to a whole cell, subcellular particle, virus, prion, viroid, lipid, fatty acid, nucleic acid, polysaccharide other than a sugar protein, lipoprotein, lipopolysaccharide, glycoprotein, peptide, cellular metabolite, hormone, pharamcological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of competing with the analyte of interest for binding sites on the complementary material not normally present in the sample, the contact being effected under conditions such that the analyte of interest and conjugate competitively bind to the complementary material;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. quantitatively determining the amount of emitted luminescence to determine whether and in what amount the analyte of interest is present in the sample.

The invention will now be described in greater detail with reference to preferred embodiment and with the aid of the accompanying drawings in which

Figure 1 depicts electrochemiluminescent measurements made for a homogeneous immunoassay for the determination of the concentration of an antigen in solution.

Figure 2 graphically depicts the results of a homogeneous ECL theophylline assay.

Figure 3 graphically depicts the results of a homogeneous theophylline assay in various sera.

● = Normal sera
■ = Hemolyzed sera
♦ = Lipemic sera
□ = Icteric sera

Figure 4 graphically depicts the results of an ECL theophylline assay compared to the results of a fluorescence polarization theophylline assay.

A. Normal sera: n = 4; slope = .986; r = 1.00.
B. Hemolyzed sera: n = 3; slope = .878; r = 1.00
C. Lipemic sera: n = 5; slope = .872; r = 0.99
D. Icteric sera: n = 4; slope = 2.14; r = 1.00

Figure 5 graphically depicts the results of an ECL theophylline assay compared to the results from a high pressure liquid chromatography assay.

n = 9; slope = 1.197; r = 0.98.

Figure 6 graphically depicts the modulation of an ECL signal generated in an ECL digoxin immunoassay.
Figure 7 graphically depicts the results of an ECL digoxin immunoassay.
■ = Blank
● = Digoxin
Figure 8 graphically depicts the ECL signal generated by various concentrations of MBI 38-Compound I.
Figure 9 shows the results of a Hybridization/Sensitivity Study of MBI 38-Compound I.
TAG = Compound I
Figure 10 shows the results of a Specificity Study of MBI 38-Compound I.

## DETAILED DESCRIPTION OF THE INVENTION

The present invention provides method for detecting in a predetermined volume of a multicomponent, liquid sample the presence or absence of an analyte of interest which, if present in the sample, is at a concentration below $10^{-3}$ molar, said method comprising:

a. contacting the sample with a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of combining with the analyte of interest, the contact being effected under conditions such that the analyte and conjugate bind;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. detecting emitted luminescence to determine whether the analyte of interest is present in the sample.

Within this application "molar" means the concentration of an analyte in solution in moles per liter or the amount of particulate matter present in a liquid sample in particles or units per liter. For example, 1 x $10^{23}$ particles per liter may be expressed as 1 molar.

The methods provided by the present invention may be performed as heterogeneous assays, i.e, assays in which unbound labeled reagent is separated from bound labeled reagent prior to exposure of the bound labeled reagent to electrochemical energy, and homogeneous assays, i.e. assays in which unbound labeled reagent and bound labeled reagent are exposed to electrochemical energy together. In the homogeneous assays of the present invention the electromagnetic radiation emitted by the bound labeled reagent is distinguishable from the electromagnetic radiation emitted by the unbound labeled reagent, either as an increase or as a decrease in the amount of electromagnetic radiation emitted by the bound labeled reagent in comparison to the unbound labeled reagent, or as electromagnetic radiation of a different wavelength. Accordingly, in one embodiment of the invention any reagent which is not combined with the analyte of interest is separated from the sample, which had been contacted with the reagent, prior to exposure of the sample to electrochemical energy. In another embodiment of the invention, prior to contacting the sample with the reagent, the sample is treated so as to immobilize the analyte of interest. Means for immobilizing analytes of interest are well known within the art and include contacting the sample with a polystyrene, nitrocellulose or nylon surface, or a surface coated with whole cells, subcellular particles, viruses, prions, viroids, lipids, fatty acids, nucleic acids, polysaccharides, proteins, lipoproteins, lipopolysaccharides, glycoproteins, peptides, cellular metabolites, hormones, pharmacological agents, tranquilizers, barbiturates, alkaloids, steroids, vitamins, amino acids, sugars, nonbiological polymers, synthetic organic molecules, organometallic molecules or inorganic molecules. Additionally, the analyte of interest may be any of these substances. In one embodiment of the invention, the analyte of interest is theophylline. In another embodiment of the invention, the analyte of interest is digoxin. In still another embodiment of the invention, the analyte of interest is human chorionic gonadotropin (hCG). Furthermore, the analyte of interest may be a whole cell, subcellular particle, virus, prion, viroid, nucleic acid, protein, lipoprotein, lipopolysaccharide, glycoprotein, peptide, hormone, pharmacological agent, nonbiological polymer, synthetic organic molecule, organometallic molecule or an inorganic molecule present in the sample at a concentration below about $10^{-12}$ molar. Moreover the analyte of interest may be a whole cell, subcellular particle, virus, prion, viroid or nucleic acid present in the sample at a concentration below about $10^{-15}$ molar.

The reagent which is contacted with the sample may comprise an electrochemiluminescent metal-containing organic compound conjugated to a whole cell, subcellular particle, virus, prion, viroid, lipid, fatty acid, nucleic acid, polysaccharide, protein, lipoprotein, lipopolysaccharide, glycoprotein, peptide, cellular metabolite,

hormone, pharmacological agent, tranquilizer, barbiturate, alkaloid, steroid, vitamin, amino acid, sugar, nonbiological polymer, synthetic organic molecule, organometallic molecule, inorganic molecule, biotin, avidin or streptavicin. In one embodiment or the invention the agent is an electrochemiluminescent metal-containing organic compound conjugated to an antibody, antigen, nucleic acid, hapten, ligand or enzyme, or biotin avidin or streptavidin.

The metal-containing organic compound may be one wherein the metal is selected from the group consisting of ruthenium, osmium, rhenium, iridium, rhodium, platinum, palladium, molybdenum and technetium. In one embodiment of the invention the metal is ruthenium or osmium.

In still another embodiment of the invention, the electrochemiluminescent chemical moiety is bis[(4,4′-carbomethoxy)-2,2′-bipyridine] 2-[3-(4-methyl-2,2′-bipyridine-4-yl)propyl]-1,3-dioxolane ruthenium (II). In yet another embodiment of the invention, the electrochemiluminescent chemical moiety is bis (2,2′bipyridine) [4-(butan-1-al)-4′-methyl-2,2′-bipyridine] ruthenium (II). In a further embodiment of the invention, the electrochemiluminescent chemical moiety is bis (2,2′-bipyridine) [4-(4′methyl-2,2′-bipyridine-4′-yl)-butyric acid] ruthenium (II). In still another embodiment of the invention, the electrochemiluminescent chemical moiety is (2,2′-bipyridine)[cis-bis(1,2-diphenylphosphino)ethylene]{2-[3-(4-methyl-2,2′-bipyridine-4′-yl)propyl]-1,3-dioxolane}osmium (II). In yet a further embodiment of the invention, the electrochemiluminescent chemical moiety is bis(2,2′-bipyridine) [4-(4′-methyl-2,2′bipyridine)-butylamine] ruthenium (II) . In yet another embodiment of the invention the electrochemiluminescent chemical moiety is bis(2,2′-bipyridine) [1-bromo-4(4′-methyl-2,2′-bipyridine-4-yl)butane] ruthenium (II). In still a further embodiment of the invention, the electrochemiluminescent chemical moiety is bis(2,2′-bipyridine)maleimidohexanoic acid, 4-methyl-2,2′-bipyridine-4′-butylamide ruthenium (II). In another embodiment of the invention, the electrochemiluminescent moiety is bis(2,2′-bipyridine) maleimidohexanoic acid, 4-methyl-2,2′-bipyridine-4′-butylamide ruthenium (II) diperchlorate.

The sample may be derived from a solid, emulsion, suspension, liquid or gas. Furthermore, the sample may be derived from water, food, blood, serum, urine, feces, tissue, saliva, oils, organic solvents or air. Moreover, the sample may comprise dimethylsulfoxide, dimethylformamide, n-methyl-pyrrolidinone or tert-butyl alcohol. The sample may comprise a reducing agent or an oxidizing agent.

The present invention also provides a competitive method for detecting in a predetermined volume of a multicomponent, liquid sample the presence or absence of an analyte of interest which, if present in the sample, is at a concentration below $10^{-3}$ molar, said method comprising:

a. contacting the sample with a complementary material not normally present in the sample and with a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of competing with the analyte of interest for binding sites on the complementary material not normally present in the sample, the contact being effected under appropriate conditions such that the analyte of interest and the conjugate competitively bind to the complementary material;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. detecting emitted luminescence to determine whether the analyte of interest is present in the sample.

The reagent may be the analyte of interest conjugated to an electrochemiluminescent chemical moeity or an analogue of the analyte of interest conjugated to an electrochemiluminescent moiety. Additionally, the analyte of interest may be theophylline, digoxin or hCG. Examples of specific electrochemiluminescent chemical moieties are those disclosed earlier in this specification in connection with the method of claim 1.

The complementary material may be a whole cell, subcellular particle, virus, prion, viroid, lipid, fatty acid, nucleic acid, polysaccharide, protein, lipoprotein, lipopolysaccharide, glycoprotein, peptide, cellular metabolite, hormone, pharmacological agent, tranquilizer, barbiturate, steroid, vitamin, amino acid, sugar, non-biological polymer, synthetic organic molecule, organometallic molecule or inorganic molecule.

As indicated hereinbefore, it is also within the scope of this application that the methods provided herein may be performed so as to quantity an analyte of interest

In accordance with this embodiment, there is provided a method for quantitatively determining in a predetermined volume of a multicomponent, liquid sample the amount present in the sample of an analyte of interest which, if present in the sample, is at a concentration which is below $10^{-3}$ molar, said method comprising:

a. contacting the sample with a known amount of a reagent (i) comprising a metal-containing organic compound conjugated to a whole cell, subcellular particle, virus, prion, viroid, lipid, fatty acid. nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of combining with the analyte of interest, the contact being effected under conditions such that the analyte and conjugate bind;

b. exposing the resulting sample to an amount of electrochemical energy from a suitable source effective to incuce the reagent to repeatedly emit electrochemiluminescence, the exposure being effected under suitable conditions so as to induce the reagent to repeatedly emit electrochemiluminescence; and

c. quantitatively determining the amount of emitted luminescence to determine whether and in what amount the analyte of interest is present in the sample.

This quantitative method may be performed as a heterogeneous assay or as a homogeneous assay. In one embodiment of the invention any reagent which is not combined with the anaalyte of interest is separated from the sample, which had been contacted with a known amount of the reagent, prior to the exposure of the sample to an amount of electrochemical energy from a suitable source effective to induce the reagent to repeatedly emit radiation. In yet another embodiment of the invention, prior to contacting the sample with the reagent, the sample is treated so as to immobilize the analyte of interest.

The analyte of interest for this embodiment of the invention may be a whole cell, subcellular particle, virus, prion, viroid, lipid, fatty acid, nucleic acid, polysaccharide, protein, lipoprotein, lipopolysaccharide, glycoprotein, peptide, cellular metabolite, hormone, pharmacological agent, tranquilizer, barbiturate, alkaloid, steroid, vitamin, amino acid, sugar, non-biological polymer, synthetic organic molecule, organometallic molecule or inorganic molecule. In one embodiment of the invention, the analyte of interest is theophylline. In another embodiment of the invention, the analyte of interest is digoxin. In yet another embodiment of the invention, the analyte of interest is hCG.

The reagent with which the sample is contacted may be an electrochemiluminescent chemical moiety conjugated to a whole cell, subcellular particle, virus, prion, viroid, lipid, fatty acid, nucleic acid, polysaccharide, protein, lipoprotein, lipopolysaccharide, glycoprotein, peptide, cellular metabolite, hormone, pharmacological agent, tranquilizer, barbiturate, alkaloid, steroid, vitamin, amino acid, sugar, non-biological polymer, synthetic organic molecule, organometallic molecule or inorganic molecule.

In one acid, this embodiment of the invention the reagent is a electrochemiluminescent chemical moiety conjugated to an antibody, antigen, nucleic acid, hapten, ligand or enzyme, or biotin, avidin or streptavidin.

The electrochemiluminescent moiety may be as described hereinbefore, that is to say it may be a metal-containing organic compound wherein the metal is selected from the group consisting of ruthenium, osmium, rhenium, iridium, rhodium, platinum, palladium, molybdenum and technetium. Preferably, the metal is ruthenium or osmium.

Specific examples of suitable electrochemiluminescent chemical moieties are bis [(4,4′-carbomethoxy)2,2′-bipyridine] 2-[3-(4-methyl-2,2′-bipyridine-4-yl)propyl]-1,3-dioxolane ruthenium (II); bis(2,2′-bipyridine)[4-(butan-1-al)-4′-methyl-2,2′-bipyridine] ruthenium (II); bis (2,2′-bipyridine) [4-(4-methyl-2,2′-bipyridine-4′-yl)-butyric acid] ruthenium (II); (2,2′-bipyridine)[cis-bis(1,2-diphenylphosphino)ethylene]{2-[3-(4-methyl-2,2′-bipyridine-4′-yl)propyl]-1,3- dioxolane}osmium (II); bis(2,2;-bipyridine) [4-(4′-methyl-2,2′-bipyridine)-butylamine]-ruthenium (II); bis(2,2′-bipyridine)[1-bromo-4(4′-methyl-2,2′-bipyridine-4-yl)butane] ruthenium (II); bis (2,2′-bipyridine)maleimidohexanoic acid, 4-methyl-2,2′-bipyridine-4′-butyl-amide ruthenium (II); and bis (2,2′-bipyridine)maleimidohexanoic acid, 4-methyl-2,2′-bipyridine-4′-butylamide ruthenium (II) diperchlorate.

The sample may be derived from a solid, emulsion, suspension, liquid or gas. Samples which comprise the analyte of interest may be derived from water, food, blood, serum, urine, feces, tissue, saliva, oils, organic solvents or air. Additionally, samples may comprise dimethylsulfoxide, dimethylformamide, n-methylpyrrolidinone or tert-butyl alcohol. Futhermore, the sample may comprise a reducing agent or an oxidizing agent.

The invention also provides a competitive method for quantitatively determining in a predetermined volume of a multicomponent, liquid sample the amount of an analyte of interest present in the sample. In accordance with the embodiment, there is provided a method for quantitatively determining in a predetermined volume of a multicomponent, liquid sample the amount present in the sample of an analyte of interest which, if present in the sample, is at a concentration which is below $10^{-3}$ molar, said method comprising:

a. contacting the sample with a known amount of a complementary material and a known amount of reagent (i) comprising a metal-containing organic compound conjugated to a whole cell, subcellular particle, virus, prion, viroid, lipid, fatty acid, nucleic acid, polysaccharide other than a sugar protein, lipoprotein, lipopolysaccharide, glycoprotein, peptide, cellular metabolite, hormone, pharamcological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of competing with the analyte of interest for binding sites on the complementary material not normally present in the sample, the contact being effected under conditions such that the analyte of interest and conjugate competitively bind to the complementary material;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. quantitatively determining the amount of emitted luminescence to determine whether and in what amount the analyte of interest is present in the sample.

The analyte of interest may be theophylline, digoxin or hCG.

In one embodiment of the invention, the reagent is the analyte of interest, or an analogue of the analyte of interest, conjugated to an electrochemiluminescent metal-containing organic compound which may be any one of those specifically mentioned above.

As before, the complementary material may be a whole cell, subcellular particle, virus, prion, viroid, lipid, fatty acid, nucleic acid, polysaccharide, protein, lipoprotein, lipopolysaccharide, glycoprotein, peptide, cellular metabolite, hormone, pharmacological agent, tranquilizer, barbiturate, alkaloid, steroid, vitamin, amino acid, sugar, non-biological polymer, synthetic organic molecule, organometallic molecule or inorganic molecule.

In one embodiment of the invention, the reagent may comprise a metal-containing organic compound Z attached to a biological entity X where X is selected from one or more nucleotides which may be the same or different, one or more amino acids which may be the same or different, an antibody, an analyte of interest or an analogue of an analyte of interest X may be, for example, theophylline, digoxigenin or a peptide derived from hCG. Optionally, X may be attached to Z by a linker Y, containing at least one carbon atom.

In one embodiment, the metal-containing organic compound has the structure

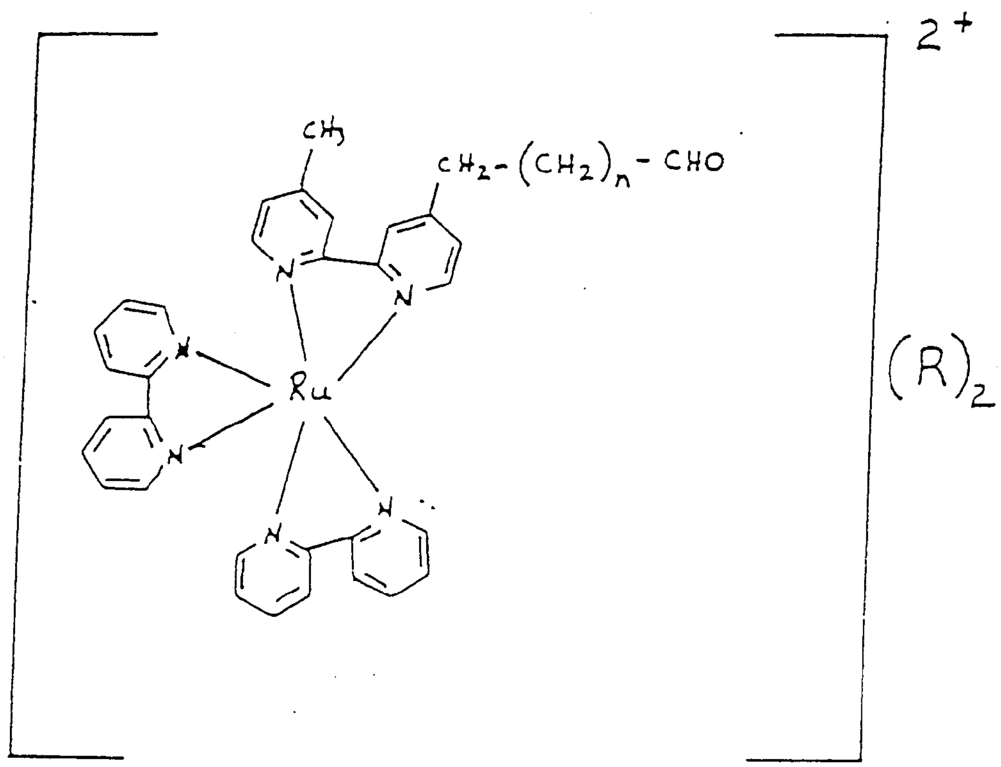

wherein R is an anion and n is an integer. In one embodiment of the invention, n is 2.

In a further embodiment, the metal-containing organic compound has the structure

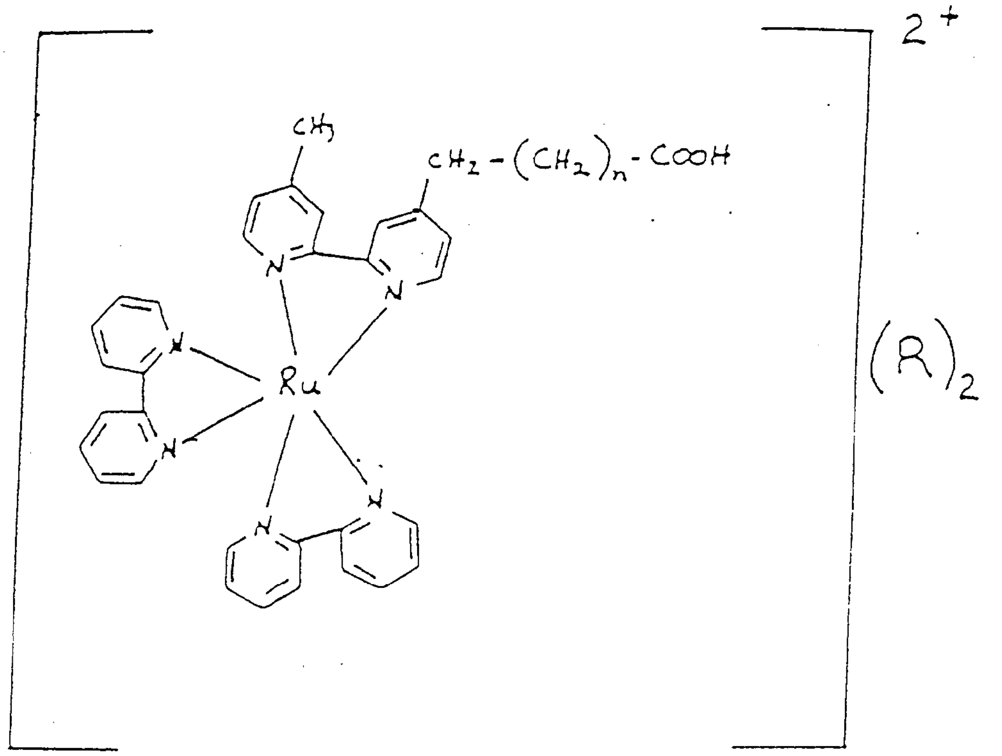

wherein R is an anion an n is an integer. In one embodiment of the invention, n is 2.

In another embodiment, the metal organic compound has the structure

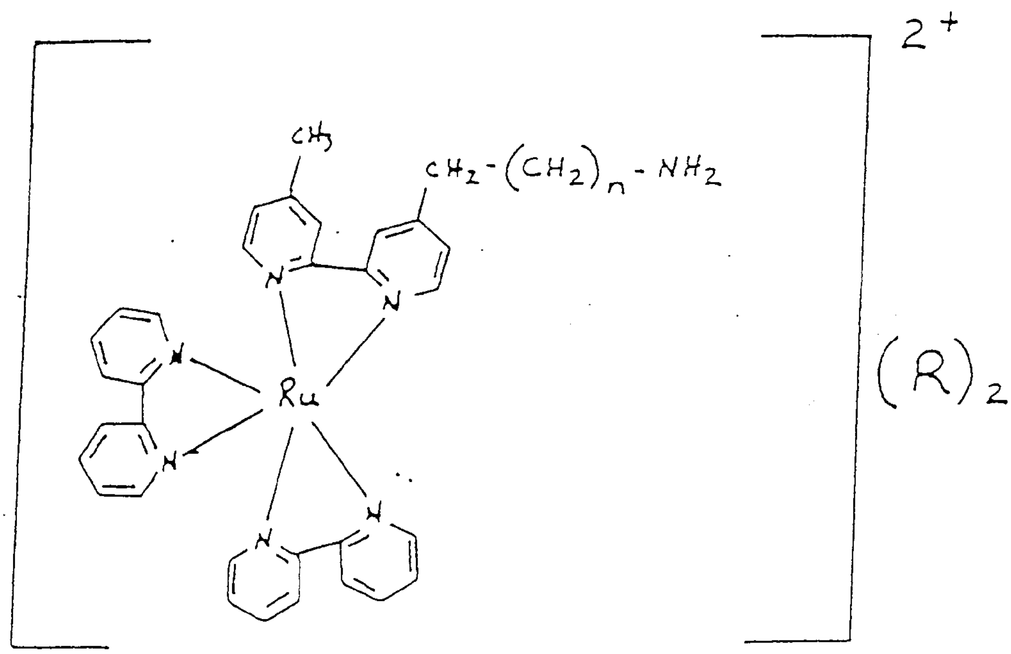

wherein R is an anion and n is an integer. In one embodiment of the invention, n is 3.

In yet another embodiment, the metal-conatining organic compound has the structure

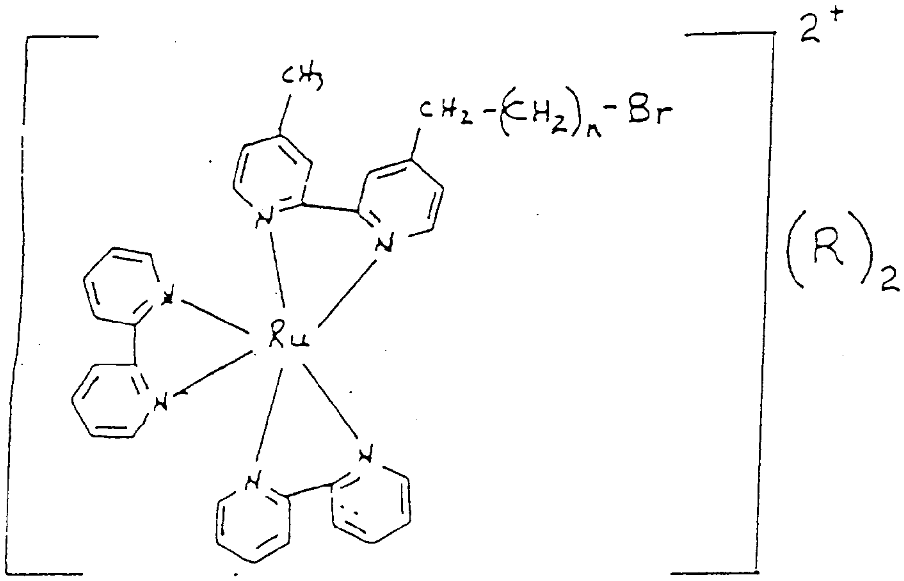

wherein R is an anion and n is an integer. In one embodiment of the invention, n is 3.

In still yet another embodiment, the metal organic compound has the structure

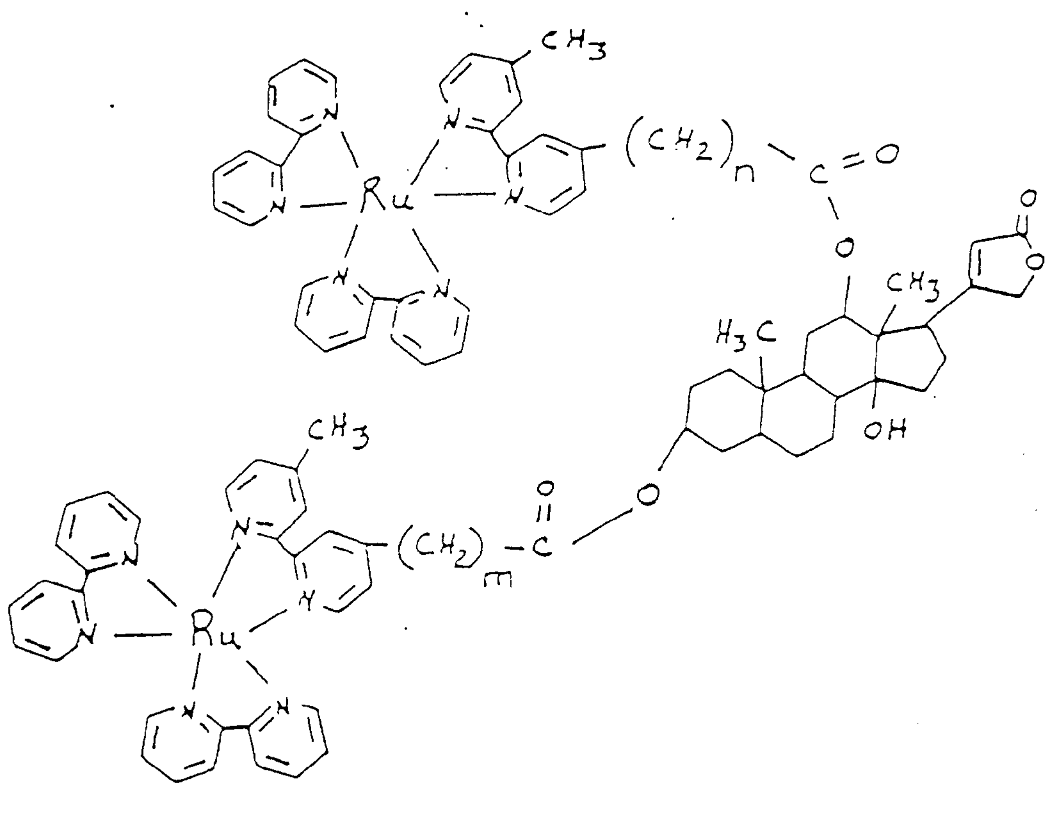

wherein m and n are each integers which may be the same or different. In one embodiment of the invention, m and n are both 3.

In another embodiment, the metal organic compound has the structure

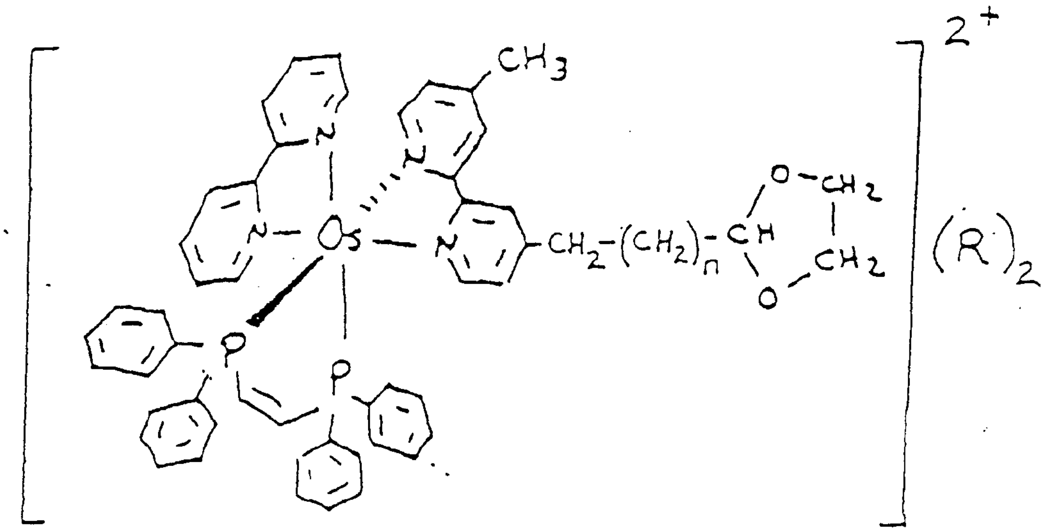

wherein R is an anion and n is an integer. In one embodiment of the invention, n is 2.

In other embodiments, the metal organic compound may have the structure

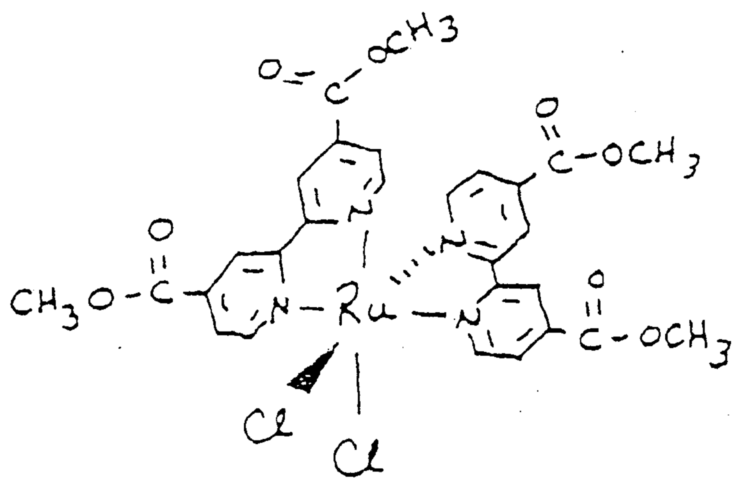

or the structure

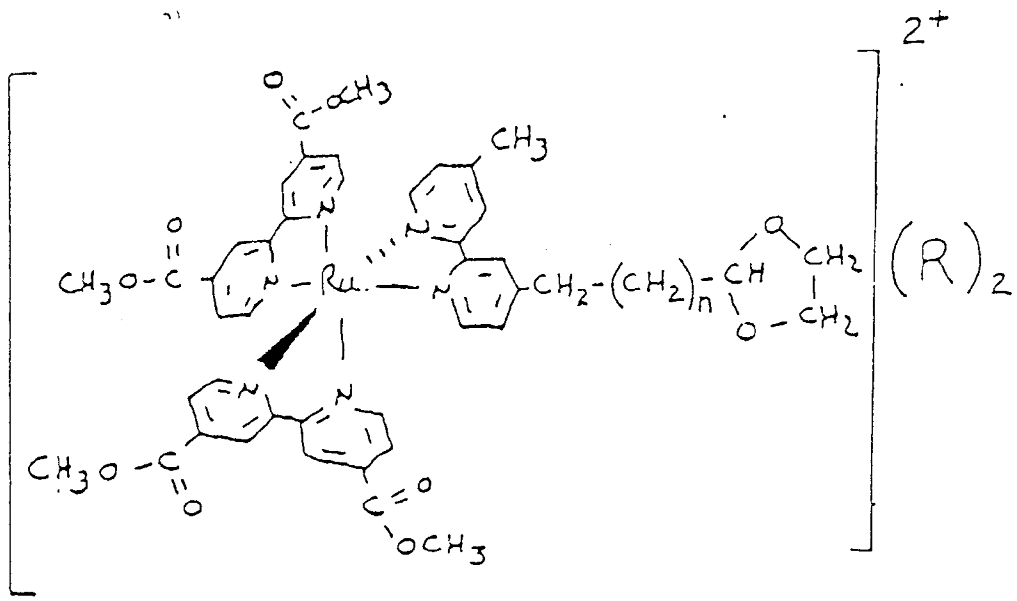

wherein R is an anion and n is an integer. In one embodiment of the invention, n is 2.
or the structure

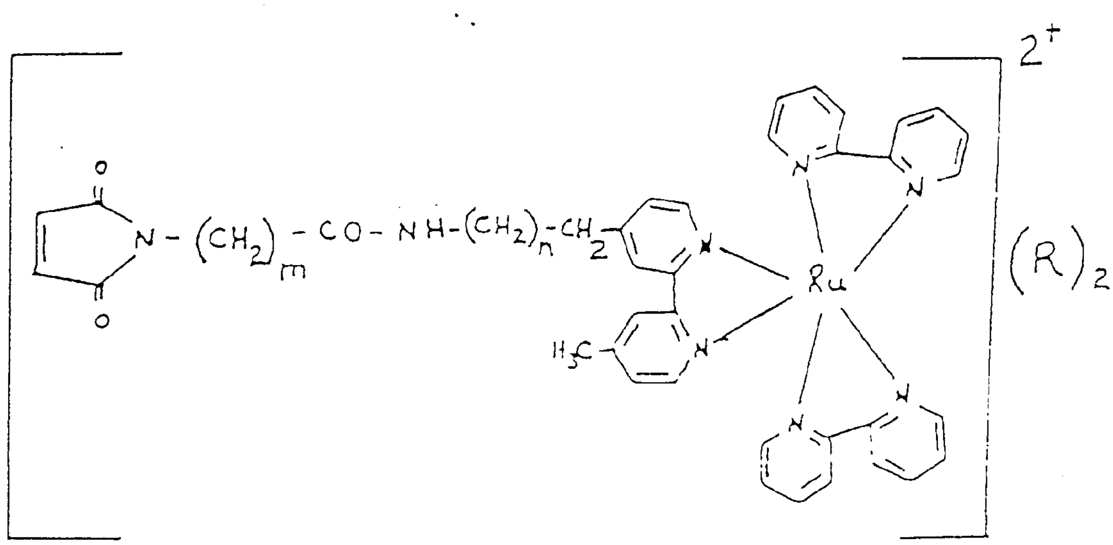

wherein R is an anion and m and n are integers. In one embodiment of the invention, m is 5 and n is 3.

The reagent may also be a composition of matter having the structure

$$X\text{-}CH{=}CH\text{-}CO\text{-}NH\text{-}(CH_2)_n\text{-}NH\text{-}CO\text{-}(CH_2)_m\text{-}Z$$

wherein:

X represents one or more nucleotides which may be the same or different;
Z represents the metal organic compound;
n represents an integer greater than or equal to 1; and
m represents an integer greater than or equal to 1.

In one embodiment of the invention, X is thymidine attached to CH at carbon 5, n is 7 and m is 3.

In another embodiment of the invention Z is bis (2,2′-bipyridine) [4 -(butan-1-al)-4′ methyl-2,2′-bipyridine] ruthenium (II).

In yet another embodiment of the invention, the thymidine nucleotide is a 3′ terminal nucleotide attached to the nucleotide sequence

TCACCAATAAACCG CAAACACCA TCCCG TCCTG CCAG

Another suitable composition of matter for the reagent has the structure

$$[T\text{-}Y\text{-}Z]^{2+}(R)_2$$

wherein T represents theophylline, Y represents a linker group attaching T to Z, Z represents bis-(2,2′-bipyridine) [4-methyl-2,2′-bipyridine-4′-yl] ruthenium (II) and R represents an anion.

In one embodiment of the invention, Y is attached to the carbon at position 8 of T. In another embodiment of the invention, Y has the structure

$$(CH_2)_m\text{-CO-NH-}(CH_2)_n$$

wherein m and n represent an integer, which may be the same or different, greater than or equal to 1. In another embodiment of the invention, m is 3 and n is 4, In another embodiment of the invention, m and n are both 3.

In yet another embodiment of the invention, Y has the structure

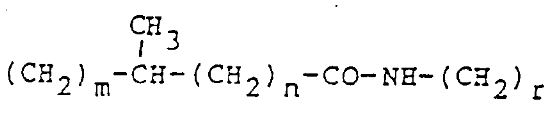

wherein m, n are r represent an integer, which may be the same or different, greater than or equal to 1. In one embodiment of the invention, m is 1, n is 1 and r is 4.

In still a further embodiment of the invention, Y has the structure

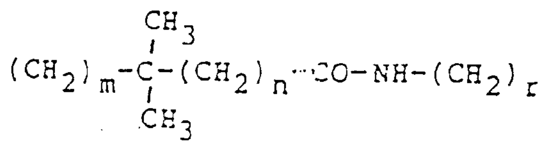

wherein m, n and r represent an integer, which may the same or different, greater than or equal to 1. In one embodiment of the invention, m is 1, n is 1 and r is 4.

In yet another embodiment of the invention, Y is attached to the nitrogen at position 7 of T. In one embodiment of the invention, Y has the structure

$$(CH_2)_n$$

wherein n is an integer greater than or equal to 1. In still another embodiment of the invention, n is 4.

Another example of a composition of matter suitable for use as the reagent is one have the structure

$$X\text{-}Z$$

wherein X represents one or more amino acids which may be the same or different, comprising at least one amino acid which is cysteine or methionine, and Z is bis (2,2′-bipyridine) maleimidohexanoic acid, 4-methyl-2,2′-bipyridine-4′-butylamide ruthenium (II) attached by the carbon at position 3 or 4 of the maleimide to a sulfur substituent of cysteine or methionine.

The following examples are provided to illustrate the present invention.

Example 1 - Electrochemiluminescence in Various Organic Solvents

The electrochemiluminesence of tris (2,2′-bipyridyl) ruthenium (II) chloride hexahydrate was measured in a 15 ml three-neck, round bottom flask containing 10 ml of a solution prepared as described below; a 1.5mm x 10 mm magnetic stir bar; a 1.0mm diameter silver wire quasi-reference electrode; a combination 28 gauge platinum wire counter electrode; and a working electrode consisting of a 22 gauge platinum wire welded to a 1 cm x 1 cm square piece of 0.1 mm thick, highly polished platinum foil. (The working platinum foil electrode was shaped into a 4.76 mm (3/16 of an inch) diameter semi-circle surrounding the 28 gauge platinum wire counter electrode by 2.38mm (3/32 of an inch) equidistantly.)

The silver wire was connected into EG&G Model 178 electrometer probe of the EG&G Model 173 potentiostat/galvanostat. The platinum wire counter electrode and the platinum working electrode were connected to the anode and cathode respectively of the EG&G Model 173 potentiostat. The device was grounded.

Cyclic voltammetry was performed with the EG&G Model 173 potentiostat to which an EG&G Model 175 universal programmer was attached. The programmer was set for 100 mV/second sweeps between +1.75 volt anodic and -1.80 volt cathodic potentials. Electrochemiluminescence was detected using a Hamamatsu R928 photomultiplier tube, set inside a Products for Research Model PR1402RF photomultiplier tube housing which

was fitted with a Kodak #23A gelatin (red) filter. The photomultiplier tube housing was connected to an Oriel Model 7070 photomultiplier detection system. The cyclic voltammogram was recorded on a Houston Instruments Model 200 X-Y recorder.

Cyclic voltammograms were generated for 1mM tris (2, 2′ - bipyridyl) ruthenium (II) chloride hexahydrate (Aldrich Chemical Company), 0.1M tetrabutylammonium tetrafluoroborate ($TBABF_4$) (Aldrich Chemical Company) solutions prepared with the following organic solvents: acetonitrile; n-dimethylformamide; dimethylsulfoxide and 1-methyl, 2-pyrrolidinone (Aldrich Chemical Company). Tert-butyl alcohol and deionized, distilled water (1:1, v/v) also was used to make a solution containing 1 mM tris (2, 2′-bipyridyl) ruthenium II chloride hexahydrate and 0.1 M $TBABF_4$. The resulting voltammograms did not indicate any change in the redox potential of the tris (2,2′-bipyridyl) ruthenium (II) chloride hexahydrate upon variation of the organic solvent.

For visual determination of electrochemiluminescence, solutions were prepared as follows: sufficient amounts of tris (2,2′-bipyridyl) ruthenium (II) chloride hexahydrate and $TBABF_4$ were dissolved in the spectroscopic grade organic solvents (Aldrich Chemical Company) described above to provide final concentrations of 1mM and 0.1M, respectively. 10ml of the resulting solution was then added to the 15 ml three-neck round bottom flask. The electrodes were immersed in the solution and the working electrode pulsed between a +1.75 and -1.45 volt potential to generate electrochemiluminescence. Electrochemiluminesence was visually observed in each of the solutions described above.

For quantitative measurements of the effect of solvent variation on electrochemiluminescence, solutions were prepared as follows: sufficient amounts of tris (2, 2′-bipyridyl) ruthenium (II) chloride hexahydrate and $TBABF_4$ were added to the organic solvents described above to provide final concentrations of 2 mM and 0.2 M respectively. To an aliquot of this solution was added an equal volume of deionized, distilled water containing a strong oxidizing ammonium persulfate, at a concentration of 36 mM. Control solutions that did not contain the tris (2, 2′-bipyridyl) ruthenium (II) chloride hexahyrate were prepared. 10ml of the resulting solution was then added to the 15 ml threeneck round bottom flask. Electrochemiluminescence was accomplished by pulsing for one second intervals, between zero and -2.0 volts cathodic potential.

Electrochemiluminescent measurements were performed by integrating the resulting electrochemiluminescent photomultiplier tube signal using an. integrator connected to a Micronta Model 22191 digital multimeter. The electrochemiluminescent signal was integrated for 10 seconds during the pulsing and recorded in millivolts. The results are shwon in Table I and indicate that variation of solvents effects quantum efficiency of the ruthenium (II) chloride.

TABLE I

| Organic Solvent | Tris RuBiPy ($10^{-6}$M) | Control | Δ |
|---|---|---|---|
| Acetonitrile | 2,540* | 104 | 2,436 |
| tert-butyl alcohol | 1,280 | 0 | 1,280 |
| N, N dimethyl-formamide | 2,390 | 143 | 2,247 |
| Dimethylsulfoxide | 2,760 | 29 | 2,731 |
| 1-methyl-2-pyrrolidinone | 1,630 | 0 | 1,630 |

*all measurements in millivolts.

Example 2 - Sensitivity of Detection of Electrochemiluminescence of Ruthenium- Labeled Rabbit Anti-Mouse Immunoglobulin G (IgG) Antibody

The electrochemiluminescence of rabbit anti-mouse IgG antibody labeled with 4,4′ - (dichloromethyl) - 2,2′ -bipyridyl, bis(2,2′ - bipyridyl) ruthenium (II) (ruthe-nium-labeled rabbit anti-mouse IgG antibody) was measured in a 15 ml three-neck, round bottom flask containing 10 ml of a solution prepared as described below; a 1.5mm x 10mm magnetic stir bar; a 1.0 mm diameter silver wire quasi-reference electrode; a combination 28 gauge platinum wire counter electrode, and a working electrode consisting of a 22 gauge platinum wire welded to a 1 cm x 1 cm square piece of 0.1 mm thick, highly polished platinum foil. (The working platinum foil electrode was shaped into a 4.76 mm (3/16 of an inch) diameter semi-circle surrounding the 28 gauge platinum wire counter electrode by 2.38 mm (3/32 of an inch) equidistantly.)

The silver wire was connected to the EG&G Model 178 electrometer probe of the EG&G Model 173 potentiostat/qalvanostat. The platinum wire counter electrode and the platinum working electrode were connected to the anode and cathode respectively of the EG&G Model 173 potentiostat. The device was grounded.

The electrochemiluminescence emitted from the ruthenium-labeled rabbit anti-mouse IgG antibody solution was detected using an Hamamatsu R928 photomultiplier tube, set inside a Products for Research Model PR1402RF photomultiplier tube housing which was fitted with a Kodak #23A gelatin (red) filter. The photomultiplier tube housing was connected to an Oriel Model 7070 photomultiplier detection system.

Electrochemiluminescence was induced by pulsing for one second intervals, between zero and -2.0 volts cathodic potential. Electrochemiluminescent measurements were performed by integrating the resulting electrochemiluminescent photomultiplier tube signal using an integrator connected to a Micronta Model 22191 digital multimeter. The electrochemiluminescent signal was integrated for 10 seconds during the pulsing and recorded in millivolts.

A stock solution of 1.25 x $10^{-7}$M ruthenium-labeled rabbit anti-mouse IgG antibody was prepared from a concentrated solution (2 mg/ml, 7.5 Ru/antibody) of the labeled antibody by dilution in phosphate-buffered saline (PBS). An aliquot of this solution (80 microliters) was added to 10 ml of dimethylsulfoxide (DMSO)/deionized, distilled water (1:1) containing 0.1 M tetrabutylammonium tetrafluoroborate ($TBABF_4$) and 18 mM ammonium persulfate in the reaction vessel. The final ruthenium-labeled antibody concentration was 1 x $10^{-9}$M. Electrochemiluminescence was measured as described above.

Additional solutions representing various dilutions of the ruthenium-labeled rabbit anti-mouse IgG antibody stock solution were made and aliquots (80 microliters) of these solutions were added to the same solution of ruthenium-labeled antibody in the reaction vessel in increments which resulted in the following concentrations of labeled antibody: 5 x $10^{-9}$M, 1 x $10^{-8}$M, and 5 x $10^{-8}$M. Electrochemiluminescence measurements were made for each solution as described. These measurements are listed in Table II below. These results indicate the sensitivity of electrochemiluminescent detection of labeled antibody (1 x $10^{-9}$M), and the dependence of the intensity of electrochemiluminescence on the concentration of the ruthenium-labeled anti-mouse IgG antibody.

TABLE II

ELECTROCHEMILUMINESCENCE (ECL) OF RUTHENIUM-LABELED RABBIT ANTI-MOUSE IMMUNOGLOBULIN G (IgG) ANTIBODY

| Concentration of Ruthenium-Labeled Anti-Mouse IgG Antibody | ECL (mV) |
|---|---|
| 5 x $10^{-8}$M | 1610 |
| 1 x $10^{-8}$M | 892 |
| 5 x $10^{-9}$M | 418 |
| 1 x $10^{-9}$M | 72 |
| 0 | 0 |

Example 3 - Immunological Reactivity of Ruthenium-Labeled Bovine Serum Albumin (BSA) In a Solid Phase Enzyme Linked-Immunosorbent Assay (ELISA)

The wells of a polystyrene microtiter plate were coated with a saturating concentration of either bovine serum albumin labeled with 4,4′ - (dichloromethyl) - 2,2′ - bipyridyl, bis(2,2′ - bipyridyl) ruthenium (II), i.e. ruthenium-labeled bovine serum albumin, (6 Ru/BSA, 20 micrograms/ml in PBS buffer, 50 microliters/well) or unlabeled BSA (20 micrograms/ml in PBS buffer, 50 microliters/well) and incubated for one hour at room temperature. After this incubation period the plate was washed three times with PBS, 5 minutes per wash. A solution containing 6 mg/ml rabbit anti-BSA antibody was diluted 1:20,000, 1:30,000, 1:40,000, 1:50,000, and 1:60,000 in PBS, and the dilutions were added in duplicate to the wells coated with ruthenium-labeled BSA or unlabeled BSA, and the plate was incubated for one hour at room temperature. After three washes with PBS as before, the presence of bound rabbit anti-BSA antibody was determined by adding goat anti-rabbit IgG-peroxidase conjugate (1:1000 dilution in PBS of a 0.5 mg/ml solution, 50 microliters/well) to each well and incubating the plate for one hour at room temperature. After washing the plate twice with PBS, 0.5% Tween-20, and twice with PBS as before, hydrogen peroxide (30%) and 2,2′-azino-di-[3-ethyl-benzthiazoline sulfonate] (KPL, Gaithersburg, MD) were mixed in equal volumes and 200 microliters were added to each well of the plate. After a 30 minute incubation at room temperature, the plate was read spectrophotometrically at 414 nm. The mean background absorbence in the control wells was subtracted from the mean value of duplicate readings for each dilution of the rabbit anti-BSA antibody that was added to the wells coated with ruthenium-labeled BSA or unlabeled BSA. These corrected absorbence values are shown in Table III.

The curves obtained for the unlabeled BSA and ruthenium-labeled BSA were parallel, with corrected absorbance values at each point on the two curves at a constant ratio, approximately 0.6. Identical results were obtained for two other lots of ruthenium-labeled BSA which were made using the same activated ruthenium complex as described previously, and which had similar Ru/BSA labeling ratios. These results indicate that the ruthenium-labeled BSA is immunologically reactive and that it retains approximately 60% of its immunoreactivity when labeled with ruthenium in comparison to unlabeled BSA.

TABLE III

ABSORBANCE AT 414 nm

| Rabbit Anti-BSA Antibody Dilution | Unlabeled BSA | Ruthenium-labeled BSA | Relative Immunoreactivity |
|---|---|---|---|
| 20,000 | 1.06 | 0.66 | 62% |
| 30,000 | 0.83 | 0.50 | 60% |
| 40,000 | 0.67 | 0.40 | 60% |
| 50,000 | 0.56 | 0.33 | 59% |
| 60,000 | 0.47 | 0.28 | 60% |

Example 4 - Immunological Reactivity of Ruthenium-Labeled Rabbit Anti-Mouse Immunoglobulin (IgG) Antibody by a Competitive Solid Phase Enzyme Linked-Immunosorbent Assay (ELISA)

Rabbit anti-mouse IgG antibody labeled with 4,4′ - (dichloromethyl) - 2,2′ - bipyridyl, bis(2,2′ - bipyridyl) ruthenium (II) (ruthenium-labeled rabbit anti-mouse IgG antibody) was compared with unlabeled rabbit anti-mouse IgG antibody with respect to its ability to compete with enzyme-labeled, anti-mouse IgG antibody for binding to mouse IgG. The wells of a 96-well polystyrene microtiter plate were coated with a solution of mouse IgG (5 micrograms/ml in PBS buffer), incubated for 60 minutes at room temperature and washed three times, 5 minutes per wash, with PBS. Two solutions were prepared, one containing a mixture of rabbit anti-mouse IgG-alkaline phosphatase conjugate and rabbit anti-mouse IgG (1 mg/ml), and the other a mixture of rabbit anti-mouse IgG-alkaline phosphatase conjugate and ruthenium-labeled rabbit anti-mouse IgG (1 mg/ml, 7.5 Ru/antibody). These two solutions, and a third containing rabbit anti-mouse IgG-alkaline phosphatase conjugate, were diluted 1:6000, 1:7000, 1:8000, 1:9000, 1:10,000, 1:12,000, 1:14,000 and 1:16,000 in PBS containing 0.5% Tween-20, and added (50 microliters/well) to separate rows of the plate containing bound mouse IgG. The plate was incubated for 60 minutes at room temperature and washed twice with PBS-Tween-20 and twice with PBS, 5 minutes per wash. The enzyme substrate p-nitrophenyl phosphate (1.5 mg/ml in 10% diethanolamine buffer, pH 9.6) was added to each well (200 microliters/well); the plate was incubated for 30 minutes at room temperature and read spectrophotometrically at 405 nm. The mean background absorbance in the control wells was subtracted from the mean value of duplicate readings for each of the three solutions at each dilution. These absorbance values are shown in Table IV.

Three parallel curves were obtained, the top curve representing the uninhibited binding of the enzyme conjugate, and the two lower curves representing inhibition by ruthenium - labeled anti-mouse IgG and unlabeled anti-mouse IgG. The ruthenium-labeled, anti-mouse IgG curve, on a point-by-point comparison, is approximately 81% as low as the unlabeled anti-mouse IgG curve in comparison to the enzyme conjugate curve. These results indicate that the ruthenium-labeled, anti-mouse IgG antibody is immunologically reactive for its antigen (mouse IgG) , and is approximately 81% as effective as unlabeled anti-mouse IgG antibody in competing with enzyme-labeled, anti-mouse IgG antibody for binding to mouse IgG.

TABLE IV

ABSORBANCE AT 405 nm

| Dilution | A<br>Anti-Mouse IgG Alkaline Phosphatase (Enzyme Conjugate) | B<br>Enzyme Conjugate+ Ruthenium Labeled Anti-Mouse IgG | C<br>Enzyme Conjugate+ Unlabeled Anti-Mouse IgG | Comparative* Degree of Inhibition |
|---|---|---|---|---|
| 6,000 | 1.48 | 0.94 | 0.79 | 77% |
| 7,000 | 1.33 | 0.82 | 0.69 | 79% |
| 8,000 | 1.21 | 0.72 | 0.62 | 82% |
| 9,000 | 1.10 | 0.65 | 0.56 | 84% |
| 10,000 | 1.01 | 0.60 | 0.51 | 82% |
| 12,000 | 0.87 | 0.51 | 0.43 | 80% |
| 14,000 | 0.77 | 0.45 | 0.37 | 81% |
| 16,000 | 0.68 | 0.40 | 0.33 | 80% |

*(A-B/A-C) x 100%

Example - 5 Electrochemiluminescence of Ruthenium-Labeled Bovine Serum Albumin (BSA)

A solution containing 7.8 x $10^{-6}$ M bovine serum albumin (BSA) labeled with 4,4′ - (dichloromethyl) - 2,2′-bipyridyl, bis(2,2′ bipyridyl) ruthenium (II) (ruthenium-labeled bovine serum albumin) was prepared from a stock solution of ruthenium-labeled BSA (2.6 mg/ml, 6 Ru/BSA) by dilution in phosphate-buffered saline. 26 microliters of this solution were added to 10 ml of DMSO/deionized, distilled water (1:1) containing 0.1 M $TBABF_4$ and 18 mM ammonium persulfate in the reaction vessel. The final ruthenium-labeled BSA concentration was 2 x $10^{-8}$M. Electrochemiluminescence was measured as described in Example 2.

In an analogous manner, a solution containing 7.8 x $10^{-6}$M unlabeled BSA was prepared and added to the

reaction vessel to give a final unlabeled BSA concentration of 2 x $10^{-8}$M. The electrochemiluminescence of this solution and of a similar solution without BSA was measured. Electrochemiluminescence measurements are shown in Table V for covalently coupled, ruthenium-labeled BSA and unlabeled BSA.

TABLE V

ELECTROCHEMILUMINESCENCE (ECL) OF RUTHENIUM-LABELED BSA

| Solution | ECL (mV) |
|---|---|
| 2 x $10^{-8}$M Ruthenium-Labeled BSA | 730 |
| 2 x $10^{-8}$M BSA | 100 |
| DMSO: $H_2O$ (1:1) | 0 |

Example 6 - Electrochemiluminescence of Ruthenium-Labeled Rabbit Anti-Mouse Immunoglobulin G (IgG) Antibody

A solution containing 1.25 x $10^{-6}$ M rabbit anti-mouse IgG antibody labeled with 4,4′ - (dichloromethyl) - 2,2′ - bipyridyl, bis(2,2′ - bipyridyl) ruthenium (II) (ruthenium-labeled, rabbit anti-mouse IgG antibody) was prepared from a stock solution of ruthenium-labeled, rabbit anti-mouse IgG antibody (2 mg/ml, 7.5 Ru/antibody) by dilution in phosphate-buffered saline. 80 microliters of this solution were added to 10 ml of DMSO/deionized, distilled water (1:1) containing 0,1M $TBABP_4$ and 18 mM ammonium persulfate in the reaction vessel. The final ruthenium-labeled antibody concentration was 1 x $10^{-8}$M. Electrochemiluminescence was measured as described in Example 2.

In an analogous manner, a solution containing 1.25 x $10^{-6}$M unlabeled, rabbit anti-mouse IgG antibody was prepared and added to the reaction vessel to give a final unlabeled antibody concentration of 1 x $10^{-8}$M. The electrochemiluminescence of this solution and of the solution without added antibody was also measured as described. Electrochemiluminescent measurements are shown in Table VI for covalently-coupled, ruthenium-labeled rabbit anti-mouse IgG antibody and unlabeled rabbit anti-mouse IgG antibody.

TABLE VI

ELECTROCHEMILUMINESCENCE (ECL) OF RUTHENIUM-LABELED RABBIT ANTI-MOUSE IMMUNOGLOBULIN G (IgG) ANTIBODY

| Solution | ECL (mV) |
|---|---|
| 1 x $10^{-8}$M Ruthenium - Labeled Rabbit Anti-Mouse IgG Antibody | 892 |
| 1 x $10^{-8}$M Rabbit Anti-Mouse IgG Antibody | 0 |
| DMSO: $H_2O$ (1:1) | 0 |

Example 7 - Homogeneous Electrochemiluminescent Immunoassay for Antibody to Bovine Serum Albumin

A solution containing 7.8 x $10^{-6}$M bovine serum albumin (BSA) labeled with 4,4′ - ( dichloromethyl-) - 2,2′ bipyridyl, bis (2,2′ - bipyridyl) ruthenium (II) (ruthenium-labeled bovine serum albumin) was prepared from a stock solution of ruthenium-labeled BSA (2.5 mg/ml, 6 Ru/BSA) by dilution in phosphate-buffered saline (PBS). 26 microliters of this solution were added to 10 ml of DMSO/deionized, distilled water (1:1) containing 0.1M $TBABF_4$ and 18 mM ammonium persulfate in the reaction vessel. The final ruthenium-labeled BSA concentration was 2 x $10^{-8}$M. Electrochemiluminescence was measured as described in Example 2.

In an analogous manner, a solution containing 7.8 x $10^{-6}$M unlabeled BSA was prepared and added to the reaction vessel to give a final unlabeled BSA concentration of 5 x $10^{-8}$M. The electrochemiluminescence of this solution and of a similar solution without BSA were measured.

A solution containing 3.75 x $10^{-5}$M rabbit anti-BSA antibody was prepared from a stock solution of rabbit anti-BSA antibody (6.0 mg/ml) by dilution in PBS, and an aliquot (26 microliters) was added to the solution of ruthenium-labeled BSA in the reaction vessel to give a final rabbit anti-BSA antibody concentration of 1 x $10^{-7}$M.

The electrochemiluminescence of the resulting mixture of ruthenium-labeled BSA antigen and antibody (rabbit anti-BSA) was measured. The results shown in Table VII indicate a reduction in the electrochemiluminescence of the ruthenium-labeled BSA upon addition of rabbit anti-BSA antibody and demonstrate that a homogeneous electrochemiluminescent detection of antibody to BSA may be achieved. Based upon these results one skilled in the art would know that a homogeneous electrochemiluminescent immunoassay for detecting other analytes of interest may be developed.

TABLE VII

REDUCTION OF ELECTROCHEMILUMINESCENCE (ECL) OF RUTHENIUM-LABELED BOVINE SERUM ALBUMIN UPON BINDING OF ANTIBODY

| UNLABELED BSA (CONTROL) | RUTHENIUM-LABELED BSA (ANTIGEN) | RABBIT ANTI-BSA (ANTIBODY) | ECL (mV) |
|---|---|---|---|
| 0 | 2 x $10^{-8}$M | 0 | 727 |
| 0 | 2 x $10^{-8}$M | 1 x $10^{-7}$M | 92 |
| 2 x $10^{-8}$M | 0 | 0 | 94 |

Example 8 - Homogeneous Electrochemiluminescent Immunoassay for Mouse Immunoglobulin G (IgG)

A solution containing 6.25 x $10^{-6}$M rabbit anti-mouse IgG antibody labeled with 4,4′ - (dichloromethyl) - 2,2′ - bipyridyl, bis (2,2′ - bipyridyl) ruthenium (II) (ruthenium-labeled rabbit anti-mouse IgG antibody) was prepared from a stock solution of ruthenium-labeled rabbit anti-mouse IgG antibody (2 mg/ml, 7.5 Ru/antibody) by dilution in phosphate-buffered saline (PBS). 80 microliters of this solution were added to 10 ml of DMSO/deionized, distilled water (1:1) containing 0.1M $TBABF_4$ and 18mM ammonium persulfate in the reaction vessel. The final ruthenium-labeled antibody concentration was 5 x $10^{-8}$M. Electrochemiluminescence was measured as described in Example 2.

In an analogous manner, a solution containing 6.25 x $10^{-6}$M unlabeled rabbit, anti-mouse IgG antibody was prepared and added to the reaction vessel to give a final unlabeled antibody concentration of 5 x $10^{-8}$M. The electrochemiluminescence of this solution and of a similar solution without antibody were measured.

A solution containing 2.5 x $10^{-5}$M mouse IgG was prepared from a stock solution of mouse IgG (4.0 mg/ml) by dilution in PBS, and different aliquots (20 microliters and 40 microliters) of this solution were added to the solution of ruthenium-labeled, anti-mouse IgG antibody in the reaction vessel to give final mouse IgG concentrations of 5 x $10^{-8}$M and 1 x $10^{-7}$M, respectively.

The electrochemiluminescence of the resulting mixture of ruthenium-labeled, anti-mouse IgG antibody and the antigen (mouse IgG) was measured. The results are shown in Table VIII. The dependence of the electrochemiluminescence measurements upon the concentration of the mouse IgG antigen is shown in Figure 1. These results demonstrate a reduction in the electrochemiluminescence of the ruthenium-labeled antibody upon addition of antigen. Based upon these results one skilled in the art would know that a homogeneous electrochemiluminescent immunoassay for determining the concentration of other analytes of interest may be developed.

TABLE VIII

REDUCTION OF ELECTROCHEMILUMINESCENCE (ECL) OF RUTHENIUM-LABELED ANTIBODY UPON BINDING OF ANTIGEN

| UNLABELED ANTI-MOUSE IgG (CONTROL) | RUTHENIUM-LABELED ANTI-MOUSE IgG (ANTIBODY) | MOUSE IgG (ANTIGEN) | ECL (MV) |
|---|---|---|---|
| 0 | $5 \times 10^{-8}$M | 0 | 1610 |
| 0 | $5 \times 10^{-8}$M | $5 \times 10^{-8}$M | 1360 |
| 0 | $5 \times 10^{-8}$M | $1 \times 10^{-7}$M | 1240 |
| $5 \times 10^{-8}$M | 0 | 0 | 0 |

Example 9 - Heterogeneous Electrochemiluminescent Immunoassay for Legionella Using a Mouse Anti-Legionella Immunoglobulin G (IgG) Antibody and Ruthenium-Labeled Rabbit Anti-Mouse Immunoglobulin G (IgG) Antibody

A formalinized suspension of the bacterium Legionella micdadei was adjusted to an optical density (at 425nm) of 1.00 by dilution with PBS buffer. Approximately $3 \times 10^9$ cells were added to a conical microcentrifuge tube. The cells were centrifuged (10 minutes, 10,000 RPM), the supernatant decanted, and the cells resuspended in a 1:50 dilution of a mouse monoclonal IgG antibody, (1.45 mg/ml) specific for Legionella midadei, in PBS (1 ml). After incubation at room temperature for 1 hour, the cells were centrifuged, the supernatant decanted, the cells resuspended in PBS buffer and centrifuged again. Following decantation of the supernatant, the cells were resuspended in a 1:50 dilution (in PBS) of rabbit anti-mouse IgG antibody labeled with 4,4′ - (dichloromethyl) - 2,2′ - bipyridyl, bis (2,2′ - bipyridyl) ruthenium (II) i.e. ruthenium-labeled rabbit anti-mouse IgG antibody, (2mg/ml, 7.5 Ru/antibody). After incubation at room temperature for 1 hour, the cells were centrifuged, the supernatant decanted, and the cells resuspended in PBS and washed twice, with centrifugation, as before. Following the last wash the cells were resuspended in 200 microliters of PBS. 100 microliters of the cell suspension was added to the reaction vessel containing 10 ml of DMSO/deionized, distilled water (1:1) containing 0.1 M $TBABF_4$ and 18 mM ammonium persulfate and transferred to the reaction vessel. The electrochemiluminescence was measured for the cell suspension. Another 100 microliters of the cell suspension was added to the reaction vessel and electrochemiluminescence measured. Electrochemiluminescence was measured for the solution without cells as a control according to the method described in Example 2. The results shown in Table IX indicate a heterogeneous electrochemiluminescent immunoassay for Legionella using ruthenium-labeled rabbit anti-mouse IgG antibody has been successfully carried out.

TABLE IX

HETEROGENEOUS ELECTROCHEMILUMINESCENT (ECL) IMMUNOASSAY FOR LEGIONELLA MICDADEI

| Sample | ECL (mV) |
|---|---|
| Legionella micdadei cell suspension, 1.9 x $10^9$ cells in DMSO/$H_2O$ (1:1) | 160 |
| Legionella micdadei cell suspension, 9.3 x $10^8$ cells in DMSO/$H_2O$ (1:1) | 90 |
| DMSO: $H_2O$ (1:1) | 0 |

Example 10 - Homogeneous Electrochemiluminescent Immunoassay for Legionella Using a Mouse Anti-Legionella Immunoglobulin G (IgG) Antibody and Ruthenium-Labeled Rabbit Anti-Mouse Immunoglobulin G (IgG) Antibody

A suspension of the bacterium Legionella micdadei was prepared and incubated with a mouse monoclonal IgG antibody specific for Legionella as described in Example 9. The cells were centrifuged, washed, and resuspended in 0.2 ml of PBS. An aliquot (80 microliters) of rabbit, anti-mousse IgG antibody labeled with 4,4′ - (dichloromethyl) - 2,2′ - bipyridyl, bis (2,2′ - bipyridyl) ruthenium (II), i.e. ruthenium-labeled rabbit anti-mouse IgG antibody (1.25 x $10^{-6}$M) was added to the cell suspension, and the mixture was incubated for 2 hours at room temperature. As a control, an identical dilution of ruthenium-labeled, rabbit anti-mouse IgG antibody was incubated in the same way in the absence of the cell suspension. After the incubation period, the solution of labeled antibody was added to 10 ml of DMSO/deionized, distilled water (1:1) containing 0.1 m $TBABF_4$ and 18mM ammonium persulfate in the reaction vessel to give a final ruthenium-labeled, rabbit anti-mouse IgG antibody concentration of 1 x $10^{-8}$M. The electrochemiluminescence was measured as described in Example 2. The same procedure was followed for the cell suspension with added ruthenium-labeled rabbit anti-mouse IgG antibody. The results, shown in Table X, indicate a reduction of the electrochemiluminescent emission upon the interaction of ruthenium-labeled, anti-mouse IgG antibody with mouse monoclonal antibody bound to Legionella and that a homogeneous electrochemiluminescent immunoassay for Legionella micdadei has been successfully carried out.

TABLE X

HOMOGENEOUS ELECTROCHEMILUMINESCENT (ECL) IMMUNOASSAY FOR LEGIONELLA MICDADEI

| SAMPLE | ECL (mV) |
|---|---|
| 1 x $10^{-8}$M Ruthenium - Labeled Anti-mouse IgG Antibody | 976 |
| 1 x $10^{-8}$M Ruthenium - Labeled Anti-Mouse IgG Antibody + Monoclonal Antibody Bound To Legionella micdadei | 803 |
| DMSO: $H_2O$ (1:1) | 0 |

Example 11 - Increase in Electrochemiluminescence Upon Release of A Ruthenium-Labeled Antibody Bound to Bacteria

A formalinized suspension of the bacterium Legionella micdadei was adjusted to an optical density (at 425nm) of 1.00 by dilution with PBS buffer and 2 ml of this suspension were added to a conical microcentrifuge tube. The cells were centrifuged (10 minutes, 10,000 RPM), the supernatant decanted, and the cells were resuspended in a 1:10 dilution in PBS (0.5 ml) of a mouse monoclonal IgG antibody, (1.45 mg/ml) specific for Legionella micdadei . After incubation at room temperature for 1 hour, the cells were centrifgued as before, the supernatant was decanted, the cells were resuspended in PBS buffer and centrifuged again. Following decantation of the supernatant, the cells were resuspended in a 1:50 dilution (in PBS) of ruthenium-labeled, rabbit anti-mouse IgG antibody (1ml, 7.5 Ru/antibody). After incubation at room temperature for 1 hour, the cells were centrifuged as before, the supernatant was decanted, and the cells resuspended in PBS and washed twice, with centrifugation as before. Following the last wash the cells were resuspended in 100 microliters of either PBS or 1.0M acetic acid - 0.9%NaCl (normal saline) solution and incubated at room temperature for 40 minutes. After centrifugation, 100 microliters of the cell supernatant fluid was transferred into the reaction vessel along with 10 ml of DMSO-deionized, distilled water (1:1) containing 0.1 M $TBABF_4$ and 18 mM ammonium persulfate. The electrochemiluminescence of the acetic acid/normal saline cell supernatant fluid and for the supernatant fluid from the PBS washed cells was measured according to the method described in Example 2. The electrochemiluminescence measurements are shown in Table XI, and demonstrate that the elution of the ruthenium-labeled, rabbit anti-mouse IgG from the monoclonal antibody coated Legionella bacteria by treating the cells with 1.0M acetic anti-mouse saline (Ref.23) results in an increase in the electrochemiluminescence generated by the unbound ruthenium-labeled antibody. These results also show that the ruthenium labeled antibody is bound to the monoclonal antibody-coated Legionella, and that the PBS wash did not result in an increase in ECL in comparison to the background signal.

TABLE XI

ELECTROCHEMILUMINESCENCE (ECL) OF SUPERNATANT FLUIDS OF CELLS COATED WITH RUTHENIUM-LABELED ANTI-MOUSE IMMUNOGLOBULIN( IgG)

| Solution | ECL (mV) |
|---|---|
| Background control: 100 microliters 1.0M acetric acid-normal saline | 134 |
| 100 microliters of PBS cell wash supernatant fluid | 121 |
| 100 microliters of 1.0M acetic acid-normal saline solution cell wash supernatant fluid | 214 |

Example 12 - Homogeneous Competitive Immunoassay For Pregnane - diol - 3 Glucuronide (PD3G) In Urine

Pregnane-diol-3-glucuronide (PD3G) may be detected and quantified in urine by contacting a sample of urine with a known amount of PD3G labeled with an electrochemiluminescent moiety and a known amount of an anti-PD3G antibody under conditions such that the PD3G present in the sample and the PD3G-electrochemiluminescent moiety compete for binding sites on the antibody. After a suitable time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of PD3G present in the sample determined therefrom.

Example 13 - Labeling Nucleic Acids with a tris-Ruthenium bipyridyl-n-hydroxysuccinimide ester

Nucleic acid samples (5 to 25 micrograms) in 10mM tris hydrochloride (pH 8.0)-1mM EDTA may be heat denatured, cooled and modified by bisulphite catalysed transamination of cytosine residues with ethylenediamine for three hours at 42°C. After overnight dialysis against three changes of 5mM sodium phosphate buffer, pH 8.5, the samples may be concentrated to 100 microliters by ultrafiltration. These modified nucleic acids (1 to 10 micrograms) may be diluted in 100 microliters with 0.1 M sodium phosphate buffer, pH 8.5.

A tris-ruthenium bipyridyl-N-hydroxysuccinimide ester derivative may be prepared as a 0.2M stock solution in N, N′ dimethylformamide (DMF) by methods known in the art. 5 microliters of this ester solution may be added to the modified nucleic acid solution in 0.1 M sodium phosphate buffer, pH 8.5 and incubated at room temperature for 1 hour. Labeled nucleic acid probes may be purified by dialysis against at least three changes of 150mM sodium chloride - 10mM sodium phosphate buffer (pH 7.0) stored at 4°C until used.

Example 14 - Nucleic Acid Hybridization Assay for Human T-Cell Leukemia III Virus (HTLV-III) in Blood

HTLV-III virus may be detected in whole blood by treating the blood to release the RNA from virus particles. A sample containing the HTLV-III RNA is contacted with a single-stranded oligonucleotide probe complementary to the HTLV-III RNA and labeled with an electrochemiluminescent moiety. After a suitable time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of HTLV-III RNA present in the sample determined therefrom.

Example 15 - Homogeneous Nucleic Acid Hybridization Assay for Legionella Bacteria in a Clinical Sample

Legionella bacteria may be detected in a clinical sample such as sputum by treating the sputum to release the ribosomal RNA from the bacteria. The resulting sample is contacted with a single-stranded oligonucleotide probe that is complementary to sequences in the ribosomal RNA, specific for Legionella, and labeled with an electrochemiluminescent moiety. After a suitable time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of Legionella Bacteria present in the sample determined therefrom.

Example 16 - Hybridization Assay for the Detection of Cytomegalovirus DNA Integrated into Genomic DNA by Strand Displacements Method

Cytomegolavirus (CMV) DNA may be detected in human genomic DNA by treating a tissue sample, for example lymphocytes, to release the DNA and cleaving the DNA with restriction enzymes. The resulting sample containing double-stranded fragments of CMV is contacted with a single-stranded oligonucleotide probe that is complementary to the CMV DNA, labeled with a electrochemiluminescent moiety and capable of displacing one of the strands of the DNA duplex. After a suitable time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of Cytomegalovirus RNA present in the sample determined therefrom.

Example 17 - Hydrization Assay for the Detection of the Ras-Oncogene in Human Bladder Carcinoma Cells using a Heterogeneous Method

The ras-oncogene may be detected in human bladder carcinoma cells by treating a tissue sample to release the DNA, cleaving the DNA with restriction enyzmes, melting the DNA to single strands and binding to nitrocellulose paper as previously described in Maniatis, T. et al. (24). The filter paper with bound single-stranded DNA is contacted with an oligonucleotide probe specific for the ras-oncogene, and labeled with a electrochemiluminescent moiety. After a suitable reaction time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be detected and the presence of ras-oncogene RNA in the sample determined.

Example 18 - An Electrochemiluminescent Polymer - Based Enzyme Assay

A macromolecular enzyme substrate, for example starch, dextran or a synthetically prepared macromolecular polymer, may be labeled with an electrochemiluminescent moiety. The labeled substrate may be used to determine and quantify the presence of an enzyme present in a multicomponent system. The labeled substrate may also be used to quantify an amount of enzyme linked to an antibody, DNA probe, RNA probe, streptavidin, avidin, or biotin. The labeled substrate may be cleaved into smaller fragments selectively by an appropriate enzyme. Any enzyme-substrate combination may be used. Examples of enzymes include glycosidases, glucosidases, amylases, proteases, endonucleases, lyases and dextranases. After a suitable time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of enzyme present in the sample determined therefrom. The advantage is that an amplification of the electrochemiluminescent signal will result from the cleaved fragments each labeled with electrochemiluminescent moiety.

Example 19 - Detection of Streptococci by an Electrochemiluminescent Enzyme Assay

A sample containing streptococci may be contacted with a reagent mixture containing a synthetic peptide labeled with an electrochemiluminescent moiety. The synthetic peptide being a substrate specific for a peptidase produce by the bacteria. Action of the peptidase on the synthetic peptide results in the production of fragments labeled with the electrochemiluminescent moiety. After a suitable time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of streptococci present in the sample determined therefrom.

Example 20 - Enzyme Immunoassay for Hepatitis B Surface Antigen Based On Electrochemiluminescence

A blood sample containing Hepatitis B surface antigen (HBsag) is contacted for a suitable time with an antibody specific for the HBsag and labeled with dextranase. The antibody not bound to HBsag is removed and the antigen-antibody complex is contacted with a dextran polymer labeled with an electrochemiluminescent moiety. Action of the dextranase on the labeled polymers will result in the production of fragments each labeled with the electrochemiluminescent moiety. After a suitable time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of Hepatitis B surface antigen present in the sample determined therefrom.

Example 21 - Homogeneous Assay for Bradykinin receptors using an Electrochemiluminescent-labeled Bradykinin

A tissue sample containing bradykinin receptors is suitably prepared and contacted with bradykinin labeled with an electrochemiluminescent moiety. After a suitable amount of time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of bradykinin receptor present in the sample determined therefrom. This assay could also be used for measuring other ligand-receptor interactions such as estrogen-estrogen receptor. In addition this assay may be used to determine and quantify the amount of unlabeled ligands using a competitive binding assay format.

Example 22 - Use of an Electrochemiluminescent moiety for Detecting Nucleic Acid Hybrids

A sample containing nucleic acid hybrids, such as double-stranded DNA, DNA-RNA duplexes, or double-stranded RNA is contacted with an electrochemiluminescent moiety that specifically intercalates into nucleic acid hybrids. After a suitable amount of time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of nucleic acid hybrids present in the sample determined therefrom.

Example 23 - Detection of Human T-Cell Leukemia Virus III (HTLV-III) Antigen Complexed to Antibody in Saliva by a Homogeneous Immunoassay using Electrochemiluminescence

A saliva sample containing HTLV-III complexed to antibody is contacted with a solution containing a chaotopic agent to disrupt the antigen-antibody complexes. This solution is then contacted with an antibody specific for HTLV-III and labeled with an electrochemiluminescent moiety. The chaotopic agent is removed allowing the labeled and unlabeled antibodies to recombine with antigen. After a suitable amount of time the resulting sample may be induced to repeatedly emit electromagnetic radiation upon direct exposure to an electrochemical energy source effective for inducing the electrochemiluminescent moiety to repeatedly emit electromagnetic radiation. Emitted radiation may be quantified, and the amount of human T-cell leukemia virus III (HTLV-III) antigen present in the sample determined therefrom. These methods are applicable to samples containing other types of antigen-antibody complexes such as hepatitis antigen-antibody complexes, cytomegolavirus-antibody complexes, and non-A, non-B hepatitis-antibody complexes in serum.

Example 24 - Preparation of 2-[3-(4-methyl-2,2′-bipyridine-4′-yl)-propyl]-1,3-dioxolane

Under an inert atmosphere of argon, 30 ml of dry tetrahydrofuran (THF) and 7.65 ml of dry diisopropylamine (54.6 mmol) were added to a 3-neck, 600 ml flask via syringe with stirring. The solution was cooled to -78°C by immersing the flask in a mixture of dry ice-isopropanol in a low form beaker. 21.6 ml of 2.5M n-butyl lithium (54 mmol) were slowly added to the flask. The resulting solution was stirred for 15 min and a solution of 9.58 g of 4,4′-dimethyl-2,2′-bipyridine (52 mmol) dissolved in 300 ml of dry THF was added dropwise by cannula with stirring over 1 hr.

The resulting brown mixture was further stirred at -78°C for 2 hrs, 10 g of 2-(2-bromoethyl)-1,3-dioxolane (55 mmol) were added by syringe and the resulting mixture stirred at -78°C for 5 min. The reaction vessel was then placed in an ice bath (10°C) and after 30 min began to change color (after 1 hr the color was dark violet; after 2 hrs the color was blue; after 2.5 hrs the color was green; and after 3.25 hrs the color was lemon yellow.

The reaction mixture was quenched with 30 ml of saturated NaCl followed by 10 ml of water and 50 ml of ether. The aqueous phase was extracted twice with 300 ml of ether and the combined ether phases were backextracted with 100 ml of water and dried over anhydrous sodium sulphate.

To purify the reaction product, the sample was separated on alumina (Merck) 90, activity III, neutral. The eluants used were petroleum ether/diethyl ether (2:1) followed by petroleum ether/diethyl ether (1:1) (the starting material elutes completely with petroleum ether/diethyl ether (2:1) , followed by the product).

Proton NMR analysis confirmed that the structure of the isolated reaction product is

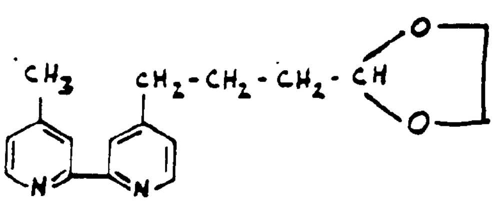

Example 25 - Preparation and Purification of 4-(butan-1-al)-4′-methyl-2,2′ bipyridine

2 g of 2-[3-(4-methyl-2,2′-bipyridine-4′-yl)-propyl]-1,3 dioxolane were dissolved in 50 ml of 1N HCl and heated for 2 hrs at 50°C. The solution was cooled, adjusted to between pH 7 and 8 with sodium bicarbonate and extracted twice with 100 ml of chloroform.

The combined chloroform phases were washed with a small amount of water, dried over sodium sulfate, filtered and rotoevaporated to yield a yellow oil.

The yellow oil was purified on a silica gel column using ethyl acetate/toluene (1:1) as the eluant, the impurity being eluted with methanol.

Proton NMR analysis [δ 1·96-2·11 (m,2H); 2·43 (s,3H); 2·46-2·50) (t,2H); 2·53-2·80 (m,2H); 7·12-7·14 (m,2H) ; 8·17-8·21 (br. s,2H); 8·52-8·58 (m,2H); 9·89 (s,1H)] confirmed that the structure of the reaction product is

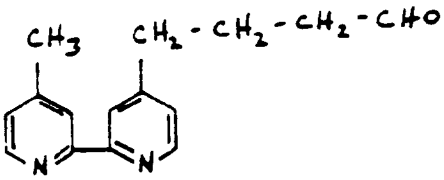

Example 26 - Preparation of 4-(4-methyl-2,2'-bipyridine-4'-yl) butyric acid

0.5 g of 4-(butan-1-al)-4'-methyl-2,2'-bipyridine (2.0 mmol) were dissolved in 10 ml absolute acetone. 225 mg of finely powdered potassium permanganate ($KMnO_4$; 1.42 mmol) were added in portions to the solution with stirring. The reaction was followed by thin layer chromatography, (silica; ethyl acetate/toluene 50:50), which indicated that while the aldehyde gradually disappeared bipyridine of low $R_f$ was formed.

After the reaction reached completion, water was added and the $MnO_2$ was filtered and washed with small portions of $Na_2CO_3$(aq.). The acetone was rotoevaporated and the residue extracted with $CH_2Cl_2$ to remove non-acidic bipyridines. The aqueous solution was made acidic by careful addition of 1.0N HCl to pH 4.8. The solution became partially cloudy upon reaching this pH, the suspension redissolving at lower pH. The mixture was extracted five times with equal volumes of $CH_2Cl_2$, dried over $Na_2SO_4$ and rotoevaporated to an oil which promptly soldified in vacuo. The crude solid was recrystallized from chloroform: petroleum ether to obtain white crystals.

Melting point: 103.5°C-105.5°C; IR: 1704 $cm^{-1}$. Proton NMR analysis was consistent with the following structure

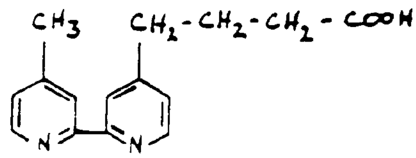

Example 27 - Preparation of bis(2,2'bipyridine) [4-(butan-1-al)-4'-methyl-2,2'-bipyridine]ruthenium (II) diperchlorate: Compound I

250 mg of ruthenium bipyridyl dichloride dihydrate (0.48 mmol) (Strem) in 50 ml of ethylene glycol were quickly heated to boiling and then immersed in a silicone oil bath (130°C). To the resulting purple-orange solution were added 150 mg of 2-[3-(4-methyl-2,2'-bypyridine-4'-yl)propyl]-1,3-dioxolane (0.53 mmol) in 10 ml of ethylene glycol. The resulting orange solution was stirred at 130°C for 30 min, cooled to room temperature and diluted 1:1 with distilled water.

A concentrated solution of sodium perchlorate in water was added to the solution, causing the appearance of a very fine orange precipitate. The mixture was refrigerated overnight, filtered and the precipitate washed with water.

The precipitate was dissolved in hot water and recrystallyzed by adding perchloric acid to the solution to produce bright orange crystals which were then filtered, washed with cold water and dried. This recrystallization procedure was repeated, yielding a total of 150 mg of bright orange crystals.

NMR analysis indicated the following structure

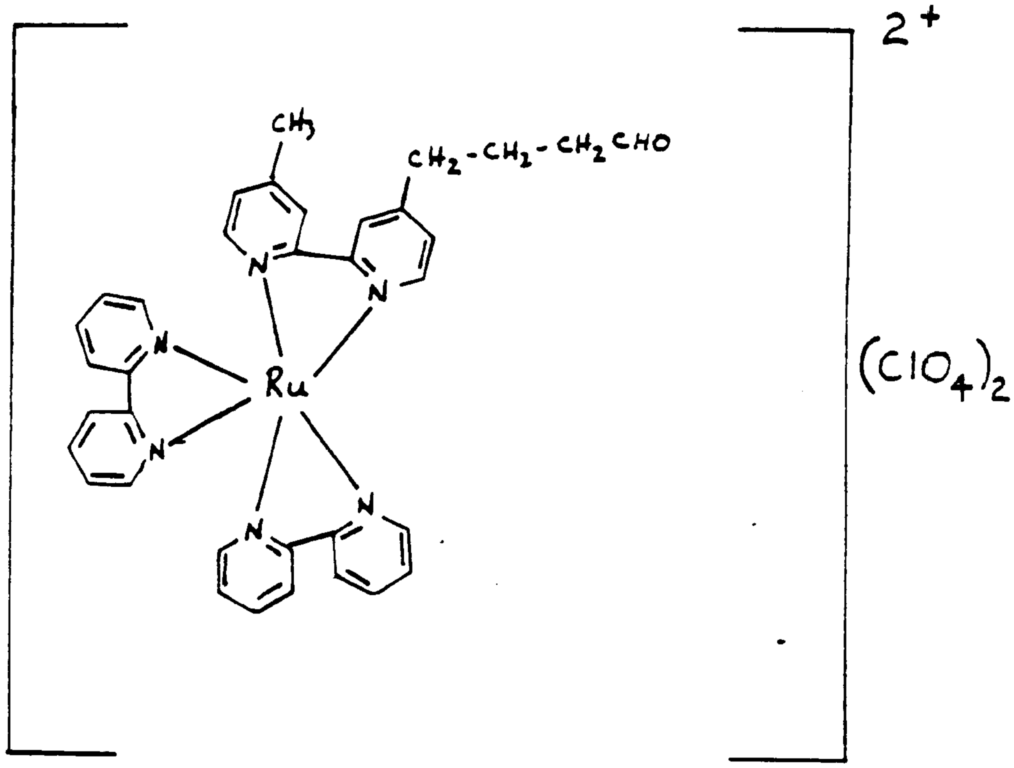

I

Within the present application, the above-identified compound is referred to as Compound I.

<u>Example 28 - Preparation of bis(2,2′-bipyridine) [4-(4-methyl-2,2′-bipyridine-4′-yl)-butyric acid] ruthenium (II) dihexafluorophosphate: Compound II</u>

134 mg of 4-(4-methyl-2,2′-bipyridine-4′-yl)-butyric acid (0.52 mmol) were dissolved in 50 ml of water. The solution were degassed with argon and 250 mg of ruthenium bipyridyl dichloride dihydrate (Strem) (0.48 mmol) were added. The mixture was refluxed under argon for 4 hrs. The water was rotevaporated and the residue redissolved in the minimum amount of water and loaded onto a SP-25-Sephadex ion-exchange column. After eluting impurities with water, the compound was eluted as a red band with 0.2 M NaCl solution and isolated as a hexafluorophosphate by addition of a saturated aqueous solution of $NH_4PF_6$. The crude product was reprecipitated twice from hot acetone with diethyl ether. Anal : Calculated; C, 43.80%; H, 3.36%; N, 8.76%. Found; C, 43.82%; H, 3.54%; N, 8.55%.

Proton NMR analysis was consistent with the following structure

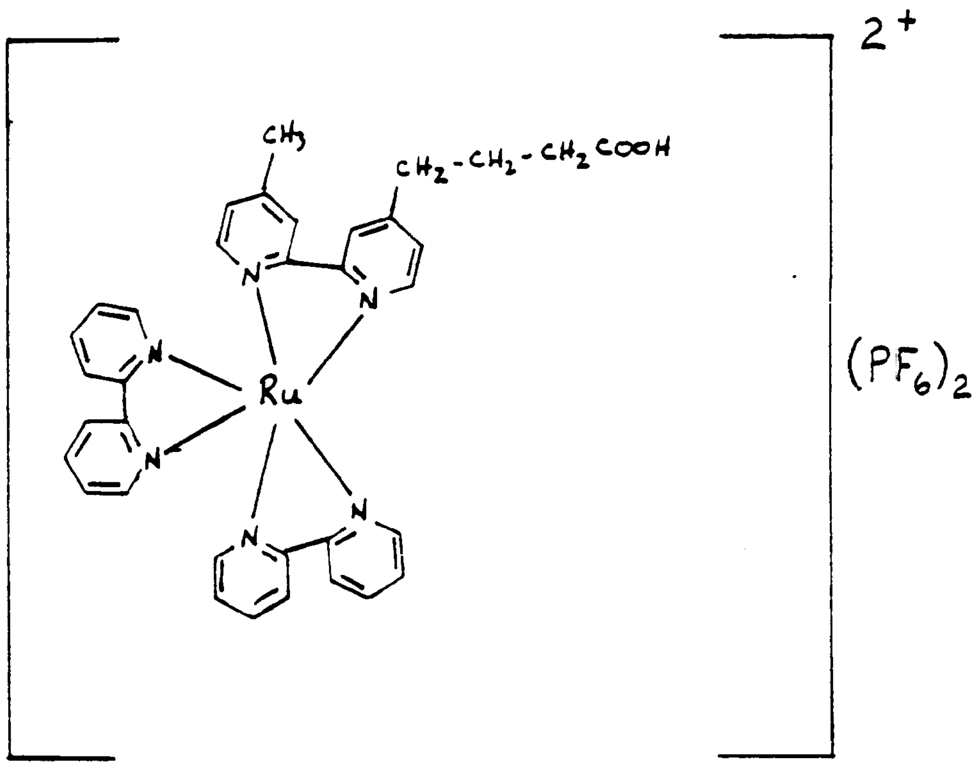

II

Example 29 - Preparation of N-[4-(4-methyl-2,2′-bipyridine-4′-yl)-butyl]-phthalimide

Under an inert atmosphere of argon, 30 ml of dry tetrahydrofuran (THF) and 7.65 ml of dry diisopropylamine (54.6 mmol) were added to a 3-neck, 600 ml flask via syringe with stirring. The solution was cooled to -78°C by immersing the flask in a mixture of dry ice-isopropanol in a low form beaker. 21.6 ml of 2.5 n-butyl lithium (54 mmol) were slowly added to the flask. The resulting solution was stirred for 15 min and a solution of 9.58 g of 4,4′-dimethyl-2,2′-bipyridine (52 mmol) dissolved in 300 ml of dry THF was added dropwise by cannula with stirring over 1 hr.

The resulting brown mixture was further stirred at -78°C for 2 hrs, 100 g of 1,3-dibromopropane (0.495 mol) were rapidly added and the resulting mixture stirred at -78°C for 1 hr. The mixture was then stirred at room temperature for 2 more hrs. The color of the mixture changed from brown to blue to yellow. Most of the solvent was rotoevaporated and 200 ml of water were added causing the formation of two phases. Concentrated HCl was added to lower the pH to 0. The organic layer was discarded. The aqueous layer was washed twice with 100 ml of ether. The pH was raised to between 1 and 2. A red oil separated and was extracted into $CH_2Cl_2$. After drying the solution with anhydrous $Na_2CO_3$, most of the $CH_2Cl_2$ was rotoevaporated and the sample was loaded onto a silica gel column. The sample was eluted with chloroform yielding a light yellow oil which crystallized after cooling overnight (12.37 g).

4g of the crude 4-(4-bromobutyl)-4′-methyl-2,2′-bipyridine (13.3 mmol) thus prepared were then added to a suspension of 2.46g potassium phthalimide (13.3 mmol) in 60 ml of dimethylformamide (DMF). The mixture was then stirred at approximately 50°C for 2 hrs. 90 ml of $CHCl_3$ were added to the mixture, followed by 125 ml of water. The $CHCl_3$ layer was separated and the aqueous layer was extracted twice with 50 ml of chloroform. The combined $CHCl_3$ layers were washed with 50 ml of $H_2O$, dried over anhydrous $Na_2SO_4$, and rotoevaporated, leaving a pale orange oil which solidified overnight. The crude product was recrystallized from acetone/ethanol, yielding 2.21 g (44.5%) of white crystals (melting point, 114.5-117.8°C). Anal:Calulated; C, 74.38%; H, 5.70%; N, 11.31%. Found: C, 73.98%; H, 6.14%; N, 11.28%. IR: Phthalimide carbonyl stretch 1771 and 1704 $cm^{-1}$. Proton NMR analysis showed aromatic resonances (δ7·75-7·85, m, 4H) in addition to the usual bipyridyl derivative signals. These data are consistent with the following structure

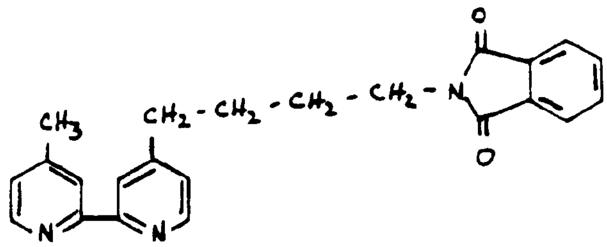

Example 30 - Preparation of theophylline-8-butyric-[4- (4-methyl-2,2′-bipyridine-4′-yl)-butyl]amide: Compound III

370 mg (1 mmol) of N-[4-(4-methyl-2,2′-bipyridine-4′-yl)-butyl]-phthalimide were slurried in 10 ml ethanol, treated with hydrazine hydrate (720 mg, 1.4 mmol), stirred and refluxed for 4 hrs, during which time all the solid went into solution. Towards the end of the reaction a white precipitate began to form.

After cooling, the reaction mixture was made basic with 50% NaOH and then poured into 100 ml of water. A solution resulted and $Na_2CO_3$ was added to salt out the product as an oil. The amine was extracted using three 40 ml portions of $CH_2Cl_2$. The extracts were dried with $CaSO_4$, filtered and evaporated to give the aminobipyridine derivative as a colorless oil (yield = 0.135 gm; 56%).

1.34 g (5.6 mmol) of 4-[4-(1-aminobutyl)]4′-methyl-2,2′-bipyridine and theophylline-8-butyric acid (1.18 g; 4.4 mmol) were dissolved at room temperature in 10 ml of dry pyridine. To the solution were added 1.16 g (5.6 mmol) of dicyclohexylcarbodiimide. Stirring at room temperature was continued overnight. Thin layer chromatography on alumina (mobile phase = 15% methanol/chloroform) revealed one product spot with an $R_f$ of 0.68. Precipitated dicyclohexylurea was removed by filtration and the pyridine was stripped to give a solid, which was triturated in ether and filtered (yield = 2.13 g; 99%).

Example 31 - Preparation of bis-(2,2′-bipyridine)[theophylline-8-butyric-4-(4-methyl-2,2′-bipyridine-4′-yl)-butyl amide] ruthenium (II) dichloride: Ru(II)-Compound III Conjugate

To a mixture of 154 mg (0.296 mmoles) of bis-(2,2′-bipyridine) ruthenium (II) dichloride dihydrate and 175 mg (0.357 mmoles) of Compound III described above were added 40 ml of ethanol/$H_2O$ (1:1, v/v). This mixture was argon degassed in the dark for 15 min and refluxed in the dark under argon for 3 hrs to produce a clear, cherry red solution. The resulting clear, cherry red solution was allowed to cool to room temperature and the solvent was stripped off using a rotary evaporator while maintaining the solution in the dark under a temperature less than or equal to 37°C.

The resulting residue was dissolved in approximately 1-3 ml of methanol, loaded onto a Sephadex LH-20 chromatography column (75 cm x 3 cm) and eluted at a flow rate of about 0.4-0.7 ml/min. A bright red band (product) was closely followed by a brown, nonluminescent band (impurity) and two luminescent bands. The red product band was found to be contaminated with a small amount of the material from the brown, nonluminescent band. This contaminating material was separated from the product by running the sample on a second Sephadex LH-20 column under similar conditions.

The red product was obtained by stripping the solvent off by rotoevaporation. The resulting solid material was dissolved in approximately 1 ml of methanol and reprecipitated in approximately 75 ml of diethyl ether to yield an orange powder which was collected by filtration.

Anal: Calculated; C, 54.51%; H, 5.72%; N, 13.99%; O, 10.47%; Cl 6.44. Found; C, 55.04%; H, 6.35%; H, 13.18%; O, 10.62%; Cl, 6.68.

Example 32 - Modulation of Electrochemiluminescent Signal Generated By Ru(II)-Compound III Conjugate Using Antibodies Specific For Theophylline

The Ru(II)-Compound III Conjugate described in Example 33 was diluted to a final concentration of 150 nM using 0.1M phosphate buffer, pH 6.0, containing 0.35 M sodium fluoride (PBF Buffer). Monoclonal antibody (clone number 9-49, ascites lot number WO399, cat number 046) specific for theophylline was obtained from Kallestad Laboratories, Inc. (Chaska, MN). The monoclonal antibody was diluted to different concentrations using PBF Buffer (between 21.9 micrograms of protein/ml to 700 micrograms/ml).

Another monoclonal antibody (control MAB) that was not reactive with theophylline was obtained from Sigma (St. Louis, MO) and was diluted to different concentrations between 21.9 micrograms of protein/ml to 700

micrograms/ml using PBF Buffer. A standard solution of theophylline was prepared using theophylline obtained from Aldrich Chemical Co., (Milwaukee, WI, cat number 26-140-8, M.W. 180.17). Theophylline was dissolved in PBF Buffer to give a final concentration of 75 micromolar and was diluted with PBF Buffer to 6 micromolar for use in assays. Prior to making electrochemiluminescence measurements a solution containing 250 mM oxalic acid and 5% (v/v) Triton-X 100 (ECl solution) was added to the reaction mixture. Measurements were made using a Berthold luminometer that was modified to allow the placement of two platinum gauze electrodes into the test tube containing the reaction solution. The electrodes were connected to a potentiostat and the electrochemiluminescence measurement was made by sweeping an applied potential across the electrodes from 1.5 to 2.5 volts at a scan rate of 50 mV/sec. The Berthold luminometer used for the measurement had a high gain, red sensitive photomultiplier tube. The luminometer output to the recorder was adjusted to $10^5$ counts/volt. The measurements were recorded on an X-Y-Y′ recoder and the peak height was used as the measurement of electrochemiluminescence. The electrodes were cleaned between measurements by rinsing with a buffer at pH 4.2 containing 0.1 M phosphate, 0.1 M citrate, 0.025 M oxalic acid, and 1% Triton X-100; pulsing the electrodes in this solution between +2.2 to -2.2 volts for 60 sec; and followed by +2.2 volts for 10 seconds. Next the electrodes were removed from this solution, rinsed in distilled water and wiped dry. The experiment was carried out as outlined in Table XII.

A solution of control monoclonal antibodies, antibodies to theophylline of PBF Buffer was added to a set of test tubes (Step 1). To the tubes, a solution of theophylline or PBF Buffer was added (Step 2). The solutions were mixed by briefly shaking the test tubes and allowed to react for 25 min at room temperature. Then a solution of Ru(II)-Compound III Conjugate was added to the tubes (Step 3). The test tubes were shaken and kept at room temperature for 15 min. Finally, 100 microliters of the ECL solution was added to each tube and electrochemiluminescence was measured as described above. The results are listed in Table XIII.

Table XII

Experimental Design for Studying the Effect of Antibody- Ru(II)-Compound III Conjugate Interactions on Electrochemiluminescence

| Step I 100 microliters of: | Step 2 200 microliters of: | Step 3 100 microliters of: |
|---|---|---|
| A. Control monoclonal antibody (2.19 micrograms to 70 micrograms) | Buffer | Ru(II)-Compound III Conjugate |
| or | | |
| B. Anti-theophylline antibody (2.19 micrograms to 70 micrograms) | Buffer or Theophylline | Ru(II)-Compound III Conjugate |
| or | | |
| C. PBF Buffer | Buffer | Ru(II)-Compound III Conjugate or Buffer |

TABLE XIII

Effect of Antibody and Theophylline on Electrochemiluminescence of Ru(II)-Compound III Conjugate

| Antibody Protein Concentration (micrograms/tube) | Control MAB + Ru(II)-Compound III | Anti-Theophylline MAB + Ru (II)-Compound III | Anti-Theophylline MAB + Theophylline Ru(II)-Compound III |
|---|---|---|---|
| | Luminescence Measurement | | |
| 2.19 | 55,000 | 40,000 | 43,000 |
| | 55,000 | 41,000 | 57,000 |
| 4.38 | 57,000 | 22,500 | 37,000 |
| | 57,000 | 25,000 | 36,000 |
| 8.75 | 53,000 | 20,000 | 33,500 |
| | 50,000 | 22,000 | 30,500 |
| 35.0 | 43,000 | 13,500 | 17,500 |
| | 41,000 | 14,000 | 16,000 |
| 70.0 | 42,000 | 11,000 | 11,000 |
| | 37,500 | 12,000 | 12,500 |

The electrochemiluminescence of duplicate samples were measured as described above. The electrochemiluminescence of Ru(II)-Compound III Conjugate used in the above study was 57,200 when measured in buffer without the addition of antibody. The background for the buffer mixture was 5750.

The data show that a monoclonal antibody which specifically recognizes theophylline, when contacted with an analog of theophylline to which a ruthenium compound is attached e.g., Ru(II)-Compound III, will decrease the electrochemiluminescence. The decrease in electrochemiluminescence is proportional to the antibody concentration when the Ru (II)-Compound III Conjugate concentration is held constant. When an antibody is used which does not react with theophylline, only a slight decrease in the electrochemiluminescence is seen at the highest concentration of antibody.

The data also show that when theophylline is contacted with the anti-theophylline antibody and then the Ru(II)-Compound III Conjugate is added to the mixture, the amount of electrochemiluminescence is greater. This demonstrates that theophylline competes for the binding of antibody to Ru(II)-Compound III Conjugate resulting in a greater amount of Ru(II)-Compound III Conjugate which can generate electrochemiluminescence.

Example 33 - Assay for Theophylline in Serum Based on a Homogeneous Electrochemiluminescent Immunoassay

Based on the results described Example 34, a homogeneous immunoassay for theophylline was developed using antibody to theophylline and the Ru(II)-Compound III Conjugate described in Example 31 in a competitive binding format. The materials used were described in Example 34 except the PBF buffer was 0.1M phosphate buffer, pH 6.0, containing 0.1M sodium fluoride. For this assay, a specific concentration of monoclonal antibody to theophylline was chosen. The antibody concentration was 55 micrograms/ml. The Ru(II)-Compound III Conjugate concentration was adjusted to 175 nM. Theophylline was added to human serum to give final concentrations of 2.5, 5, 10, 20 and 40 micrograms of theophylline/ml of serum.

The assay was performed by adding 10 microliters of serum to 290 microliters of anti-theophylline monoclonal antibody and holding the solution at room temperature for 25 min. Then 100 microliters Ru(II)-Compound III Conjugate were added to each tube to give a final concentration of 35 nM and holding this solution at room temperature for 15 min. 100 microliters of the ECL solution described in Example 34 were then added to each tube and electrochemiluminescent properties of the solutions were measured as previously described using

a sweep mode for 1.5 volts to 2.5 volts at 50 mV/sec. The data are shown in Figure 2 and demonstrate that there is a correlation between the concentration of theophylline in a serum sample and the amount of electrochemiluminescence that is emitted by the reaction mixture. This observation demonstrates that it is possible to develop an assay for theophylline.

Based on these results, one skilled in the art would be able to develop a homogeneous electrochemiluminescence immunoassay for detecting and quantifying an analyte of interest in a biological matrix.

Example 34 - Assay for Theophylline in Hemolyzed, Lipemic, Icteric and Normal Serum Samples Based on a Homogeneous Electrochemiluminescent Immunoassay and Comparison to a Fluorescence Polarization Assay

The concentration of theophylline in different types of serum samples was determined using a homogeneous electrochemiluminescent immunoassay. The format for the assay was a competitive binding assay using a monoclonal antibody specific for theophylline and the Ru(II)-Compound III Conjugate described in Example 31. The reagents and methods for electrochemiluminescence are described in the previous example.

The fluorescence polarization assay used to measure the concentration of theophylline in the different serum samples was carried out using an automated TDX instrument from Abbott Laboratories (North Chicago, IL). Hemolyzed, lipemic, icteric and normal sera were used in the assays and data for the abnormal sera are listed in Table XIV below.

Table XIV

Homogeneous Theophylline Assay
Characteristics of Potentially Problematic Sera

| Serum | Factor Concentration | Normal Range |
|---|---|---|
| Hemolyzed | 12.4 mg/dl hemoglobin | 0-3.2 mg/dl |
| Lipemic | 405 mg/dl triglycerides<br>196 mg/dl cholesterol | 10-190 mg/dl<br>120-200 mg/dl |
| Icteric | 10 mg/dl bilirubin | 0-1.5 mg/dl |

Different amounts of theophylline were added to the serum samples to give final concentrations between 2.5 micrograms theophylline/ml and 40 micrograms theophylline/ml. The results for the homogeneous electrochemiluminescent immunoassay are displayed in Figure 3.

Each serum sample was also analyzed for the concentration of theophylline by a fluorescence polarization assay. The concentration of theophylline measured by the homogeneous electrochemiluminescence immunoassay and the fluorescence polarization assay were compared. The data were plotted as a scattergram and are shown in Figures 4A-D. The data points were analyzed by linear regression and the correlation coefficients were calculated. The analysis demonstrates an excellent correlation between the two assays. The correlation coefficients (r) were between 0.98 and 1.00. The slopes of the curves for normal, hemolyzed, and lipemic serum samples were between 0.8 and 1.2, demonstrating excellent recovery of theophylline from these serum samples.

Although the electrochemiluminescence emitted by the icteric serum samples containing theophylline was higher than for the other serum samples, it was proportionally higher at each theophylline concentration. This can be seen in Figure 4D. The correlation coefficient is 1.00 for the data points comparing electrochemiluminescence and fluorescence polarization; however, the slope is 2.14, demonstrating higher recovery for the theophylline in the icteric serum sample.

Based on these results, the concentration of theophylline in an icteric sample may be determined by establishing a standard curve for the sample by adding known amounts of the Ru(II)-Compound Conjugate to aliquots of the icteric serum. These data demonstrate that a homogeneous electrochemiluminescent immunoassay may be used to measure the concentration of theophylline present in serum samples containing abnormal levels of hemoglobin, lipid and bilirubin.

A homogeneous electrochemiluminescent immunoassay offers advantages over a fluorescence polarization method because of the versatility of ECL detection, e.g., more sensitive detection at higher concentrations of biological molecules.

A homogenous electrochemiluminescent immunoassay offers further advantages over a fluorescence polarization method because no incident light source is required; electrical excitation being the only requirement for efficient light-generation. Consequently, no sophisticated optics are necessary. Since the measurement principle is purely specific photon emission induced by electrochemical stimulation, the sensitivity of the system is potentially much greater than fluorescence polarization and a wider dynamic range will be achievable. Also, measurement of a much greater variety of analytes is possible with a homogeneous electrochemiluminescent immunoassay than is provided by the fluorescence polarization technique, due to the selective modulation of electrically-stimulated chemiluminesence by biomolecular recognition events, e.g., antibody-antigen interactions.

Based on these results, one skilled in the art would know that homogeneous electrochemiluminescent immunoassays for detecting other analytes of interest in abnormal serum samples may be developed.

Example 35 - Assay for Theophylline in Serum Based on a Homogeneous Electrochemiluminescence Immunoassay and Comparison to a High Pressure Liquid Chromatographic (HPLC) Method

Different amounts of theophylline were added to human serum samples to give final concentrations between 2.5 micrograms theophylline/ml and 40 micrograms theophylline/ml. Each sample was then divided into two aliquots and the concentration of theophylline in the sample was determined by a homogeneous electrochemiluminescence immunoassay and compared to the results obtained for the same serum samples using an HPLC method. The format for the homogeneous electrochemiluminescence immunoassay was a competitive binding assay using a monoclonal antibody specific for theophylline and the Ru(II)-Compound III Conjugate. The reagents and methods for this assay are described in a previous example. The HPLC method used to measure the concentration of theophylline in different serum samples is described as follows.

Theophylline (1,3-dimethylxanthine) was separated from serum proteins by precipitation of the latter with acetonitrile. The supernatant fluid containing theophylline was run on an HPLC system equipped with a Waters Associates Micro Bondapak C18 column, (3.9 mmx30 cm). The chromatogram was completely resolved in less than 10 min.

The following reagents were used: sodium acetate (reagent grade), deionized water (purified by the Millipore Milli Q• system), acetonitrile (HPLC grade) and theophylline standard, (Sigma). The solvent used for precipitating the serum proteins was a 20 mM sodium acetate buffer, pH 4.0, containing 14% (v/v) acetonitrile. The HPLC mobile phase buffer was 10 mM sodium acetate buffer, pH 4.0, containing 7% (v/v) acetonitrile. The flow rate was 1.5 ml/min, and the eluant was monitored by a UV spectrophotometer set at 270 nm. The sensitivity of the UV absorbance detector was set at 0.02 Absorbance Units Full Scale (AU FS). The ambient temperature ranged typically between 22°C and 24°C.

The results for the homogeneous electrochemiluminescent immunoassay and the HPLC assay for determining the concentration of theophylline in serum are shown in Figure 5. The data were plotted as a scattergram and the data points were analyzed by linear regression. The correlation coefficient was calculated. The correlation coefficient (r) was 0.98, which demonstrates excellent correlation between the two assays.

The slope of the curve was 1.197, demonstrating excellent recovery of the theophylline from the serum sample for the homogeneous electrochemiluminescent immunoassay compared to a standard method based on HPLC. The homogeneous electrochemiluminescence assay offers advantages over the HPLC method because of the speed, sensitivity and ability to easily handle multiple samples. Based on these results, one skilled in the art would know that homogeneous electrochemiluminescent immunoassays for detecting analytes of interest, which may be detected by HPLC and similar methods, may be developed.

Example 36 - Preparation of Theophylline-Bovine Serum Albumin Conjugates

Theophylline-bovine serum albumin (BSA) conjugates were prepared from theophylline-3-methyl-butyric acid, theophylline-8-butyric acid and theophylline-7-acetic acid by the following procedure. 50 mg of BSA were dissolved in 1.5 ml of 0.15M $NaHCO_3$, pH 9.0. Separately, 16 mg of ethyl 3′3-dimethyl amino propyl carbodiimide hydrochloride (EDCI), 11 mg N-hydroxy succinimide (NHS) and either 17.8 mg of theophylline-7-acetic acid, 20 mg of theophyllic-8-butyric acid or 20.9 mg of theophylline-3-methyl-butyric acid dissolved in dimethylsulfoxide were added and the solution heated at 45°C for 1-2 hrs. The solution was added to the BSA solution dropwise and allowed to react for 1 hr. Theophylline conjugates of BSA were purified by gel filtration chromatography on a Sephadex G-25 column (1.6 cm x 38 cm) using 0.15 M PBS/0.1% azide, pH 7.4, as the mobile phase and a flow rate of 30 ml/hr.

Example 37 - Preparation of Theophylline-BSA Biomag• Particles

A 4 ml volume of Biomag•-amine particles (Advanced Magnetic, Inc., Cambridge, MA) was washed 2-3 times in separate T-flasks with 20 ml of phosphate buffered saline (Sigma) (PBS), pH 7.4, containing 0.008% Nonidet P-40 (NP-40). To the Biomag• wet cake, 10 ml of 5% glutaraldehyde (Sigma) in PBS was added and activation was allowed to proceed for 3 hrs using a rotary mixer. The activated Biomag• particles were washed as described above for a total of 4 washes and transfered to a T-flask.

6.8 mg of theophylline-BSA prepared as described in Example 38 in 10 ml of PBS/NP-40 were added to the activated Biomag• wet cake. The reaction was allowed to proceed overnight at 4°C with mixing.

The activated Biomag• wet cake was washed 3 times with 20 ml of 1% BSA/0.15M PBs/0.1% azide (pH 7.4), the first wash lasting for approximately 30 min using a rotary mixer.

Example 38 - Preparation of Compound I-Anti-Theophylline Conjugate

1.1 ml of a mouse anti-theophylline monclonal antibody (Kallestad, lot no. WO399; 4.6 mg/ml) were centrifuged at 10,000 g for 8 min. The buffer was exchanged with 0.2M $NaHCO_3$, 0.15 M NaCl, with azide, pH 9.0, using an Amicon Centricon• 30 concentrator (centrifuged at 3000 x g). The antibody solution was diluted to 2 ml and 3.2 mg of Compound I were added. The reaction was allowed to proceed for 2 hrs at room temperature with stirring and 79 microliters of aqueous $NaBH_4$ at 1 mg/ml was added and the solution stirred for 30 min.

The resulting solution was loaded onto a Sephadex G-25 (1.0 cm x 18.0 cm) previously equilibrated with tris buffer and eluted at a flow rate of 15 ml/hr. The fractions containing the Compound I-anti-theophylline conjugate were pooled, transfered to dialysis tubing, and dialyzed against 0.15M phosphate/0.15 M NaF (PBS), pH 6.9 (4 l).

Example 39 - Assay for theophylline in serum based on a heterogeneous electrochemiluminescent assay

Using an immunometric assay format, a heterogeneous assay for theophylline was developed using a Compound I labeled anti-theophylline antibody and theophylline BSA immobilized on Biomag• magnetic particles. The antibody concentration was 20 micrograms/ml. The magnetic particle concentration was 1% solids (wt/vol). Theophylline was added to a final concentration of 10 and 40 micrograms/ml of serum. The theophylline serum standards were diluted 1000 fold in PBF Buffer (sodium phosphate buffer, pH 7.0, 0.1 M sodium fluoride) containing 0.1% BSA.

The assay was performed by the addition of 75 microliters of the diluted serum standards to 75 microliters of antibody conjugated to Compound I and incubating the solution at room temperature for 20 min. Then 50 microliters of the theophylline-BSA-Biomag• particles were added and the suspension was allowed to stand for 5 min. The particles were separated magnetically and 100 microliters of the supernatant was measured for electrochemiluminescence as described in Examine 46.

| Theophylline Concentration micrograms/ml++ | ECL* | SD | %CV |
|---|---|---|---|
| 0 | 8,758 | 81 | 0.9% |
| 10 | 11,078 | 368 | 3.0% |
| 40 | 14,106 | 674 | 4.8% |

*ECL counts per 10 seconds
++ corrected for dilution

Based on these results, one skilled in the art would be able to develope a heterogeneous electrochemiluminescence immunoassay for other analytes of interest in a biological matrix.

Example 40 - Preparation of a Compound II-Digoxigenin Conjugate

100.2 mg (0.104 mmol) of Compound II, 41 mg (0.105 mmol) of digoxigenin (Sigma) and 28 mg (0.136 mmol) of 1,3-dicyclohexylcarbodiimide (DCC) were added to a 50 ml round bottom flask equipped with a stirbar. To the resulting mixture were added 8-10 ml of anhydrous pyridine (Aldrich Sure-Seal) using a syringe with an 18 gauge needle. The flask was stoppered, sealed with Teflon tape and the contents gently stirred to yield a red solution. The flask was allowed to stand in the dark with stirring in the hood for 24 hrs, at which time 6 mg (0.015 mmol) of digoxigenin and 20 mg (0.097 mmol) of DCC were added. The solution was capped and allowed to stir at room temperature in the dark. The next day no evidence of dicyclohexyl urea precipitation was observed. An additional 18 mg (0.05 mmol) digoxigenin and 103 mg (0.50 mmol) DCC were added. The flask was resealed and stirring continued in the dark. After 72 hrs an additional 103 mg of DCC were added and the solution stirred in the dark for 3 hrs.

5-10 drops of $H_2O$ were added to the solution, which was then stripped to dryness on a rotary evaporator in the dark to produce a red solid.

The resulting red solid was covered with aluminum foil and dried overnight under vacuum over $CaSO_4$ in a dessicator. The dried solid was then dissolved in 3-5 ml of methanol and approximately 0.25 g of anhydrous solid $LiClO_4$ were added to the mixture. Once the $LiClO_4$ was dissolved, the solution was loaded onto a Sephadex LH-20 column (75 cm x 19 mm) and eluted with methanol at a flow rate between 7-10 sec/drop.

Three bands were noted as the chromatography proceeded; a pale orange (red luminescent) first band (Fraction 1); a dark red second band that represented the major portion of the reaction (Fraction 2); and a third band which trailed the first two bands (probably unreacted starting material) which was discarded.

Because bands 1 and 2 just barely separated on the column, any orange product in band 2 was isolated by dripping a methanol solution of Fraction 2 (15 ml) into approximately 300 ml of dry diethyl ether (stirred). The resulting precipitate was collected by suction filtration on a 15 ml medium frit and washed five times with 15 ml of diethyl ether. Residual ether was removed by drying the complex overnight over $CaSO_4$ in a vacuum desiccator. This solid material was determined to be

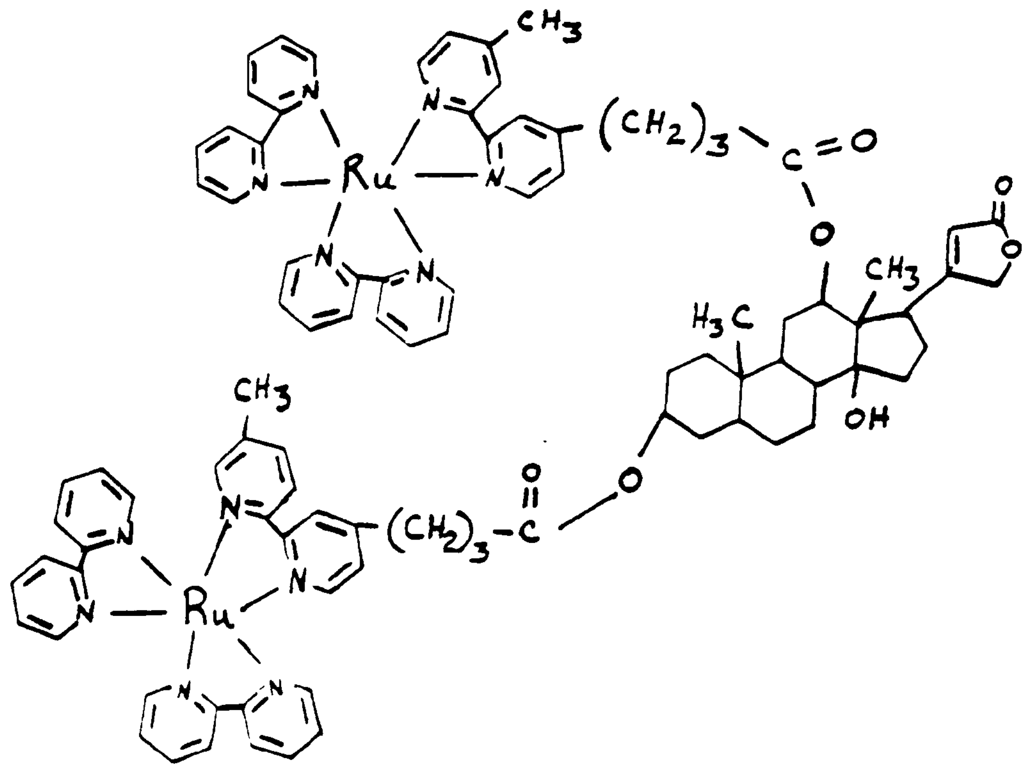

Example 41 - Electrochemiluminescence (ECL) of Compound II-Digoxigenin Conjugate: Modulation of ECL Signal by anti-digoxin antibody

Monoclonal antibody to digoxin was diluted to the following concentrations in immunoassay buffer:
1, 10, 50, 200, 400 microgams/ml

Compound II-Digoxigenin Conjugate (50 micromolar) was diluted to 150 nM in immunoassay buffer (0.1M phosphate buffer, pH 6.0, containing 0.1M sodium fluoride).

To 200 microliters of immunoassay buffer in polypropylene tubes (12 mm x 75 mm) were added 100 microliters of various concentrations of digoxin antibody and 100 microliters of Compound II-Digoxigenin conjugate (150 nM). Tubes were mixed on a vortex and incubated at room temperature for 15 min. Following incubation, 100 microliters of ECL solution (previously described) were added and electrochemilumescence was measured.

Results:

| Specific Antibody Concentration microgram/tube | $\bar{x}$ ECL Signal |
|---|---|
| 0.1 | 108000 |
| 1 | 114000 |
| 5 | 82500 |
| 10 | 64000 |
| 20 | 52000 |
| 40 | 36000 |

Total ECL Counts for 30 nM Compound II-Digoxigenin Conjugate = 113666 (peak height)

At a concentration of 40 micrograms/tube, non-specific antibody modulation of signal was 67,000 counts compared to 36,000 counts in the presence of a specific antibody to digoxin.

As can be seen from Figure 6, an increasing concentration of anti-digoxin antibody when reacted with a fixed concentration of Compound II-Digoxigenin Conjugate showed increasing modulation of the electrochemiluminescent signal. This characteristic may be used advantageously to develop a homogeneous electrochemiluminescence based assay for the measurement of digoxin in serum or plasma.

## Example 42 - Homogeneous Digoxin Assay

Based on the results described in Example 41, a homogenous electrochemiluminescent immunoassay for digoxin may be developed using antibody to digoxin and the Compound II-Digoxigenin conjugate using a competitive binding assay format. The reagents which may be used have been described in Example 41. For this assay, a specific concentration of monoclonal antibody to digoxin would be chosen. The antibody concentration may be between 75 to 100 micrograms per ml. The Compound II-Digoxigenin Conjugate concentrations may be between 5-15 nM (Final Concentration).

Digoxin Standard would be added to human serum to give a final concentration of 0.1, 0.5, 1, 2, 4, 8 and 16 nanograms of digoxin per ml of serum.

The assay may be performed by adding 10-30 microliters of serum to 300 microliters of anti-digoxin monoclonal antibody and holding the solution at room temperature of 30 min. Then 100 microliters of the Compound II-Digoxigenin conjugate may be added to each tube to give a final concentration within the range of 5 to 15 nM and incubating the solution at room temperature for 20 min. 100 microliters of the ECL solution previously described may be added to each tube and ECL may be measured as previously described.

## Example 43 - Preparation of Oubain-Bovine Serum Albumin Conjugate

50 mg of oubain octahydrate (Aldrich) was dissolved in 5 ml of deionized $H_2O$. 81 mg of $NaIO_4$ was added to the dissolved oubain and the mixture was incubated for 2 hrs at room temperature. The reaction was stopped by passing the mixture over a Dowex 1X-8 ion exchange resin column (5 ml) which had been equilibrated with deionized $H_2O$ until the pH was between 5 and 6. The oxidized oubain fraction was collected when the drops entering the waste container showed signs of mixing.

100 mg of crystalline, lyophilized bovine serum albumin (Sigma) (BSA) was dissolved in 5 ml of 0.1 M potassium phosphate buffered saline, pH 7.4, containing 0.05% azide. The oxidized oubain solution was added

dropwise to the BSA solution with stirring. The resulting solution was allowed to react for 1 hr at room temperature before adding 30 mg of $NaCNBH_4$ (Aldrich). The solution was then stirred overnight at room temperature.

The solution was concentrated (11 ml to 4 ml) using polyethylene glycol-8000 and free oubain and excess borohydride were removed from the solution by gel filtration on Sephadex G-25 (column = 0.6 cm x 37 cm; eluant = 0.1 M $K_2PO_4$/0.15 M NaCl, pH 7.5, 0.05% $NaN_3$). Fractions 11-17 were pooled and the protein concentration determined by measuring absorbance at 280nm (after dilution).

Example 44 - Preparation of Oubain-BSA-Sepharose

2 g of cyanogen bromide activated Sepharose 4B (Pharmacia) was washed with 400 ml of 1mM HCl in 50 ml portions on a sintered disk funnel. The resin was then washed with 20 ml of 0.1M $NaHCO_3$, 0.5M NaCl buffer, pH 9.0 (coupling buffer). After transferring the resin to a polypropylene container, 30 mg of oubain-BSA dissolved in 15 ml of coupling buffer were added. The activated resin was allowed to react with the oubain-BSA for 2 hrs with rotary mixing. The remaining activated sites were reacted with 7 ml of 0.5M ethanolamine (pH 8.0) for 2 hrs at room temperature. Using a sintered disk funnel, the resin was subjected to 100 ml washes with each of the following solutions: coupling buffer; 0.15M PBS; 1mM HCl; couling buffer; 0.2% NP-40 in PBS; and PBS. The resin was resuspended to 11 ml and a sufficient amount of this suspension was added to a 1.0 cm inside diameter column (Pharmacia) to give a total bed volume of 3.5 ml.

Example 45 - Preparation and Affinity Purification Of An Anti-Digoxin-Compound I Conjugate

450 microliters of a 1 mg/ml stock solution of a mouse anti-digoxin monoclonal antibody (Cambridge Medical Diagnostics, cat. no. 200M-014, lot no. MA 2507F) were concentrated to 100 microliters using an Amicon Centricon• 30 ° concentrating unit. To this concentrate were added 900 microliters of 0.2 M sodium bicarbonate buffer, pH 9.6 and 0.6 mg of Compound I. The reaction (amidation) was allowed to proceed at room temperature for 2 hours before 30 microliters of 1.0 M $NaBH_4$ (aq) (Sigma) was added. The resulting solution was allowed to stand for 1 hour.

Excess Compound I and other products were separated from the antibody conjugate by Sephadex G-25 chromatography (column = 1.0 $cm_{ID}$ x 18 cm; eluant = 0.1 M phosphate buffered saline). The sample was fractionated in 1 ml portions at a flow rate of 20 ml/hr and the absorbance at 280 nm monitored at 2.0 AU FS and 0.5 AU FS.

Fractions 5, 6, 7 and 8 were pooled, loaded onto a prewashed oubain-BSA-Sepharose affinity column (3.5 ml column with 1 cm diameter), and eluted at a flow rate of 15 ml/hr. After unretained material was eluted, the flow rate was increased to 30 ml/hr until 20 ml of eluate were collected. The saline concetration of the mobile phase were increased to 0.5M and another 20 ml were eluted through the column.

Oubain specific anti-digoxin-Compound I conjugate was removed by addition of 4 M and 6 M KSCN. Fractions corresponding to anti-digoxin-Compound I conjugate were pooled and dialyzed against 10 mM phosphate buffered saline (4 l; pH 7.4) followed by 0.1 M phosphate buffer (4 l; pH 7.0).

Example 46 - Heterogeneous electrochemiluminescent immunoassay for digoxin

10 mg of solid digoxin were dissolved in 10 ml of DMSO:$H_2O$ (8:2), to give a digoxin concentration of 1 mg/ml (hereinafter Stock Standard).

Working standards were prepared from the Stock Standard to the following concentrations in 0.15 M phosphate buffer, pH 7.0, containing 0.1% BSA and 0.15 M NaF (hereinafter ECL Buffer): 80 ng/ml, 40 ng/ml, 20 ng/ml, 10 ng/ml, 5 ng/ml and 0 ng/ml.

75 microliters of anti-digoxin-Compound I conjugate (diluted 1:90) and 75 microliters of the each standard were pipetted into a class tube, mixed on a vortex and incubated at room temperature for 20 min.

50 microliters of prewashed oubain-BSA-Biomag• particles were added to each tube, mixed on a vortex and incubated at room temperature for 5 min. Biomag• particles were separated and supernatant was transferred to a separate tube.

100 microliters of supernatant were mixed with 400 microliters of 0.125 M potassium phosphate 0.125 M citric acid; 32 mM oxalic acid; 1.25% Triton X-100 in a tube.

The sample was placed into a Berthold instrument and the electrochemiluminescence was measured as previously described except the procedure was modified by stepping the applied potential from open circuit to 2.2V and integrating the photon counts for 10 sec.

The electrode was cleaned between measurements using phosphate-citrate buffer as follows:

(a) Pulse electrode using 3 sec intervals alternating between -2.2V and +2.2V for 1 min.

(b) Poise the electrode at +2.2V for 10 seconds.
(c) Rinse electrode with deionized water $H_2O$ and blot dry.
The results are shown in Figure 7.

Example 47 - Labelling DNA with an electrochemiluminescent moiety

The following two methods have been used to label DNA with an electrochemiluminescent moiety.

Synthesis A

1.0 $A_{260}$ of the custom synthesized 38 mer (MBI 38)

TCACCAATAAACCGCAAACACCATCCCGTCCTGCCAGT*

where T* is thymidine modified at carbon 5 with

$$-CH=CH-CO-NH-(CH_2)_7-NH_2$$

were dissolved in 100 microliters of 0.01 M phosphate buffer, pH 8.7. 100 microliters of a solution of bis(2,2′-bipyridine)[4-(butan-1-al)-4′-methyl-2,2′-bipyridine]ruthenium (II) diperchlorate (Compound I) (2.3 mg in 300 microliters of 0.01 M potassium phosphate buffer, pH 8.7). The contents were stirred and allowed to stand at room temperature overnight.

100 microliters of a saturated aqueous solution of sodium borohydride was added to the mixture to convert the reversible imine Schiff's base linkage into non-reversable amine linkage. The reaction was allowed to run at room temperature for 2 hrs. The solution was then treated carefully with a few drops of dil. acetic acid to quench excess of sodium borohydride. The reaction solution was loaded onto a P-2 gel filtration column 457.2 mm x 12.7 mm (18 inches x 1/2 inch) which had been preequilibrated with 0.1 M triethylammonium acetate, pH 6.77. The column was eluted with the same buffer and 2 ml fractions were collected at a flow rate of 20 ml/hr. DNA eluted in fractions 9 and 10 were well separated from unreacted ruthenium bipyridyl complex. The collected DNA sample exhibited typical UV absorption and additionally showed a fluorescent emission spectrum at 620 nm when excited at 450 nm. The fluoresent emission shows the presence of the ruthenium bipyridyl moiety in the DNA sample. The product travels as a single orange fluorescent band on polyacrylamide gel electrophoresis. The electrophoretic mobility of the labeled DNA (MBI 38-Compound I Conjugate) is approximately the same as the unlabeled DNA.

Synthesis B

The ruthenium complex was first converted into an N-hydroxysuccinimide derivative by dissolving 3 mg in 60 microliters of anhydrous dimethylformamide and treating it with a solution of N-hydroxysuccinimide (52 mg) in 200 microliters of anhydrous DMF in the presence of 94 mg dicyclohexylcarbodiimide (DCC). The reaction was allowed to proceed for 4 hrs at 0°C. Precipitated dicyclohexylurea was removed by centrifugation and the supernatant (200 microliters) was added to the solution of amino-linked DNA (described in Synthesis A) in .01 M phosphate buffer pH 8.77 ($2A_{260}$ in 100 microliters of buffer). The reaction was allowed to proceed overnight at room temperature. A considerable amount of solid appeared in the reaction which was removed by filtration through glass wool. The filtrate was concentrated and dissolved in 0.5 ml of 1 M triethylammonium acetate (pH 6.8). The reaction mixture was then chromatographed as described in Sythesis A. The labeled DNA exhibited all spectral and electrophoretic characteristics as discussed for the material prepared in Synthesis A.

Example 48 - Electrogenerated Chemiluminescent Properties of labeled DNA

The labeled DNA sample from Example 47, Synthesis A (MBI 38-Compound I) was used to study its electrochemiluminescent properties. Various concentrations of labeled DNA were dissolved in 0.5 ml of 0.1 M phosphate buffer, pH 4.2, containing 0.1 M citrate, 25 mM oxalate and 1.0% Triton X-100 and measured on a modified Berthold luminometer. Figure 8 shows the response of the electrochemluminescent signal to various DNA concentrations.

Example 49 - Hybridization studies of Compound I-labeled oligonucleotide

The complementary strand to the 38 mer described in Example 48 was synthesized using the ABI model

380 B DNA Synthesizer and was designated MGEN-38.

To determine if the covalent attachment of Compound I to the oligonucleotide affected the hybridization properties of the MB I 38 oligonucleotide, the following experiment was devised. Various concentrations of the target fragment (MG EN-38) were spotted on a sheet of Gelman RP nylon membrane, fixed and probed with either MB I 38 or MB I 38-Compound I. Both fragments were treated with T4 polynucleotide kinase and gamma $^{32}P$[ATP] and labeled with $^{32}P$ at the 5′ end. The hybridization sensitivities of DNA and Compound I-labelled DNA were then compared.

Concentrations of MG EN-38 DNA, ranging from 50 ng down to 0.05 ng, were spotted on a nylon membrane and allowed to air dry. Duplicate membranes were set up. The blots were treated for 2 min each in : 1.5M NaCl-0.5M NaOH to fully denature the DNA; 1.5M, NaCl-0.5M TRIS to neutralize the blot, and finally in 2X SSC. The blot was baked in a vacuum oven at 80°C for 2 hrs.

The hybridization probe was prepared as follows: 3 micrograms of MB I 38 and MB I 38-Compound I were kinased with 10 units of T4 kinase and 125 microcuries of gamma $^{32}P$-ATP. The percentage of isotope incorporation into DNA was determined and shown below.

MB I 38 total count 4.1 X $10^6$ cpm/microliter
incorporated counts 3.1 X $10^5$ cpm/microliter
% incorporation = 75.6%
MB I 38-Compound I total count 3.2 X $10^6$ cpm/microliter
incorporated count 2.6 X $10^5$ cpm/microliter
% incorporation = 81.2%

Prehybridization and hybridization solutions were prepared according to Maniatis (24). Blots were prehybridized for 4 hrs at 53°C with 50 micrograms/ml of calf thymus DNA. The blots were then placed in hybridization solution containing the respective probes at 10,000,000 cpm, and allowed to hybridize overnight (12 hrs) at 53°C. The following day, the blots were washed as follows:

- twice with 2 X SSC + 0.1%SDS at 53°C for 15 minutes each wash
- twice with 0.2 X SSC + 0.1%SDS (same as above)
- twice with 0.16 X SSC + 0.1%SDS (same as above)

The blots were then air dried and exposed to Kodak X-omat• film at -70°C.

Analysis of the X-ray (see Figure 9) showed that very similar hybridization patterns were observed between the MB I 38 and MB I 38-Compound I probe. In both cases hybridization of probe to 0.5 ng of target was observed, and faint traces of hybridization were observed down to 0.05 ng of target DNA. No hybridization activity by the probe was detected for the negative control DNA (phage lambda DNA spotted at 50 ng).

Example 50 - Compound I-labeled DNA probe hybridization specificity study

Genomic DNA from several E. coli and non-E. coli strains were isolated according to methods described by Maniatis (24). The organisms used were:

E. coli strain EC8 - natural isolate
E. coli strain PCIA - enteropathogenic strain
E. coli strain 10HO7 - enterotoxigenic strain
E. coli strain EC50 - natural isolate
E. coli strain B - lab strain
E. coli strain Kl2 - lab strain
Enterobacter aerogenes
Citrobacter freundii
Salmonella paratyphi B
Salmonella potsdam

DNA from the above-mentioned strains were spotted in duplicate in 3 micrograms aliquots on a Gelman RP nylon membrane and on a S & S nitrocellulose membrane. For negative control, 50 ng of phage lamda DNA were spotted. Positive control consisted of various concentrations of the complementary strand, MG EN-38, ranging from 50 ng down to 0.5 ng. The blots were prepared and treated as described in Example 49.

The probing DNA consisted of 3.0 micrograms of the MB I 38-Compound I fragment which was T4 kinased with 125 microcuries of gamma $^{32}P$-ATP to incorporate radioactivity. The following amount of activity was incorporated.

MB I 38-Compound total counts - 3.35 X $10^6$ cpm/microliter
incorporated counts - 1.50 X $10^6$ cpm/microliter
% incorporation - 44.7%

For this assay, 2,600,000 cpm of activity was used for probing each filter. The hybridization solutions, conditions, washing solutions and protocols were the same as those described in Example 49.

The X-ray film of the blot (Figure 10) shows that both the positive and negative controls reacted accordingly. No hybridization activity was detected with lambda DNA (50 ng), and strong hybridization was observed for the complementary sequence, MG EN-38, down to 0.5 ng concentration. These findings are in total agreement with the results of the sensitivity study.

Example 51 - Preparation of 2,2′-bipyridinium-hexachloroosmate (VI)

1.01 g of ammonium hexachloroosmate (VI), $(NH_4)_2OsCl_6$ (Alfa) (1.00 equivalents) were dissolved in 50 ml of 3N HCl (aq) at 70°C. To the hot, stirred solution was added dropwise a solution of 2,2′-bipyridine in 3N HCl (aq). A red salt,

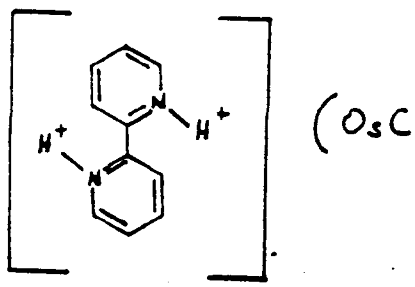

(hereinafter $(bpyH_2^{2+})OsCl_6^{2-}$), with a molecular weight of 562.2g/mole, precipitated from the solution.

Precipitation was completed by cooling the solution at 0°C in an ice bath for 2 hrs. The product was collected by suction filtration on a glass fritted filter. The precipitate was washed successively with 5-10 ml portions of ice cold 3N HCl (aq) and water. After a final washing with 20 ml of anhydrous diethyl ether, the product was dried at 70°C under vacuum for 48 hrs. Yield = 1.01 g orange powder (78% based on $(NH_4)_2OsCl_6$). The product was used without further purification.

Example 52 - Preparation of 2,2 ′-bipyridine-tetrachloroosmate (VI)

0.9g(1.60 mmol) of the $(bpyH_2^{2+})(OsCl_6^{2-})$ salt prepared in Example 51 was weighed into a pyrex test tube. The test tube was placed into a pyrex furnace tube fitted with an argon inlet and outlet and a thermometer (400°C max. reading).

The entire assembly was placed in a tube furnace and flushed with argon. The furnace was activated and brought to a temperature of 270-300°C. A slow stream of argon was passed through the tube throughout the reaction. The argon outlet was vented into the fume hood to carry HCl produced during the reaction out of the laboratory. As the temperature approached 270°C, pyrolysis of the reactant began. HCl gas was observed to evolve from the solid. Vigorous evolution of HCl was observed for 30 min and then slowly began to subside. After 6 hrs the oven was shut off and the sample cooled to room temperature. The product $(bpy)OsCl_4$, a finely divided red-brown solid, was purified by suspension in a stirred 3N HCl (aq) solution for 12 hrs followed by suspension for 6 hrs in stirred anhydrous diethyl ether. Isolation of the product via suction filtration on a glass fritted filter gave 0.75 g (95%) yield of 2,2′-bipyridine-tetrachloroosmate (VI), hereinafter $(bpy)OsCl_4$. (MW=489.3 g/mole).

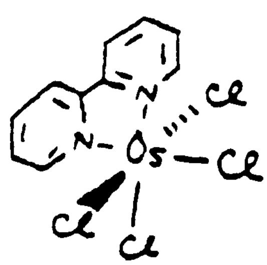

The product was used without further purification.

Example 53 - Preparation of cis-(2,2′-bipyridine)[cis- bis(1,2-diphenylphosphino)ethylene]-dichloroosmium (II)

Into a 50 ml round bottom flask equipped with a stirbar and reflux condenser were added 230 mg (0.47 mmol) (bpy)$OsCl_4$ prepared as in Example 52 and 465 mg (1.17 mmol) cis-bis (1,2-diphenylphosphino)ethylene (DPP-ene). 25 ml diglyme were added and the contents were refluxed under an argon atmosphere for 5 hrs. The dark green-black solution was cooled to room temperature under argon atmosphere and transferred to a 200 ml beaker. Addition of 80 ml anhydrous diethyl ether gave a dark green precipitate. The precipitate was collected by suction filtration on a glass fritted filter (medium porosity) and washed with five 20 ml portions of anhydrous diethyl ether. The precipitate was then dissolved in 15 ml $CH_2Cl_2$ to give a forest green solution. The solution was allowed to gravity drip through a glass fritted stirred (medium porosity) into approximately 300 ml stirred anhydrous diethyl ether. Precipitation of the gray-green cis-(2,2′-bipyridine) [cis-bis(1,2-diphenylphosphino)ethylene-dichloroosmium II complex, hereinafter cis-(bpy)(DPP-ene)$OsCl_2$), occured. The complex was collected by suction filtration on a glass fritted filter (medium porosity). The product was washed quickly with 20 ml cold 1:2 v/v ethanol/water followed by seven 30 ml portions of diethyl ether. The slate-gray powder was dried over $CaSO_4$ overnight in the vacuum desiccator.

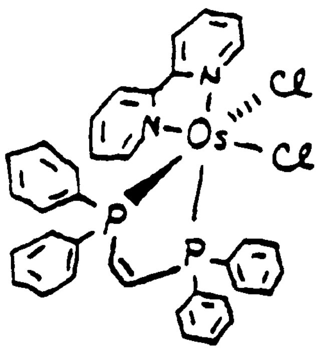

Yield: 240 mg (63% based on (bpy)$OsCl_4$; MW=814.4 g/mole. The product was used without further purification.

Example 54 - Preparation of (2,2′-bipyridine)[cis-bis(1,2-diphenylphosphino)ethylene{2-[3-(4-methyl-2,2′-bipyridine-4′-yl)propyl]-1,3-dioxolane}osmium (II) dichloride

Into a 100 ml round bottom flask equipped with stirbar and reflux condenser were added 129 mg (0.158 mmol) cis-(bpy)(DPPene)$OsCl_2$ prepared as in Example 53 and 0.3g (1.0 mmol) of 2-[3-(4-methyl-2,2;-bipyridine-4-yl) propyl]-1,3-dioxolane, i.e., bpyoxal. To this was added 15 ml ethylene glycol. The suspension was refluxed under an argon atmosphere for 3 hrs. After cooling to room temperature, 10 ml $H_2O$ were added to the contents of the flask.

A column (30 cm height x 19 mm i.d.) of SP-Sephadex C-25 ion exchange resin in water was prepared. The contents of the reaction flask were loaded onto the column. The column was eluted with $H_2O$ (approximately 500 ml) to remove ethylene glycol and excess bpyoxal ligand. elution with 0.25 NaCl (aq) solution separated a small pale yellow band (orange fluorescence under long wave UV light) followed by a non-luminescent olive green band. These bands represented side-products of the reaction. No attempt was made to identify them and they were discarded. Once these bands were removed from the column, elution with 0.5M NaCl (aq) was begun. Three bands were eluted. The first, an orange band with orange luminescence, was identified as the chloride salt of the desired product moiety, (2,2′-bipyridine) [cis-bis] (1,2-diphenyl phosphino) ethylene] 2-[3-(4-methyl-2,2′-bipyridine-4′-yl)propyl]-1, 3,-dioxolane osmium (II), hereinafter (bpy) (DPPene) (bpyoxal)$Os(II)^{2+}$. It separated cleanly from a trailing yellow band (green luminescence) and brown band (non-luminescent).

The volume of solution in the fraction (approximately 400 ml) containing the (bpy) (DDPene) (bpyoxal)$Os(II)^{2+}$was reduced by evaporation to approximately 50 ml on a rotary evaporator. The aqueous NaCl concentrate was then extracted with three 50 ml portions of $CH_2Cl_2$. The $CH_2Cl_2$ extracts were combined, dried over anhydrous $Na_2SO_4$, and gravity filtered through a fluted filter paper. The red $CH_2Cl_2$ solution was concentrated to a volume of 10 ml by evaporation on the rotary evaporator. The complex was isolated as an orange solid by slowly dripping the $CH_2Cl_2$ solution into 200 ml of well-stirred petroleum ether. The precipitated complex

was collected by suction filtration on a glass fritted filter (medium porosity). The product was washed three times with 15 ml portions of diethyl ether. The product was dried over $CaSO_4$ overnight in the vacuum desiccator.

Yield: 110 mg of (bpy) (DPPene) (bpyoxal)Os(II)$^{2+}$(Cl$^-$)$_2$ ·2$H_2O$ (63% based on cis-(bpy) (DPPene)$OsCl_2$). The product is a dihydrate and is analytically pure: $C_{57}H_{54}N_4O_4P_2Cl_2Os·2H_2O$ (MW = 1109.59 g/mole).

Theory: C, 55.20%; H, 4.87%; N, 5.05%; O, 5.77%; Cl, 6.39%. Found: C, 56.03%; H, 5.18%; N, 4.88%; O, 6.87%; Cl, 7.07%.

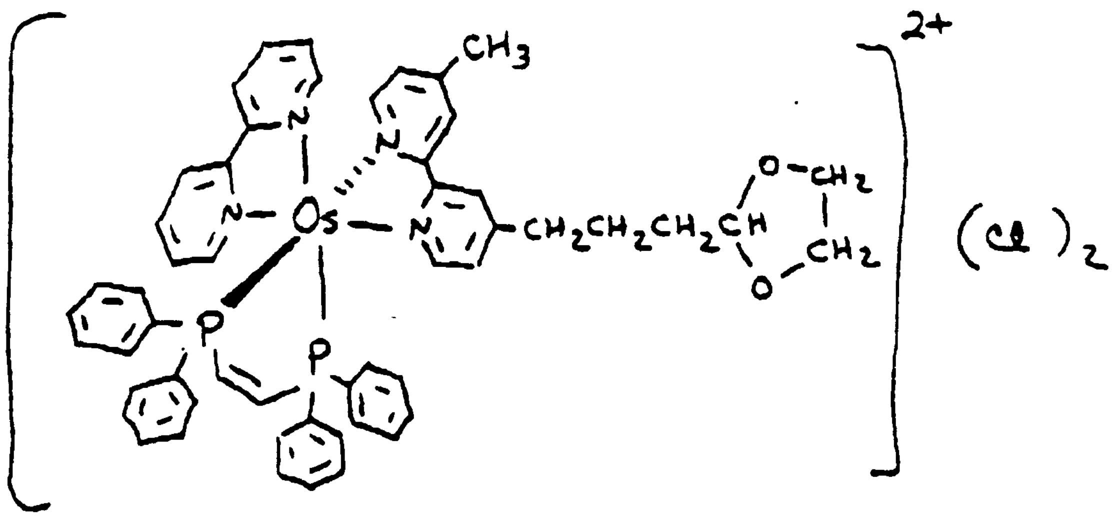

Example 55 - Preparation of cis-dichloro-bis-[4,4′-carbomethoxy)-2,2′-bipyridine] ruthenium (II)

Into a 250 ml round bottom flash equipped with stirbar and reflux condenser were added 750 mg (1.55 mmol) of cis-tetra-(dimethylsulfoxide) dichlororuthenium (II) ($Ru(DMSO)_4Cl_2$) and 100 ml ethylene glycol. The contents of the flask were brought to a gentle boil under argon atmosphere and 756 mg (3.09 mmol) of (4,4′-carbomethoxy)-2,2′-bipyridine were added. Heating under argon was continued for 5 min. The orange solution became brown/black and 0.75 g of lithium chloride and 50 ml ethylene glycol were added. The solution was heated for another 10 min. After cooling to room temperature, approximately 100 ml $H_2O$ were added to the mixture. The mixture was extracted with five 200 ml portions of $CH_2Cl_2$.

The $CH_2Cl_2$ extracts were washed with six 200 ml portions of water. The water layers were tested for fluorescence (red) after each washing and washing was continued if necessary until no fluorescence could be detected in the aqueous layer. The $CH_2Cl_2$ extracts were dried over anhydrous $Na_2SO_4$. The product was isolated by evaporation of the $CH_2Cl_2$ solution of the product into a stirred 10-fold volume excess of anhydrous diethyl ether. The precipitated product was collected by suction filtration, washed once with 30 ml diethyl ether and dried over $CaSO_4$ overnight in a vacuum desiccator. Yield = 25% dark metallic green crystals.

The product is analytically pure.

Theory: C, 44.57; H, 4.01; N,7.43; Cl, 9.40; O, 21.21.

Found: C, 44.16; H, 3.72; N, 7.11; Cl, 9.53; O, 20.15.

MW = 754.5 g/mole.

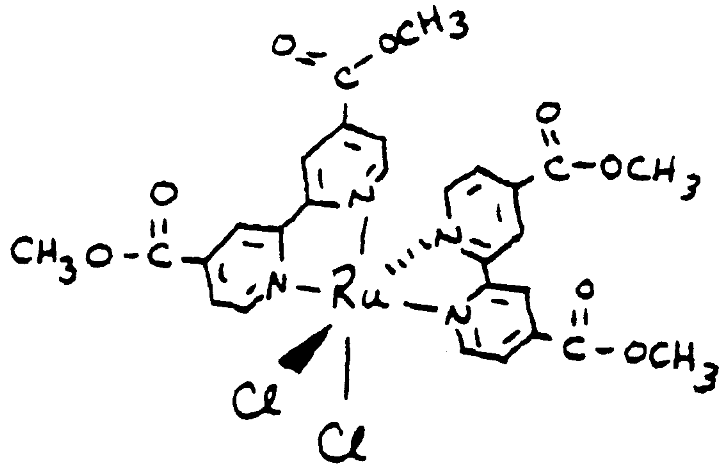

Example 56 - Preparation of bis[(4,4'-carbomethoxy)-2,2'-bipyridine] 2-[3-(4-methyl-2,2'-bipyridine-4-yl)propyl]-1,3-dioxolane ruthenium (II) diperchlorate

To 250 mg of bis(4,4'-dicarbomethoxy-2,2'-bipyridine) ruthenium (II) dichloride (0.33 mmol) in 50 ml methanol/water (1:1) were added 105 mg (0.37 mmol) of 2-[3-[4-methyl-2,2'-bipyridine-4'-yl)-propyl]-1,3-dioxolane (bpyoxal) and the mixture was refluxed for 12 hrs under an argon atmosphere. The solution was cooled and 0.5 ml of 70% $HClO_4$ were added. The methanol was slowly evaporated. Red crystals precipitated and were collected on a fritted funnel, washed with a small amount of cold water followed by ethanol and ether and dried to vacuo. Similar methods were used to prepare complexes from bis (4,4'-dicarbomethoxy-2,2'-bipyridine) ruthenium (II) dichloride and either 4-(4-methyl-2,2'-bipyridine-4'-yl)-butyric acid or 4-4'-methyl-2,2'-bipyridine.

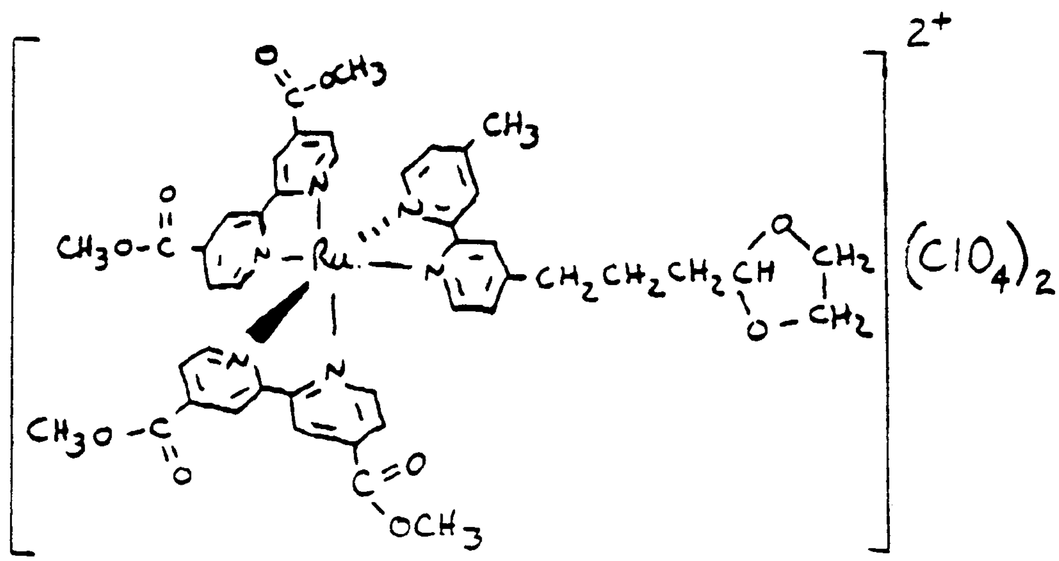

Example 57 - Compound I Labeling of Human IgG

2 ml of human IgG (2.5 mg/ml) were dialyzed against 2 liters of 0.2M sodium bicarbonate buffer, pH 9.6, overnight at 4°C with gentle stirring. Compound I was prepared in a 100 molar excess to protein present (2.7 mg/100 microliters dimethylformamide) and allowed to dissolve. The dialyzed protein was added dropwise to the tag-aldehyde while gently stirring at room temperature for 2 hrs. A 100 molar excess (to protein) of sodium borohydride (100 microliters of a 1.24 mg/ml solution in deionized water) was added to the solution and gently stirred for an additional 30 min at room temperature. The conjugate was loaded onto a Sephadex G-25 column (1.0 cm x 18.0 cm) equilibrated at room temperature with 0.2M Tris, pH 8.0, and the eluant was monitored at 280 nm. The void volume peak was collected, pooled, and dialyzed against 2 liters of the Tris buffer. The conjugate was tested for immunological activity by standard ELISA methods, and stored at 4°C until used.

Example 58 - Biomag•/Goat-Anti-Human IgG Particle Preparation

A 2.5 ml aliquot of 5% Biomag•-amine terminated particles (Advanced Magnetics, Cambridge, MA) was transferred to a clean T-flask and washed 5 times with phosphate buffered saline (PBS). The wet cake was resuspended in 12.5 ml of 5% fresh glutaraldehyde (Sigma) and rotated end-over-end at ambient temperature for 3 hrs. The wet cake was transferred to a second T-flask and washed 3 times with PBS. The activated particles were resuspended to 12.5 ml with PBS, and transferred to a 15 ml centrifuge tube. To this suspension were added 12.5 mg of goat anti-human IgG (H+L) (Jackson Labs) in 2 ml of PBS. The tube was rotated end-over-end for 3 hrs at room temperature and then overnight at 4°C. The next day the particles were washed 2 times with PBS containing 1% (w/v) bovine serum albumin (BSA) followed by storage in 12.5 ml of PBS containing 0.1% BSA at 4°C until use.

Example 59 - Pseudo-Homogeneous Human IgG Electrochemiluminescence Assay Using Biomag• Magnetic Based Particle Assay

Compound I-human IgG conjugate was diluted 1:50 in 0.15M phosphate buffer containing 0.1% BSA and aliquoted at 250 microliters/tube. 150 microliters of diluent (PB w/BSA as above) were added per tube, followed by the addition of either various dilutions of analyte (human IgG) or diluent (negative controls). Additionally, a non-sepecific analyte (goat anti-rabbit IgG) was used in some tubes to check for assay specificity. 50 microliters of 1% Biomag• coupled with goat anti-human IgG (H&L) were added to each tube. The tubes were mixed on a vortex and incubated at room temperature for 15 min with gentle shaking. The particles were magnetically removed from solution and 100 microliters of the resulting supernatant were transferred to a Berthold tube to which 400 microliters of a citrate-oxalate-Triton X-100 solution were added. The tube was mixed on a vortex and read in a modified Berthold luminometer fitted with an R-268 photomultiplier tube and a 0.29 inch diameter circular 52 gauge double platinum mesh electrode supported by a conductive paint covered polycarbonate support and connected to the voltage source through a 0.01 inch diameter platinium wire/silver paint contact. The potential applied was stepped from +1.4 to +2.15 V while a 10 second integration was done. Between readings the electrode was electrochemically cleaned by rinsing with deionized water, then immersing in 0.1M phosphate-citrate buffer containing oxalic acid and Triton X-100 and stepping the applied potential from -2.2V to +2.2V (with 3 seconds at each potential) for 2 min and 20 seconds followed by pausing the potential at +2.2V for 10 seconds. The electrode was removed from the cleaning solution, rinsed with deionized water and blotted dry with an absorbent wiper. In this format the electrochemiluminescent signal was directly proportional to the analyte concentration.

Example 60 - Preparation of bis(2,2′-bipyridine)maleimidohexanoic acid, 4-methyl-2,2′-bipyridine-4′-butylamide ruthenium (II) diperchlorate: Compound IV

4-[4-(1-aminobutyl)]-4′-methyl-2,2′-bipyridine, prepared as described in Example 30 from 500 mg (1.35 x $10^{-3}$ mol) of pthalimide, was dissolved in 5 ml of dry pyridine with 0.313 g (1.48 x $10^{-3}$ mol) of maleimide hexanoic acid and 0.306 g (1.48 x $10^{-3}$ mol) of DCC. After storing overnight, the dicyclohexylurea was filtered off and the pyridine was stripped under vacuum. The residue was purified by column chromatography (activity II alumina, 5% $MeOH/CH_2Cl_2$) to give the purified product. Yield: 0.56 g (95%).

100 mg (2.3 x $10^{-4}$ mol) of maleimido-hexanoic acid, 4-methyl-2,2′-bipyridine-4′-butylamide and 100 mg (2.06 x $10^{-4}$ mol) of bis-(2,2′-bipyridine) ruthenium dichloride dihydrate were dissolved in 50 ml of ethanol/water (1:1), degassed with argon and refluxed for 4 hr. The resulting clear orange solution was diluted with 25 ml of solid $NaClO_4$, 25 ml of ethanol and 25 ml of acetone, and slowly evaporated under vacuum. When dry, another 25 ml of water and 25 ml of acetone were added and the solution was rotoevaporated to about 15-20 ml. The precipitated solid was collected, washed with water and dried. The sample was purified by preparative TLC on alumina using 1:9 methanol/chloroform. The fast running band was isolated by scraping off the plate and eluting the compound by stirring in methanol/chloroform (1:1). After filtration to remove the alumina, the orange solution was evaporated to dryness to give 86.7 mg of purified compound (72%). The structure was confirmed by NMR.

Example 61 - Labeling of hCG Peptide with Compound IV

2 mg of human chorionic gonadotropin (hCG) peptide (#109-145, JP141, Vernon Stevens, Ohio State University) were suspended in 1 ml of 0.15M citrate buffer, pH 6.0, and 1.13 mg of Compound IV were dissolved in 300 microliters of dimethylformamide. The peptide solution was added dropwise to the Compound IV solution over a one minute period. The solution was stirred gently at room temperature for 1 hr. The sample was then loaded onto a Bio-Gel P-2 column (Bio-Rad; 1 cm X 45 cm) which was equilibrated at room temperature with 0.2M Tris-base, pH 8.5 at a flow rate of 15 ml/hr and the eluant was monitored at 280 nm. The void volume of the run was collected, pooled, and loaded onto a QAE-Sephadex A-25 column (Pharmacia; 1 cm x 10 cm) which was equilibrated at room temperature with 50 mM Tris-HCl, pH 7.0. This was done to remove any unlabeled peptides (which will adsorb to the positively charged resin; the positive charge of the labeled peptide allowed it to pass out of the column without further treatment). The eluant was monitored at 280 nm, and the first major peak was collected, pooled, and concentrated by lyophilization. The dried compound was resuspended in a minimal volume of PBF. The hCG peptide-Compound IV conjugate was stored at 4°C until used.

Example 62 - Purification of Rabbit Anti-hCG Peptide By DEAE AFFI-Gel Blue Chromatography

4 ml of DEAE AFFI-Gel Blue (Bio-Rad) was poured into a chromatography column (1 cm x 10 column size) and equilibrated at room temperature with 0.2M Tris-HCl containing 0.028M NaCl, pH 8.0, for 1 hr at a 40 ml/hr flow rate. The flow rate was slowed to 20 ml/hr and 1 ml of rabbit anti-hCG peptide (anti 109-145, Vernon Stevens, Ohio State University), which was predialyzed against the column buffer, was loaded onto the column. 1 ml fractions were collected and the eluant was monitored at 280 nm. The void volume peak was collected, pooled from several runs, and concentrated by placing the eluant in a 12K MWCO dialysis sack which was surrounded with polyethlyene glycol 6,000 (Sigma). The antibody was tested for immunoreactivity using standard ELISA techniques and for purity by HPLC, which resulted in one major peak, indicating a pure, active preparation.

Example 63 -Titration of hCG Peptide-Compound IV Conjugate Against Purified Rabbit Anti-hCG Peptide: Electrochemiluminescence Signal Modulation

hCG peptide-Compound IV Conjugate was diluted in 0.1M phosphate buffer containing 0.1M citrate, pH 6.2. Aliquots of this mixture were placed in microfuge tubes. The previously purified anti-peptide antibody was diluted to various concentrations and added to the tubes, which were mixed on a vortex and incubated for 1 hr at room temperature. Just before reading electrochemiluminescence, an aliquot was transferred to a Berthold tube to which oxalic acid and Triton X-100 (Sigma) were added. The tubes were mixed on a vortex and read using sweep mode (+1.5 to +2.5V at 50mV/sec of which 3 scans were done, taking the peak height of the second scan as the value) on a Berthold luminometer using an R-268 photomulitplier tube with a double platinum mesh electrode. Between readings the electrode was cleaned electrochemically by first rinsing it with deionized water and then immersing it in 0.1M phosphate-citrate buffer, pH 6.1, containing 25mM oxalate and 1% Trion X-100. The applied potential was switched, with three seconds at each potential, between +2.2V and -2.2V for 1 min and 50 sec and poised at +2.2V for 10 sec. The power was then shut off and the electrode removed from the cleaning solution and rinsed with deionized water and blotted dry with absorbent toweling.

The results showed a general trend in which the electrochemiluminescent signal was directly proportional to the concentration of antibody to the peptide. This is in contrast to other experiments in which the electrochemilumienscent signal drops as the electrochemiluminescent labelled analyte is contacted with antibody.

Example 64 - Electrochemiluminescence Signal Output: Stability Data Of Ru(II)-Compound III Conjugate

Ru(II)-Compound III conjugate was diluted to 300 nM in 0.1M phosphate-citrate buffer, pH 6.1, either with or without 1% normal human serum (NHS). One of each buffer type was incubated at 4°, 20°, 37° and 55°C and sampled on incubation days 3, 4, and 5. The samples were equilibrated at room temperature and evaluated for their electrochemiluminescence signal output. 400 microliters of sample was mixed with 100 microliters of 125mM oxalic acid-5% Triton X-100 in a tube and placed in a modified Berthold luminometer with a Hamamatsu R-268 photomultiplier tube and a double mesh platinum gauze electrode. The reading was made by sweeping the applied potential from +1.5V to +2.5V at 50 mV/second for 3 cycles and the height of the second cycle peak was recorded and converted into electrochemiluminescent counts. Between readings the electrode was cleaned electrochemically by rinsing with deionized water, immersing the electrode in 0.1M phosphate-citrate buffer containing 25 mM oxalate and 1% Triton X-100 and pulsing the potential from -2.2V to +2.2V with a 3 sec pause at each potential for 1 min and 50 sec. The potential was poised at +2.2V for 10 sec and then removed. The electrode was removed from the cleaning solution, rinsed with deionized water and blotted dry with absorbent toweling. The results show that there was consistent signal in each buffer type over the course

of the study. This indicates the stability of the reagent and its capability of generating an electrochemiluminescent signal.

Example 65 - Immunoreactivity Testing of Ru(II)-Compound III Conjugate: Stability Studies

The Ru(II)-Compound III conjugate was diluted and incubated under the conditions as described in Example 64. Samples were taken on days 3, 4, and 5 cooled to room temperature and tested for immunoreactivity by Particle Concentration Fluorescent Immunoassay (PCFIA) using a Pandex Screen Machine obtained from Pandex, Inc., Mundelein, IL. The PCFIA was run in a competitive assay format. The latex particles (Pandex) were conjugated with theophylline-BSA. A constant amount of these particles were mixed with the Ru(II)-Compound III Conjugate test solution to final concentrations of anti-theophylline monoclonal antibody (ascites, Hyclone cat #E-312OM, lot #RD200) and a constant amount of goat anti-mouse IgG-FITC conjugate (Pandex, cat #33-020-1 lot # CO1) . After incubation, the samples were processed and read on the Pandex Screen Machine. The results showed that there was no appreciable loss of activity even after 5 days incubation at 55°C.

Example 66 - Electrochemiluminescence of various ruthenium and osmium compounds

The electrochemistry of various osmium and ruthenium compounds was measured as 1 mM solutions in 10 ml of nitrogen-purged acetonitrile with 0.1 M tetrabutylammonium tetrafluoroborate as an electrolyte. The working electrode was a platinum disk electrode obtained from Bioanalytical Systems, Inc., West Lafayette, IN. A platinum wire counter electrode and a 1.0 mm silver wire was used as a reference electrode. Measurements were made by scanning from -2.2V to +2.2V (vs SCE) at a scan rate of 100 mV/second. After each electrochemical measurement the potential difference between a Saturated Calomel Reference Electrode (SCE) and the silver wire was determined. Thus, the values reported are corrected to the potential versus SCE.

Electrochemiluminescent (ECL) measurments were made in 0.5 ml aqueous solutions containing 0.1M phosphate-citrate buffer (pH 4.2), 25 mM oxalic acid, and 1% Triton X-100. The electrode system used consisted of two platinum gauze (52 gauge) electrodes connected to a Radio Shack transistor socket (#276-548) by a 0.1 mm platinum wire. The electrodes were mounted on the outside of a 60 ml thick piece of cellulose acetate plastic. This plastic was machined so that a 6.35 mm (1/4 inch) diameter hole allows solution to easily flow between the working and counter-reference electrodes. The electrodes were connected to the potentiostat so that one electrode functioned as a working electrode (which was closer to the photomultiplier tube) and one electrode functioned as the counter and reference electrode. Measurements were made by sweeping from 1.5V to 2.5V (bias potential) at a scan rate of 50 mV/second. The ECl measurements are reported as the signal to noise ratio, i.e., or signal to background ratio for a given concentration of compound. Background is defined as the luminescent counts observed with buffer and no ECl compounds added. Luminescent measurements were the peak light output observed during the first or second linear sweep.

Both electrochemiluminescent (ECL) and cyclic voltammetric measurements of each solution were performed with either a EG&G Model 273 potentiostat or a bipotentiostat from Ursar Scientific Instruments, Oxford, England. The photon flux of each ECL measurement was monitored with a Berthold Biolumat LB 9500 luminometer from Wilebad, West Germany, modified so that either a two or three electrode system could be placed in the 0.5 ml measuring solution. Both electrochemical and electrochemiluminescent measurements were recorded and a Kipp & Zonen Model BD 91 X, Y, Y′ recorder from Delft, Holland.

Fluorescence measurements were made with 50 micromolar solutions of the desired compound in 3.0 ml of ECL solution, or when insoluble in ECL solution, in acetonitrile. Measurements were made on a Perkin-Elmer LS-5 Fluorescence Spectrophotometer. Prescans of the solutions' excitation and emission spectra were performed before the excitation and emission spectra were recorded so that the emission spectrum could be measured while irradiating at the maximum excitation wavelength and conversely, the excitation spectrum could be recorded while monitoring the maximum emission wavelength.

| | Compound | $E_{ox}$ vs. SCE | $E_{red}$ | Fluorescence Emission Max | ECL(S/N)[1] & Conc. |
|---|---|---|---|---|---|
| a) | $Ru(bipy)_3$ | 1.07 V | -1.52 V | 625 nm | $1 \times 10^{-9}$M (2.5) |
| b) | Ru(4,4' $-CO_2$-bipy$)_3$ | 1.19 V | N.D. | 628 nm | $2 \times 10^{-9}$M (4.05) |
| c) | Ru(4,4'$CO_2$-Et-bipy$)_3$ | 1.54 V | -0.89 V | 636 nm | $1 \times 10^{-10}$M (2.01) |
| d) | $Ru(bipy)_2$ (C-8-theo-phylline C-4-bipy) | 1.17 V | N.D. | 624 nm | $1 \times 10^{-9}$M (.97) |
| e) | $Os(bipy)_2$(CO) (py) | 1.82 V | -0.99 V | 585 nm | $1 \times 10^{-8}$M |
| f) | Os(DPPene) (bpy) (bpyoxal) | 1.60 V | N.D. | 630 nm | $6 \times 10^{-8}$M (10.8) |

[1](S/N) is the signal to noise ratio, where the signal is defined as the ECL output (luminescent counts) of a compound at a given concentration, and noise is the luminescent counts of the buffer in which the compound was dissolved.

Unike the ECL of the other compounds which were measured at pH 4.2, compound C) also displays significant ECL at the physiological pH of 7.0.

a) Tris (2,2′-bipyridine) ruthenium $^{2+}$

b) Tris (4,4′-carboxylate-2,2′-bipyridine) ruthenium $^{2+}$

c) Tris (4,4′-carboethoxy-2,2′-bipyridine) ruthenium $^{2+}$

d) bis(2,2′-bipyridine)[theophylline-8-butyric-4-(4-methyl-2,2′-bipyridine)-4′-yl)-butyl)amide]ruthenium (II) dichloride

e) [bis-(2,2′-bipyridine {monocarbonyl} pyridyl] osmium (II) dihexafluorophosphate

f) (2,2′-bipyridine[cis-bis(1,2-diphenylphosphine)ethylene]{2-[3-(4-methyl-2,2′-bipyridine-4′-yl)propyl]1,3-dioxolane} osmium (II) dichloride

Example 67 - Preparation of bis(2,2′-bipyridine)[4-(4′-methyl-2,2′-bipyridine)butyl amine] ruthenium (II) diperchlorate

1.29 g (2.48 mmol) of bis-(2,2′-bipyridine) ruthenium (II) dichloride dihydrate (Strem) and 0.719 g (2.98 mmol) of 4-[4-(1-aminobutyl)]4′-methyl-2,2′-bipyridine (described in Example 30) were suspended in 50 ml of 50/50 ethanol/$H_2O$ and refluxed under argon for 3 hr. The reaction solution was concentrated and subjected to chromatographic separation on Sephadex C-25, eluting first with distilled water and then with 0.25 M NaCl. Final elution was performed with 0.4 M NaCl. The fractions containing the product were concentrated to about 100 ml and perchloric acid was added until the solution became cloudy. Upon refrigeration overnight, red orange crystals of the product (1.215 g) were obtained. Elemental analysis confirmed the structure to be

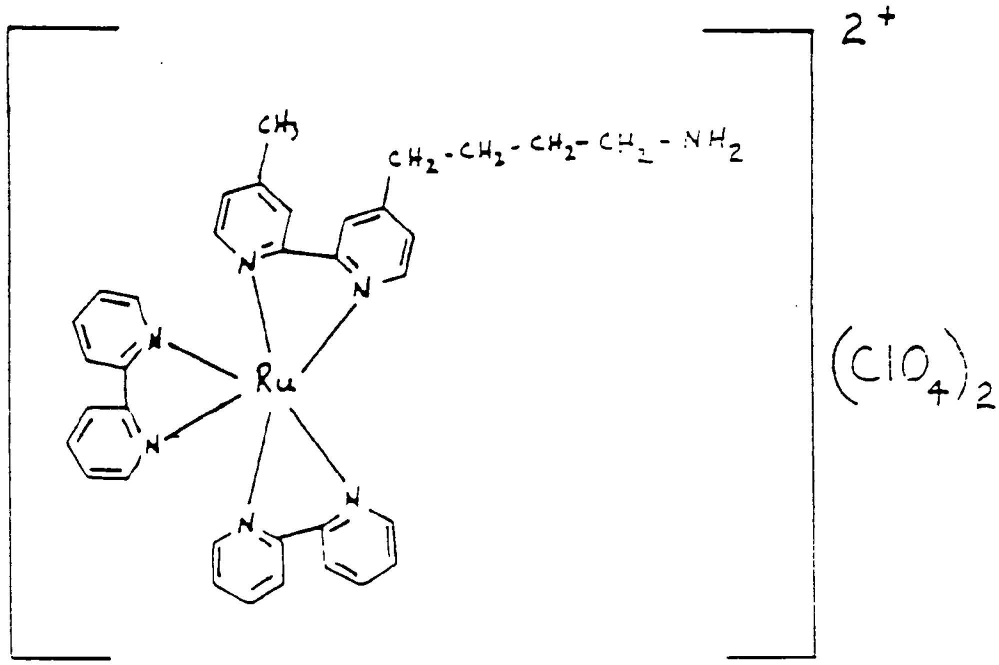

Example 68 - Preparation of theophylline-8-(3,3-dimethyl) butyric acid

5,6-diamino, $N^1$, $N^3$-dimethyl uracil hydrate (10 g, 0.059 mol ) and 3,3-dimethylglutaric acid anhydride (12.6 g, 8.85 x $10^{-2}$ mol) were dissolved in 100 ml N,N-dimethyl aniline and brought to reflux using a Dean-Stark trap. The contents of the reaction became solubilized after heating and turned orange red. After 4 hrs of reflux, 1 ml of water was collected in the trap, indicating the reaction had completed. The reaction was allowed to cool to room temperature, at which time the entire mass solidified. The solid was filtered on a fritted funnel and washed until it became pale yellow in color. After two cyrstallizations from DMF, pure white cyrstals of the intermediate lactam (melting point 283.1-284.9°C) were obtained. The lactam was boiled in water for 8 hrs. Upon cooling, the solution yielded pure white crystals (melting point 218-220°C). Proton NMR and elemental analysis were consistent with the following structure

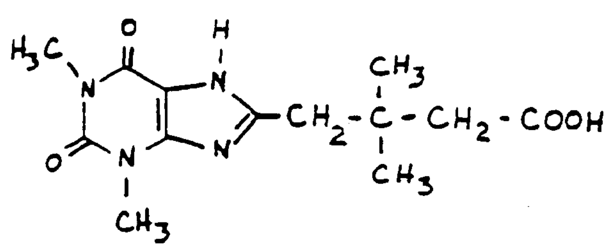

Example 69 - Preparation of bis-(2,2′- bipyridine)[theophylline-8-(3,3-dimethylbutyric-{4-(4-methyl-2,2′-bipyridine-4′-yl)-butyl}amide]ruthenium (II) diperchlorate

100 mg (0.34 mmol) of theophylline-8-(3,3-dimethyl) butyric acid and 0.325 g (0.34 mmol) of bis (2,2′-bipyridine) [4-(4′-methyl-2,2′-bipyridine) butyl amine ruthenium (II) diperchlorate were dissolved in 10 ml of dry pyridine along with 0.351 g (1.7 mmol) of dicyclohexylcarbodiimide. The reaction was allowed to stir for 2 days. Copious dicyclohexylurea precipitate was removed by filtration and the pyridine solution was concentrated under vacuum. The residue was dissolved in methanol and chromatographed on Sephadex LH-20. On concentration of the desired fractions the product was precipitated from ether to give an orange precipitate. Elemental analysis and proton NMR were consistent with the structure

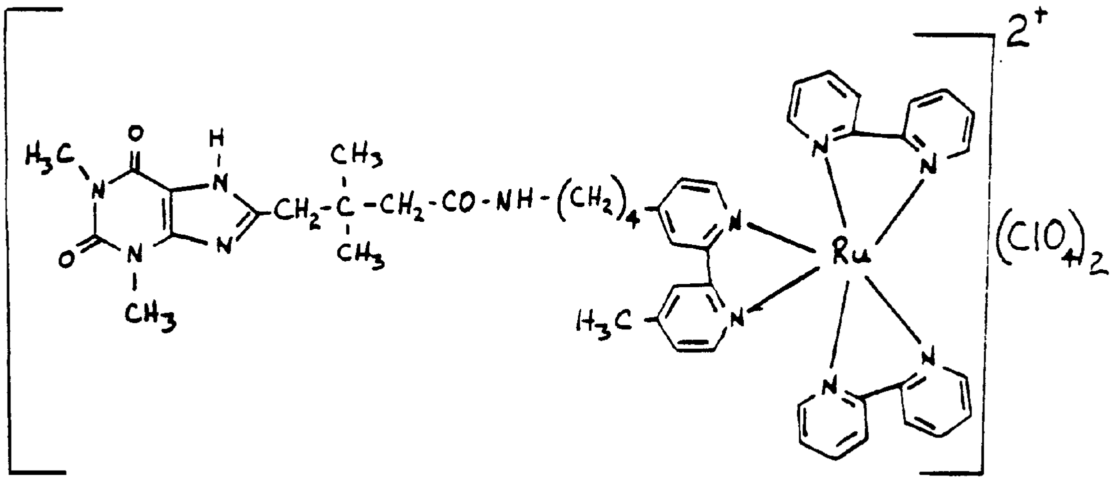

Example 70 - Preparation of theophylline-8-propyl(4-methyl-2-2'-bipirydine-4'-butyl) carboxamide

61.8 mg ($1.55x10^{-4}$mol) of 8-(gamma-aminopropyl) theophylline phthalic acid hydrazide salt were dissolved in 1 ml $H_2O$ and acidified with HCl. The precipitated phthalic acid hydrazide was filtered off and the aqueous filtrate was evaporated and dried under vacuum to give 34.5 mg ($1.31 \times 10^{-4}$ mol) of theophylline-8-propylamine hydrochloride. This material was dissolved in 5 ml dry pyridine and 36.9 mg ($1.44 \times 10^{-4}$ mol) of 4-(4-methyl-2,2'-bipyridine-4'-yl) butyric acid and 26.4 mg ($2.62 \times 10^{-4}$ mol) of triethylamine were added to it. Finally, 29.7 mg ($1.44 \times 10^{-4}$ mol) of dicyclohexylcarbodiimide were added to the solution. The resulting cloudy solution was allowed to stir overnight. The precipitated salt and dicyclohexylurea were filtered off and the solution was stripped to dryness to give a white solid which was chromatographed on alumina using 5% methanol in dichloromethane as the eluant to yield 44.2 mg (71%) of the product as a white powder (melting point 215°-216.5°C). Proton NMR and elemental analysis were consistent with the following structure

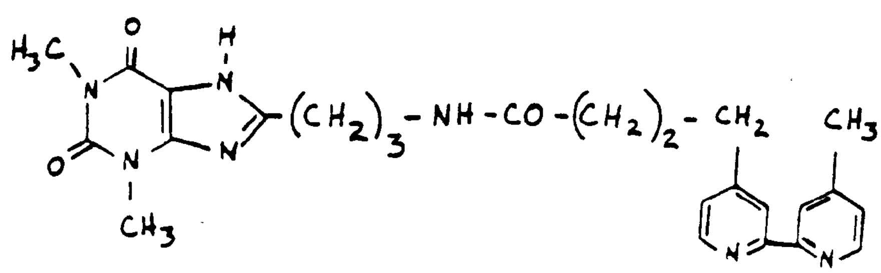

Example 71 - Preparation of bis-(2,2'-bipyridine)[theophylline-8-propyl(4-methyl-2,2'-bipyridine-4'-butyl)carboxamide]ruthenium (II) dichloride

100 mg ($2.06 \times 10^{-4}$ mol) of bis(2,2'-bipyridine)-ruthenium dichloride (Strem) and 108 mg ($2.27 \times 10^{-4}$ mol) of theophylline-8-propyl(4-methyl-2,2'-bipyridine 4'-butyl) carboxamide were added to 50 ml of 50% ethanol which had been degassed with argon. The solution was heated at reflux. The resulting cherry red solution was concentrated under high vacuum at room temperature. The residue was chromatographed on Sephadex LH-20 (75 cm x 19 mm I.D.) using methanol as eluent. The product band was isolated, evaporated and precipitated into anhydrous ether. The yield of the product was 156 mg (75%). Elemental analysis indicated the presence of 4 mols of methanol in the product.

Analysis: Calculated; C, 54.09; H, 5.65; N, 14.16; O, 10.29; Cl, 6.52; Found: C, 53.30; H, 5.22; N, 14.56; O, 10.63 and Cl, 6.95.

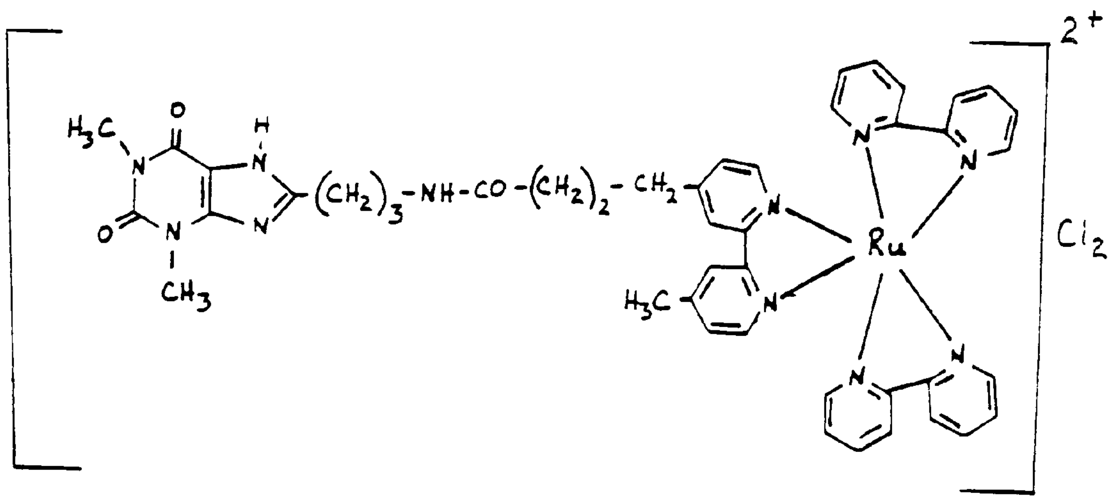

Example 72 - Preparation of bis(2,2'-bipyridine)[1-bromo-4-(4'-methyl-2-2'-bipyridine-4-yl)butane]ruthenium (II) diperchlorate

0.29 g of the ligand 1-bromo-4-(4'-methyl-2,2'-bipyridine-4-yl) butane prepared in Example 29 and 0.32 g of bis (2,2'-bipyridine) ruthenium (II) dichloride were transferred to a flask in 60 ml of 1:1 v/v ethanol/water mixture. The solution was degassed with nitrogen for 15 min. The reaction was heated at reflux under nitrogen for 3 1/2 hrs. An aqueous saturated solution of $NaClO_4$ was added to the cooled red solution. Solvents were removed under vacuum and the deep red residue was chromatographed on an alumina column (20 cm x 2.5 cm, Merck, neutral activity 3) using acetonitrile/toluene 1:1 v/v as the eluant. The product eluted as a dark red band (band #2) on the column. The desired fraction was concentrated, redissolved in approximately 10 ml volume of dichloromethane, precipitated from anhydrous ethyl ether and finally dried under vacuum to yield 320 mg (53%) of the product. The structure of the product was confirmed by elemental analysis and spectral characteristics and is shown below.

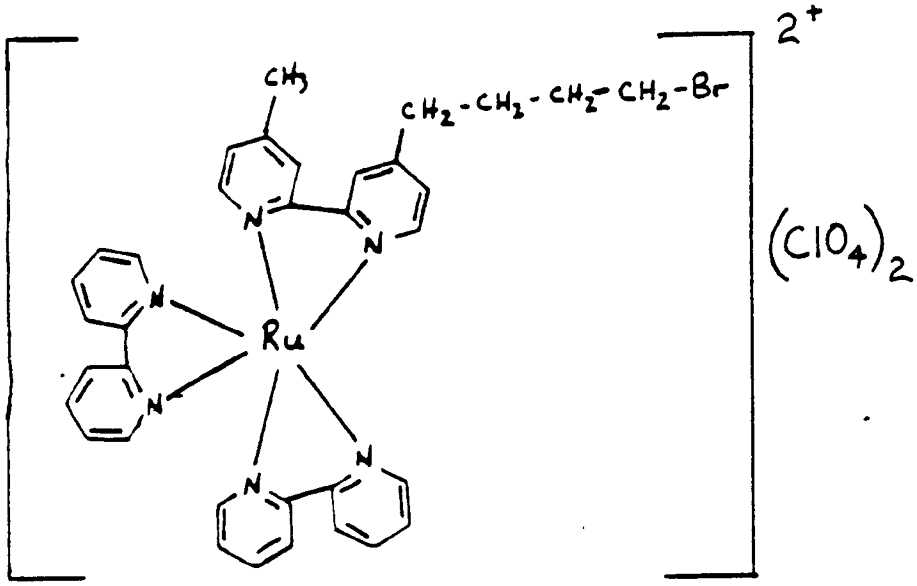

Example 73 - Preparation of bis(2,2'-bipyridine)[theophylline-9-[4-(4-methyl-2,2'bipyridine-4'-yl)butane] ruthenium (II) diperchlorate

The silver salt of theophylline was prepared by mixing together an ammonia solution of theophylline (2.168 g in 25 ml conc. $NH_4OH$ and 75 ml water) and an ammonia solution of silver nitrate (2.006 g in 10 ml conc.

$NH_4OH$ and 30 ml $H_2O$), the reaction being carried out in the dark. Shortly after mixing the two solutions, a white product appeared. Once the precipitation of the product was completed, the precipitate was collected by filtration on a 60 ml medium glass frit. The precipitate was washed with dilute ammonia and dried under vacuum. The structure of the silver salt of theophylline was supported by elemental analysis.

41 mg of the silver salt of theophylline and 122 mg of bis(2,2′-bipyridine)[1-bromo-4-(4′-methyl-2,2′-bipyridine-4-yl)butane] ruthenium (II) diperchlorate were dissovled in 15 ml of anhydrous dimethylformamide (DMF), the reaction being carried out in the dark. The resulting red solution was degassed with argon and heated at reflux for 24 hrs and then cooled to room temperature under argon. Cooling of the solution was continued down to ice temperature and then the black silver metal suspension was filtered off and washed with 5 ml acetone . The red filtrate was treated with 0.2g of lithium perchlorate and after dissolution of $LiClO_4$, the solution was evaporated under vacuum and the red residue was dried overnight in the dark under vacuum. The sample was redissolved in 3-5 ml of methanol and 0.25 g of anhydrous $LiClO_4$ was added and dissolved. The resulting solution was chromatographed on a Sephadex LH-20 Column (75 cm x 19 cm i.d.) using methanol as the eluant. The major product eluted as fraction #2 (dark red band). Fraction #2 was concentrated, redissolved in methanol and precipitated into ethyl ether. The product was dried under vacuum to yield 80 mg (50% yield) of material, the structure of which was confirmed by elemental analysis, infra red and emission spectra.

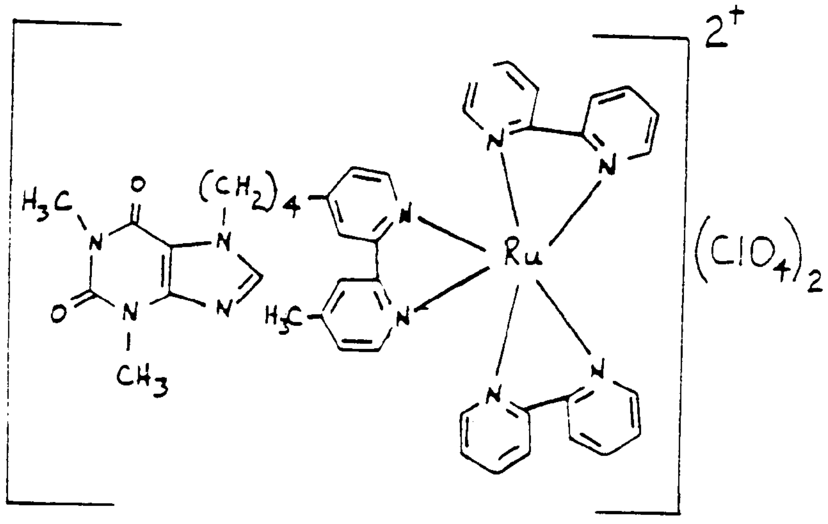

One or more of the following Marks may be Registered Trade Marks in at least one of the territories designated by this application: "TWEEN", "METRICELL", "TITERTEK", "SEPHADEX", "TRITON", "BONDAPAK", "MILLIPORE", "BIOMAG", "DOWEX", "SEPHAROSE" and "X-OMAT"

## REFERENCES

1. Weber, S.G., et al., Photoelectroanalytical Chemistry: Possible Interference in Serum and Selective Detection of Tris (2, 2′ - bypyridine) ruthenium(II) in the Presence of Interferents, Clinical Chemisty, 29, 1665-1672 (1983).

2. Rubinstein, I. and Bard, A.J., Electrogenerated Chemiluminescence. 37. Aqueous ECL Systems Based On Ru (2, 2′ - bypyridine)$_3^{2+}$ and Oxalate or Organic Acids, J. Am. Chem. Soc., 103, 512-516 (1981).

3. White, H.S. and Bard, A.J., Electrogenerated Chemiluminescence. 41. Electrogenerated Chemiluminescence and Chemiluminescence of the Ru (2,2′ - bpy)$_3^{+2}$ - $S_2O_8^{-2}$ System in Acetonitrile - Water Solutions, J. Am. Chem. Soc., 104, 6891 (1982).

4. Curtis, et al., Chemiluminescence; A New Method for Detecting Fluorescent Compounds Separated By Thin Layer Chromatography, J. Chromatography, 134, 343-350 (1977).

5. Sprintschnik, G., et al., Preparation and Photochemical Reactivity of Surfactant Ruthenium (II) Complexes in Monolayer Assemblies and at Water - Solid Interface, J. Am Chem. Soc., 99, 4947-4954 (1977).

6. Minnich, S.A., et al., Enzyme Immunoassay for Detection of Salmonellae in Foods, Appl. and Environ. Micro., 43, 1124-1127 (1982).

7. Thomason, B.M., Current Status of Immunofluorescent Methodology for Salmonellae, J. Food Prot., 44, 381-384 (1981).

8. Mattingly, J.A., An Enzyme Immunoassay for the Detection of All Salmonella Using a Combination of a Myeloma Protein and a Hybridoma Antibody, J. Immunol. Meth., 73, 147-156 (1984).
9. Thompson, N.E. et al., Detection of Staphylococcal enterotoxins by enzyme-linked immunosorbent assays and radio-immunoassays: Comparison of monoclonal and polyclonal antibody systems, Appl. and Environ. Micro., submitted publication.
10. American Public Health Association, Standard methods for the examination of water and wastewater. 15th ed. American Public Health Association, Inc., New York (1980).
11. American Public Health Association, Compendium of methods for the microbiological examination of foods. American Public Health Association, Washington, D.C (1976).
12. Clark, H.F., Geldreich, E.E., Lester, H.L., and Kabler, P.W., The membrane filter in sanitary microbiology, Public Health Rep. 66:951-957 (1951).
13. Feng, P., and Hartman, P.A., Fluorogenic assays for immediate confirmation of Escherichia coli., Appl. Environ. Microbiol. 43:1320-1329 (1982).
14. Geldreich, E.E., Standard method Revisions (16th edition) for Conventional coliform Procedures. In: New developments in drinking water microbiology workshop, 85th Annual Meeting of the American Society for Microbiology (1985).
15. Hussong, D., Colwell, R.R., and Weiner R.M., Rate of occurrence of false-positive results from total coliforms most-probable-number analysis of shellfish and estuaries. Appl. Environ. Microbiol. 40:981-983 (1980).
16. Hussong, D., Demare, J.M., Weiner, R.M., and Colwell, R.R., Bacteria associated with false-positive most-probable-number coliform test results for shellfish and estuaries, Appl. Environ. Microbiol 41:35-45 (1981).
17. Lin, S., Evaluation of coliform tests for chlorinated secondary effluents, J. Water Pollut. Control Fed. 45:498-506 (1973).
18. Mckee, J.E., McLaughlin, R.T. and Lesgourgues, P., Application of molecular filter techniques to the bacterial assay of sewage III. Effects of physical and chemical disinfection, Sewage Ind. Waste 30:245-252 (1958).
19. Mead, J.A.R., Smith, J.N., and Williams, R.T., The biosynthesis of the glucuronides of umbelliferone and 4-methylumbelliferone and their use in fluorimetric determination of beta-glucuronidase, Biochem. J. 61:569-574 (1954).
20. Olson, B.H., Enhanced accuracy of coliform testing in seawater by modification of the most-probable-number method. Apl. Environ. Microbiol, 36:438-444 (1978).
21. Presnell, M.W., Evaluation of membrane filter methods for enumerating coliforms and fecal coliforms in estuarine waters, Proc. National Shellfish Sanitation Workshop. 1974:127-131 (1974).
22. Presswood, W.G. and Strong, D.K., Modification of mFC medium by eliminating rosolic acid, Appl. Environ. Microbiol. 36:90-94 (1978).
23. Warr, G.W. and Marchalonis, J.J., Purification of Antibodies. In: Antibody as a Tool, J. Wiley and Sons, NY, pp. 59-96. (1982)
24. Maniatis, T. , Fritsch, E.F. and Sambrook, J., Molecular Cloning: A Laboratory Manual, p. 150-160, Cold Spring Harbor Press, Cold Spring Harbor, NY (1982).

## Claims

**1.** A method for detecting in a predetermined volume of a multicomponent, liquid sample the presence or absence of an analyte of interest which, if present in the sample, is at a concentration below $10^{-3}$ molar, said method comprising:

a. contacting the sample with a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of combining with the analyte of interest, the contact being effected under conditions such that the analyte and conjugate bind;

b. exposing the resulting sample to an amount of electrochemical energy from a suitable source effective to induce the reagent to repeatedly emit electrochemiluminescence, the exposure being effected

under suitable conditions so as to induce the reagent to repeatedly emit electrochemiluminescence; and

c. detecting emitted luminescence to determine whether the analyte of interest is present in the sample.

2. A method for quantitatively determining in a predetermined volume of a multicomponent, liquid sample the amount present in the sample of an analyte of interest which, if present in the sample, is at a concentration which is below $10^{-3}$ molar, said method comprising:

a. contacting the sample with a known amount of a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of combining with the analyte of interest, the contact being effected under conditions such that the analyte and conjugate bind;

b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and

c. quantitatively determining the amount of emitted luminescence to determine whether and in what amount the analyte of interest is present in the sample.

3. A method according to claim 1 or claim 2 wherein prior to step (b) any reagent which is not combined with the analyte of interest is separated from the sample resulting from step (a).

4. A method according to claim 1 or claim 2 wherein prior to contacting the sample with the reagent in step (a), the sample is treated so as to immobilize the analyte of interest.

5. A method according to any one of claims 1 to 4, wherein the analyte of interest is a whole cell, subcellular particle, virus, prion, viroid, lipid, fatty acid, nucleic acid, polysaccharide other than a sugar, protein, lipoprotein, lipopoly-saccharide, glycoprotein, peptide, cellular metabolite, hormone, pharmacological agent, tranquilizer, barbiturate, alkaloid, steroid, vitamin, amino acid, sugar, non-biological polymer, synthetic organic molecule, organometallic molecule or inorganic molecule.

6. A method according to claim 5, wherein the pharmacological agent is theophylline.

7. A method according to claim 5, wherein the pharmacological agent is digoxin.

8. A method according to claim 5, wherein the hormone is human chorionic gonadotropin.

9. A method according to any one of claims 5 to 8, wherein the analyte of interest is a whole cell, subcellular particle, virus, prion, viroid, nucleic acid, protein, lipoprotein, lipopolysaccharide, glycoprotein, peptide, hormone, pharmacological agent, non-biological polymer, synthetic organic molecule, organometallic molecule or inorganic molecule, and is present in the sample at a concentration below about $10^{-12}$ molar.

10. A method according to claim 9, wherein the analyte of interest is a whole cell, subcellular particle, virus, prion, viroid or nucleic acid, and is present in the sample at a concentration below about $10^{-15}$ molar.

11. A method according to any one of claims 1 to 10, wherein the reagent comprises a metal-containing organic compound conjugated to an antibody, antigen, hapten, ligand, enzyme, biotin, avidin or streptavidin.

12. A method according to any one of claims 1 to 11, wherein the metal of the metal-containing organic compound is selected from ruthenium, osmium, rhenium, iridium, rhodium, platinum, palladium, molybdenum and technetium.

**13.** A method according to claim 12, wherein the metal-containing organic compound is selected from
bis[(4,4′-carbomethoxy)-2,2′-bipyridine]2-[3-(4-methyl-2,2′-bipyridine-4-yl)propyl]-1,3-dioxolane ruthenium (II);
bis (2,2′ bipyridine) [4-(butan-1-al)-4′-methyl-2,2′-bipyridine] ruthenium (II);
bis (2,2′-bipyridine) [4-(4-methyl-2,2′-bipyridine-4′-yl)-butyric acid] ruthenium (II) ;
is (2,2′-bipyridine)[cis-bis(1,2-diphenylphosphino)ethylene]{2-[3-(4-methyl-2,2′-bipyridine-4′-yl)propyl]-1,3-dioxolane}osmium (II) ;
bis(2,2′-bipyridine)[4-(4′-methyl-2,2′-bipyridine)butylamine] ruthenium II ;
bis(2,2′-bipyridine)[1-bromo-4(4′-methyl-2,2′-bipyridine-4-yl)butane] ruthenium (II) ;
bis[(2,2′-bipyridine)-maleimidohexanoic acid, 4-methyl-2,2′-bipyridine-4′-butylamide ruthenium (II) diperchlorate; and
bis[(2,2′-bipyridine)-maleimidohexanoic acid, 4-methyl-2,2′-bipyridine-4′-butylamide ruthenium (II).

**14.** A method as claimed in any one of claims 1 to 12 wherein the reagent comprises a metal containing compound Z attached to a biological entity X where X is selected from one or more nucleotides, which may be the same or different, one or more aminoacids which may be the same or different, an antibody, an analyte of interest or an analogue of an analyte of interest, wherein X may optionally be attached to Z by a linker Y, containing at least one carbon atom and Z is selected from compounds having the following structures:

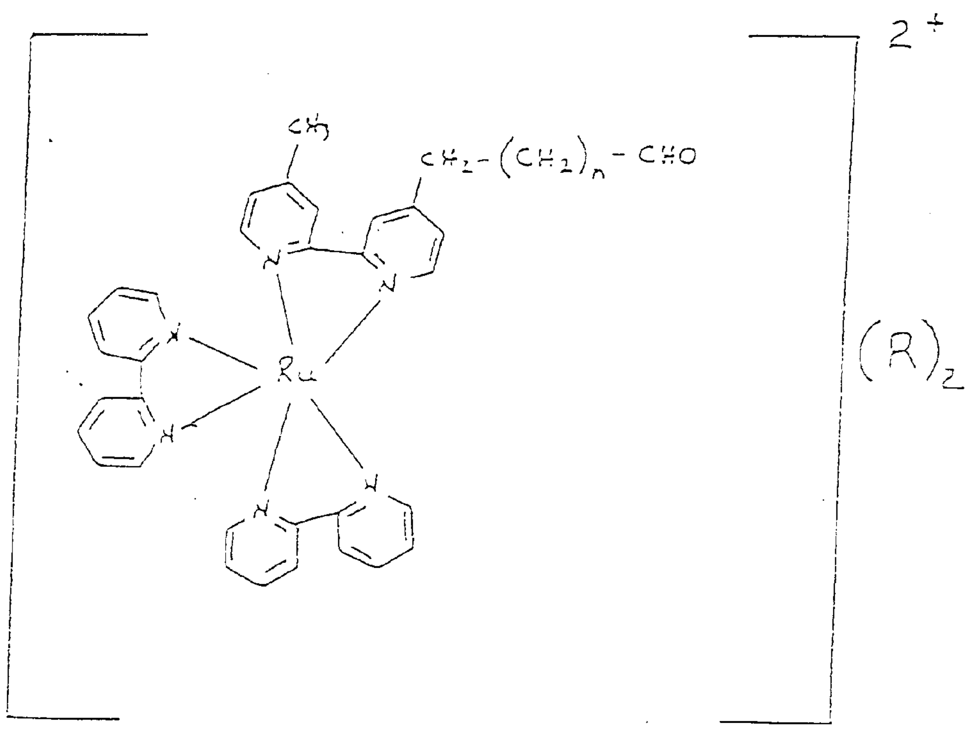

I

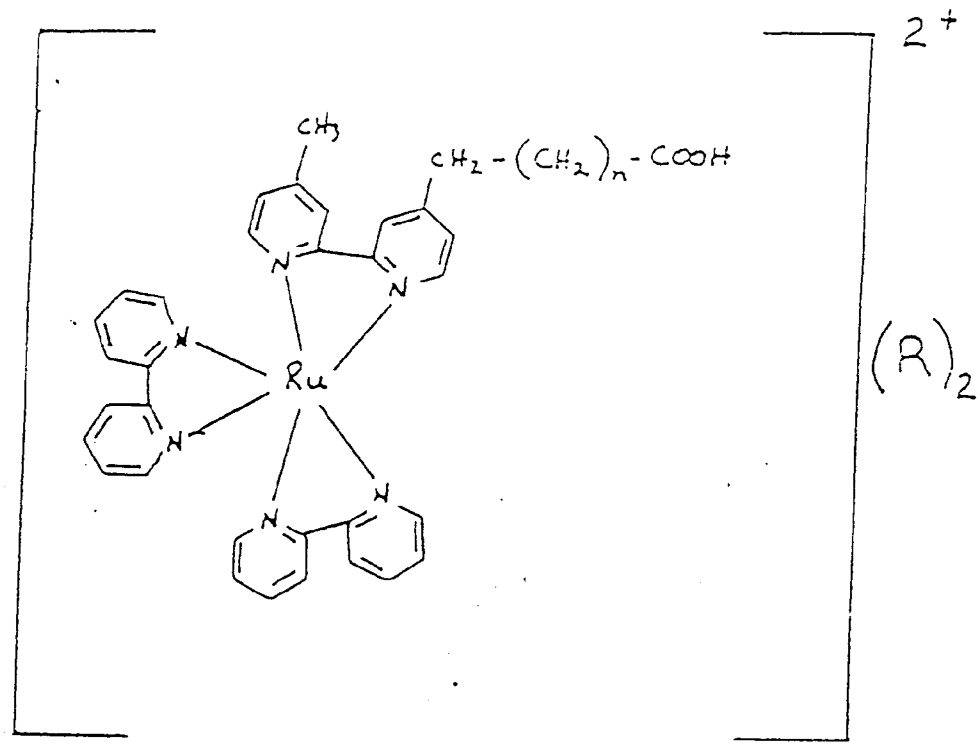

II

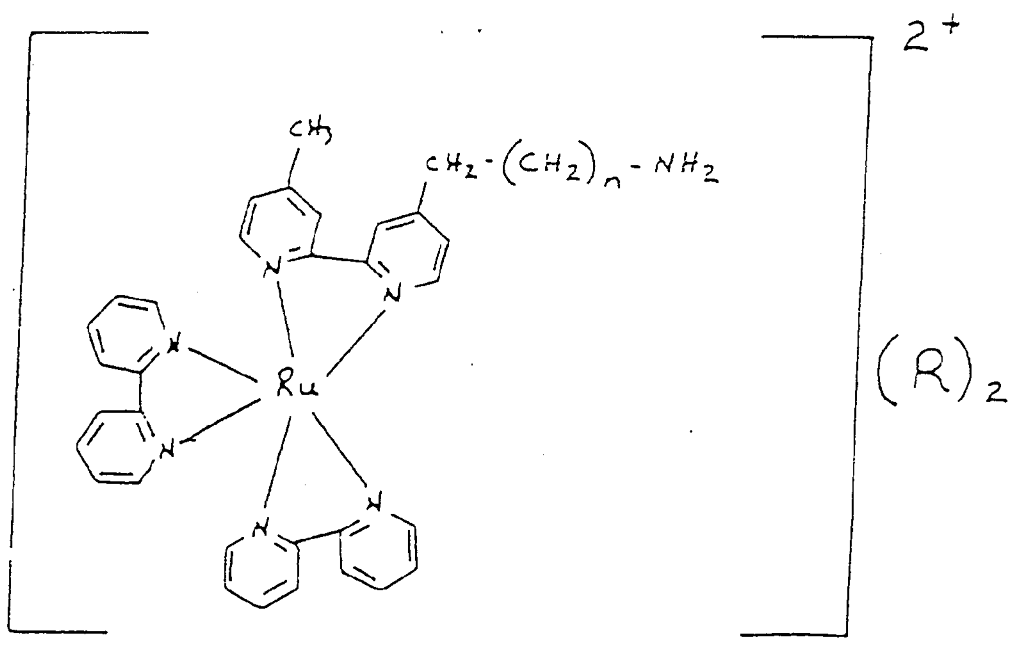

III

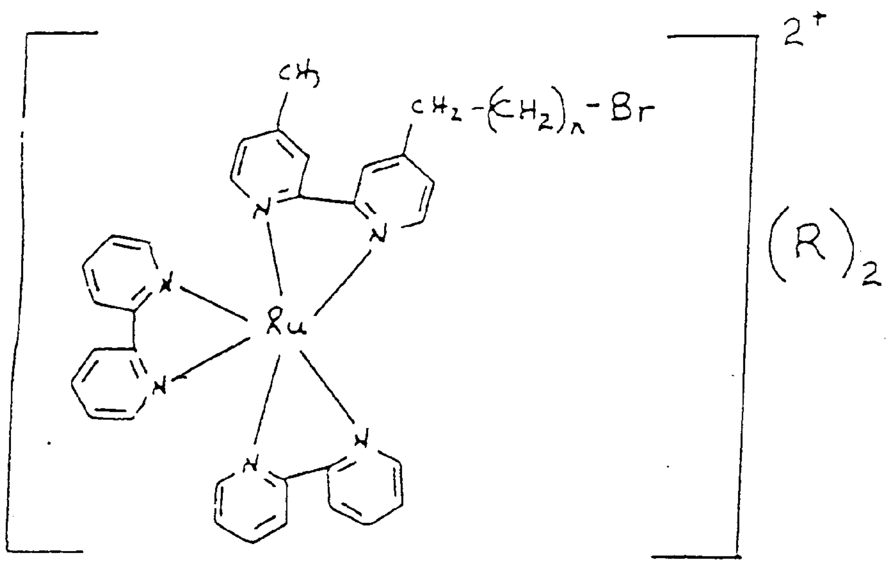

IV

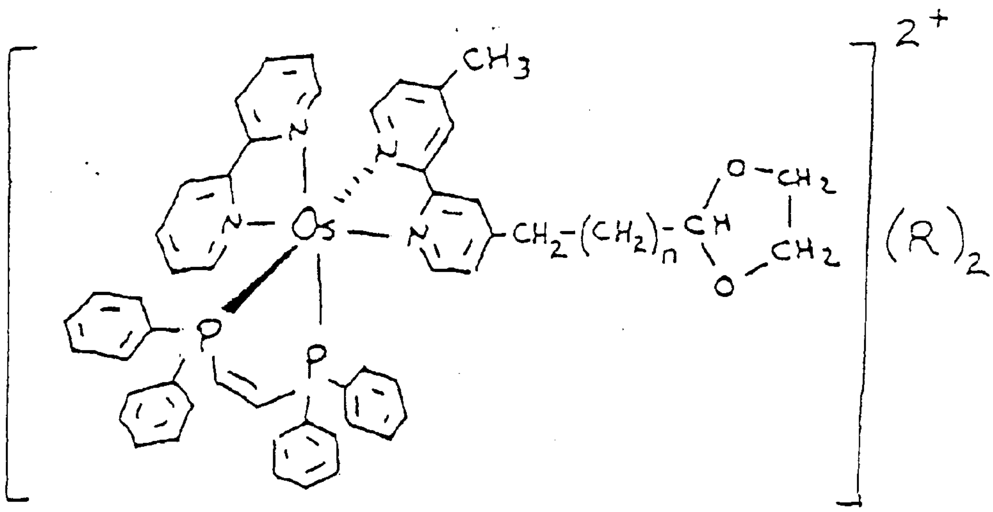

V

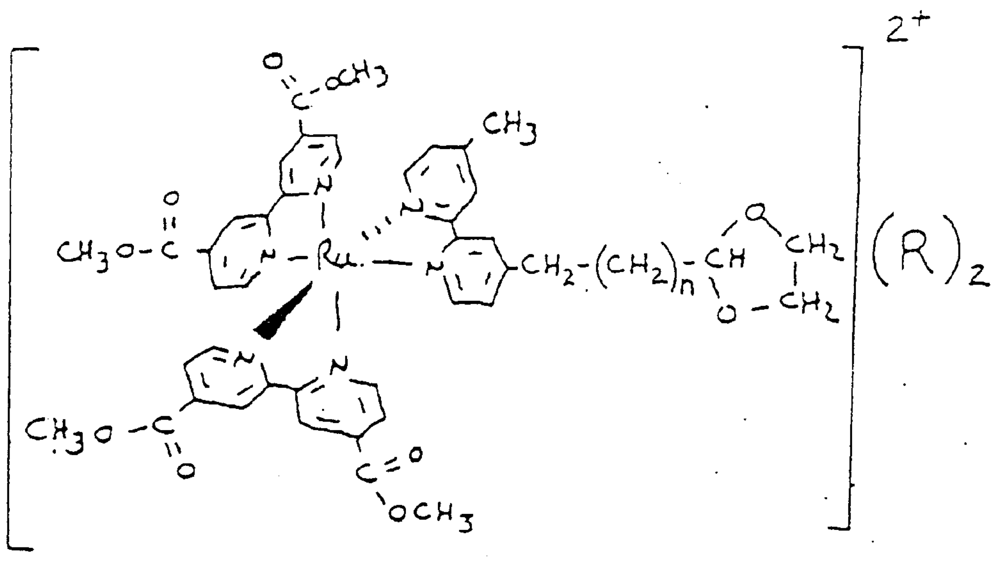

VI

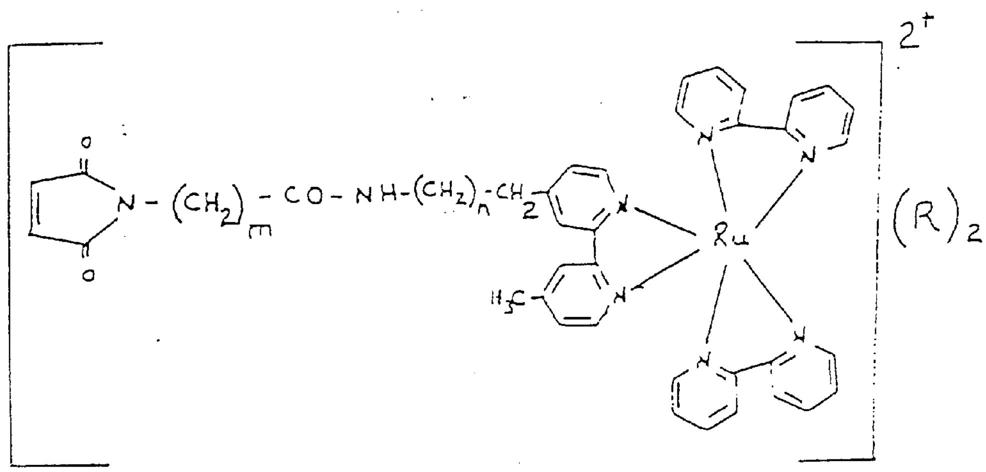

VII

wherein in each of formulae I to VI, R is an anion and n is an integer, and wherein in formula VII R is an anion and m and n are each integers which may be the same or different.

15. A method as claimed in claim 14 wherein in each of formulae I, II, V and VI n is 2.

16. A method as claimed in claim 14 wherein in each of formulae III and IV, n is 3.

17. A method as claimed in claim 14 wherein in formula VII, m is 5 and n is 3.

18. A method as claimed in any one of claims 14 to 17 wherein X is selected from theophylline, digoxigenin and a peptide derived from human chorionic gonadotropin.

19. A method as claimed in any one of claims 1 to 12 wherein the reagent has the structure

$$X\text{-}CH{=}CH\text{-}CO\text{-}NH\text{-}(CH_2)_n\text{-}NH\text{-}CO\text{-}(CH_2)_m\text{-}Z$$

wherein:
X represents one or more nucleotides which may be the same or different;
Z represents a metal-containing organic electrochemiluminescent moiety;

n represents an integer greater than or equal to 1; and
m represents an integer greater than or equal to 1.

**20.** A method as claimed in claim 19 wherein X is thymidine attached to CH at carbon 5, n is 7 and m is 3.

**21.** A method as claimed in claim 20 wherein Z is bis (2,2′-bipyridine) [4-(butan-1-al)-4 methyl 1-2,2′- bipyridine] ruthenium (II).

**22.** A method as claimed in claim 21 wherein the thymidine nucleotide is a 3′ terminal nucleotide attached to the nucleotide sequence

TCACCAATAAACCGCAAACACCATCCCGTCCTGCCAG

**23.** A method as claimed in any one claims 1 to 12 wherein the reagent has the structure

$$[T\text{-}Y\text{-}Z]^{2+}(R)_2$$

wherein:
T represents theophylline;
Y represents a linker group attaching T to Z;
Z represents bis-(2,2′-bipyridine) [4-methyl-2,2′-bipyridine-4′-yl] ruthenium (II); and
R represents an anion.

**24.** A method as claimed in claim 23 wherein Y is attached to the carbon at position 8 of T.

**25.** A method as claimed is claim 24 wherein Y has the structure

$$(CH_2)_m\text{-CO-NH-}(CH_2)_n$$

and wherein m and n each represent an integer, which may be the same or different, greater than or equal to 1.

**26.** A method as claimed in claim 25 wherein m is 3 and n is 4.

**27.** A method as claimed in claim 25 wherein m and n are both 3.

**28.** A method as claimed in claim 24 wherein Y has the structure

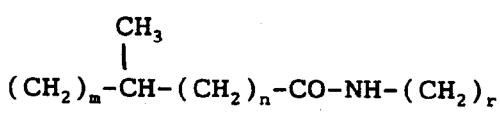

and wherein m, n and r each represent an integer, which may be the same or different, greater than or equal to 1.

**29.** A method as claimed in claim 28 wherein m is 1, n is 1 and r is 4.

**30.** A method as claimed in claim 24 wherein Y has the structure

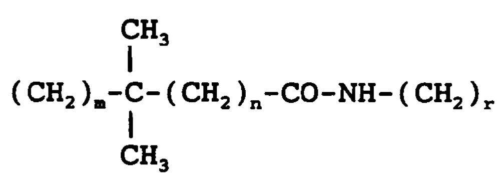

and wherein m, n and r each represent an integer, which may be the same or different, greater than or equal to 1.

**31.** A method as claimed in claim 30 wherein m is 1, n is 1 and r is 4.

**32.** A method as claimed in claim 23 wherein Y is attached to the nitrogen at position 7 of T.

**33.** A method as claimed in claim 32 wherein Y has the structure.

$$(CH_2)_n$$

and wherein n is an integer greater than or equal to 1.

**34.** A method as claimed in claim 33 wherein n is 4.

**35.** A method as claimed in any one of claims 1 to 12 wherein the reagent has the structure

X-Z

wherein:
X represents one or more amino acids which may be the same or different, comprising at least one amino acid which is cysteine or methionine; and
Z is bis(2,2′-bipyridine)maleimidohexonoic acid, 4-methyl-2,2′-bipyridine-4′-butylamide ruthenium (II) attached by the carbon at position 3 or 4 of the maleiimide to a sulfur subsitutent of the cysteine or methionine of X.

**36.** A method according to any one of claims 1 to 35 wherein the sample is derived from a solid, emulsion, suspension, liquid or gas.

**37.** A method according to any one of claims 1 to 36 wherein the sample is derived from water, food, blood, serum, urine, feces, tissue, saliva, oils, organic solvents or air.

**38.** A method according to any one of claims 1 to 37 wherein the sample comprises dimethylsulfoxide, dimethylformamide, n-methylpyrrolidinone or tert-butyl alcohol.

**39.** A method according to claim 38 wherein the sample additionally comprises a reducing agent.

**40.** A method according to claim 38 wherein the sample additionally comprises an oxixizing agent.

**41.** A method for detecting in a predetermined volume of a multicomponent, liquid sample the presence or absence of an analyte of interest which, if present in the sample, is at a concentration below $10^{-3}$ molar, said method comprising:

a. contacting the sample with a complementary material not normally present in the sample and with a reagent (i) comprising a metal-containing organic compound conjugated to a whole cell, subcellular particle, virus, prion, viroid, lipid, fatty acid, nucleic acid, polysaccharide other than a sugar, protein, lipoprotein, lipopolysaccharide, glycoprotein, peptide, cellular metabolite, hormone, pharmacological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy from a suitable source effective to induce the reagent to repeatedly emit electrochemiluminescence and (iii) capable of competing with the analyte of interest for binding sites on the complementary material not normally present in the sample, the contact being effected under appropriate conditions such that the analyte of interest and the conjugate competitively bind to the complementary material;
b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and
c. detecting emitted luminescence to determine whether the analyte of interest is present in the sample.

**42.** A method for quantitatively determining in a predetermined volume of a multicomponent, liquid sample the amount present in the sample of an analyte of interest which, if present in the sample, is at a concentration which is below $10^{-3}$ molar, said method comprising:

a. contacting the sample with a known amount of a complementary material and a known amount of reagent (i) comprising a metal-containing organic compound conjugated to a whole cell, subcellular particle, virus, prion, viroid, lipid, fatty acid, nucleic acid, polysaccharide other than a sugar protein, lipoprotein, lipopolysaccharide, glycoprotein, peptide, cellular metabolite, hormone, pharamcological agent, tranquilizer, barbiturate, alkaloid, steroid, vitamin, amino acid, sugar, nonbiological polymer, organometallic molecule, synthetic organic molecule or inorganic molecule, (ii) capable of being induced to repeatedly emit electrochemiluminescence upon exposure to an amount of electrochemical energy

from a suitable source effective to induce the reagent to repeatedly emit electrochemiluminescence and (iii) capable of competing with the analyte of interest for binding sites on the complementary material not normally present in the sample, the contact being effected under conditions such that the analyte of interest and conjugate competitively bind to the complementary material;
b. exposing the resulting sample to an amount of electrochemical energy from a suitable source effective to induce the reagent to repeatedly emit electrochemiluminescence, the exposure being effected under suitable conditions so as to induce the reagent to repeatedly emit electrochemiluminescence; and
c. quantitatively determining the amount of emitted luminescence to determine whether and in what amount the analyte of interest is present in the sample.

**43.** A method according to claim 41 or claim 42, wherein the analyte of interest is theophylline, digoxin or human chorionic gonadotropin.

**44.** A method according to any one of claims 41 to 43 wherein the reagent is the analyte of interest conjugated to a metal-containing organic compound.

**45.** A method according to any one of claims 41 to 43 wherein the reagent is an analogue of the analyte of interest conjugated to a metal-containing organic compound.

**46.** A method according to any one of claims 41 to 45 wherein the metal-containing organic compound is selected from those specified in any one claims 13 to 35.

**47.** A method according to any one of claims 41 to 46 wherein the complementary material is a whole cell, subcellular particle, virus, prion, viroid, lipid, fatty acid, nucleic acid, polysaccharide, protein, lipoprotein, lipopolysaccharide, glycoprotein, peptide, cellular metabolite, hormone, pharmacological agent, tranquilizer, barbiturate, alkaloid, steroid, vitamin, amino acid, sugar, non-biological polymer, synthetic organic molecule, organometallic molecule or inorganic molecule.

## Patentansprüche

**1.** Verfahren zum Nachweis des Vorhanden- oder Nicht- vorhandenseins eines relevanten Analyten in einem festgelegten Volumen einer flüssigen Multikomponentenprobe, welcher bei Vorhandensein in der Probe eine Konzentration von unter $10^{-3}$ Mol aufweist, bei dem man
a. die Probe mit einem Reagenz in Kontakt bringt, welches (i) eine metallhaltige organische Verbindung umfaßt, die mit einer ganzen Zelle, einem subzellulären Teilchen, einem Virus, einem Prion, Viroid, Lipid, einer Fettsäure, einer Nukleinsäure einem Polysaccharid außer einem Zucker, einem Protein, Lipoprotein, Lipopolysaccharid, Glykoprotein, Peptid, zellulären Metaboliten, Hormon, pharmakologischen Wirkstoff, Beruhigungsmittel, Barbiturat, Alkaloid, Steroid, Vitamin, einer Aminosäure, einem Zucker, einem nichtbiologischen Polymeren, einem organometallischen Molekül, synthetischen organischen Molekül oder anorganischen Molekül konjugiert ist,
(ii) induziert werden kann, wiederholt elektrochemische Lumineszenz auszustrahlen, wenn es dem Einfluß einer Menge an elektrochemischer Energie aus einer geeigneten Quelle ausgesetzt wird, die die Induktion einer wiederholten Ausstrahlung elektrochemischer Lumineszenz durch das Reagenz bewirkt, und
(iii) mit dem relevanten Analyten kombiniert werden kann, wobei der Kontakt unter solchen Bedingungen durchgeführt wird, daß sich der Analyt und das Konjugat aneinander binden;
b) die entstehende Probe der Einwirkung einer Menge an elektrochemischer Energie aus einer geeigneten Quelle aussetzt, die das Reagenz anregen kann, wiederholt elektrochemische Lumineszenz auszusenden, wobei diese Exposition unter solchen Bedingungen erfolgt, daß das Reagenz induziert wird, wiederholt elektrochemische-Lumineszenz auszustrahlen, und
c) die ausgestrahlte Lumineszenz nachweist, um festzustellen, ob der relevante Analyt in der Probe vorhanden ist.

**2.** Verfahren zum quantitativen Nachweis der Menge eines relevanten Analyten in einem festgelegten Volumen einer flüssigen Multikomponentenprobe welcher bei Vorhandensein in der Probe eine Konzentration von unter $10^{-3}$ Mol aufweist, bei dem man

a) die Probe mit einer bekannten Menge eines Reagenz in Kontakt bringt, welches (i) eine metallhaltige organische Verbindung umfaßt, die mit einer ganzen Zelle, einem subzellulären Teilchen, einem Virus, einem Prion, Viroid, Lipid, einer Fettsäure, einer Nukleinsäure, einem Polysaccharid außer einem Zucker, einem Protein, Lipoprotein, Lipopolysaccharid, Glykoprotein, Peptid, zellulären Metaboliten, Hormon, pharmakologischen Wirkstoff, Beruhigungsmittel, Barbiturat, Alkaloid, Steroid, Vitamin, einer Aminosäure, einem Zucker, einem nichtbiologischen Polymeren, einem organometallischen Molekül, synthetischen organischen Molekül oder anorganischen Molekül konjugiert ist

(ii) induziert werden kann, wiederholt elektrochemische Lumineszenz auszustrahlen, wenn es dem Einfluß einer Menge an elektrochemischer Energie aus einer geeigneten Quelle ausgesetzt wird, die die Induktion einer wiederholten Ausstrahlung elektrochemischer Lumineszenz durch das Reagenz bewirkt, und

(iii) mit dem relevanten Analyten kombiniert werden kann, wobei der Kontakt unter solchen Bedingungen durchgeführt wird, daß sich der Analyt und das Konjugat aneinander binden;

b) die entstehende Probe der Einwirkung einer Menge an elektrochemischer Energie aus einer geeigneten Quelle aussetzt, die das Reagenz anregen kann, wiederholt elektrochemische Lumineszenz auszusenden, wobei diese Exposition unter solchen Bedingungen erfolgt, daß das Reagenz induziert wird, wiederholt elektrochemische Lumineszenz auszustrahlen und

c) die ausgestrahlte Lumineszenz quantitativ nachweist, um festzustellen, ob und in welcher Menge der relevante-Analyt in der Probe vorhanden ist.

3. Verfahren nach Anspruch 1 oder 2, bei dem vor Schritt (b) jedes Reagenz, das nicht mit dem relevanten Analyten kombiniert wird, von der aus Schritt (a) resultierenden Probe abgetrennt wird.

4. Verfahren nach Anspruch 1 oder 2, bei dem die Probe behandelt wird, ehe man sie in Schritt (a) in Kontakt mit dem Reagenz bringt, um den relevanten Analyten zu immobilisieren.

5. Verfahren nach einem der Ansprüche 1 bis 4, bei dem der relevante Analyt eine ganze Zelle, ein subzelluläres Teilchen, ein Virus, Prion, Viroid, Lipid, eine Fettsäure, eine Nukleinsäure, ein Polysaccharid außer einem Zucker, ein Protein, Lipoprotein, Lipopolysaccharid, Glykoprotein, Peptid, zellulärer Metabolit, Hormon, pharmakologischer Wirkstoff, Beruhigungsmittel, Barbiturat, Alkaloid, Steroid, Vitamin, eine Aminosäure, ein Zucker, ein nichtbiologisches Polymer, ein synthetisches organisches Molekül, ein organometallisches Molekül oder anorganisches Molekül ist.

6. Verfahren nach Anspruch 5, bei dem der pharmakologische Wirkstoff Theophyllin ist.

7. Verfahren nach Anspruch 5, bei dem der pharmakologische Wirkstoff Digoxin ist.

8. Verfahren nach Anspruch 5, bei dem der pharmakologische Wirkstoff menschliches choriales Gonadotropin (HCG) ist.

9. Verfahren nach einem der Ansprüche 5 bis 8, bei dem der relevante Analyt eine ganze Zelle, ein subzelluläres Teilchen, ein Virus, Prion, Viroid, eine Nukleinsäure, ein Protein, Lipoprotein, Lipopolysaccharid, Glykoprotein, Peptid, Hormon, pharmakologischer Wirkstoff, nichtbiologisches Polymer, synthetisches organisches Molekül, organometallisches Molekül oder anorganisches Molekül ist und in der Probe in einer Konzentration von unter etwa $10^{-12}$ Molar vorhanden ist.

10. Verfahren nach Anspruch 9, bei dem der relevante Analyt eine ganze Zelle, ein subzelluläres Teilchen, ein Virus, Prion, Viroid oder eine Nukleinsäure ist und in der Probe in einer Konzentration von unter etwa $10^{-15}$ Molar vorhanden ist.

11. Verfahren nach einem der Ansprüche 1 bis 10, bei dem das Reagenz eine metallhaltige organische Verbindung umfaßt, die mit einem Antikörper, Antigen, Hapten, Liganden, Enzym, Biotin, Avidin oder Streptavidin konjugiert ist.

12. Verfahren nach einem der Ansprüche 1 bis 11, bei dem das Metall aus der metallhaltigen organischen Verbindung aus Ruthenium, Osmium, Rhenium, Iridium, Rhodium, Platin, Palladium, Molybdän und Technetium ausgewählt ist.

**13.** Verfahren nach Anspruch 12, bei dem die metallhaltige organische Komponente aus
Bis[(4,4′-carbomethoxy)-2,2′-bipyridin]2-[3-(4-methyl-2,2′-bipyridin-4-yl)propyl]-1,3-dioxolanruthenium(II);
Bis(2,2′-bipyridin)-[4-(butan-1-al)-4′-methyl-2,2′-bipyridin]ruthenium(II);
Bis(2,2′-bipyridin)-[4-(4-methyl-2,2′-bypiridin-4′-yl)buttersäure]ruthenium(II);
Bis(2,2-bipyridin)-[cis-bis(1,2-dihenylphosphino)-ethylen]-{2-[3-(4-methyl-2,2′-bipyridin-4′-yl)propyl]1,3-dioxolan}osmium(II);
Bis(2,2′-bipyridin)-[4-(4′-methyl-2,2-bipyridin)butylamin]ruthenium(II);
Bis(2,2′-bipyridin)-[1-brom-4)-(4′-methyl-2,2′-bipyridin-4-yl)butan]ruthenium(II);
Bis[(2,2′-bipyridin)-maleinimidhexansäure,4-methyl-2,2′-bipyridin-4′-butylaminruthenium(II)diperchlorat und
Bis[(2,2′-bipyridin)-maleinimidhexansäure,4-methyl-2,2′-bipyridin-4′-butylamidruthenium(II) ausgewählt ist.

**14.** Verfahren nach einem der Ansprüche 1 bis 12, bei dem das Reagenz eine metallhaltige Verbindung Z umfaßt, welche an einer biologischen Einheit X hängt, wobei X aus einem oder mehreren Nucleotiden, welche gleich oder verschieden sein können, einer oder mehreren Aminosäuren, welche gleich oder verschieden sein können, einem Antikörper, einem Analyten von Interesse oder einem Analog eines Analyten von Interesse ausgewählt ist und wahlweise durch ein Zwischenglied Y, welches mindestens ein Kohlenstoffatom enthält, mit Z verbunden sein kann, und Z aus Verbindungen mit folgenden Strukturen ausgewählt ist:

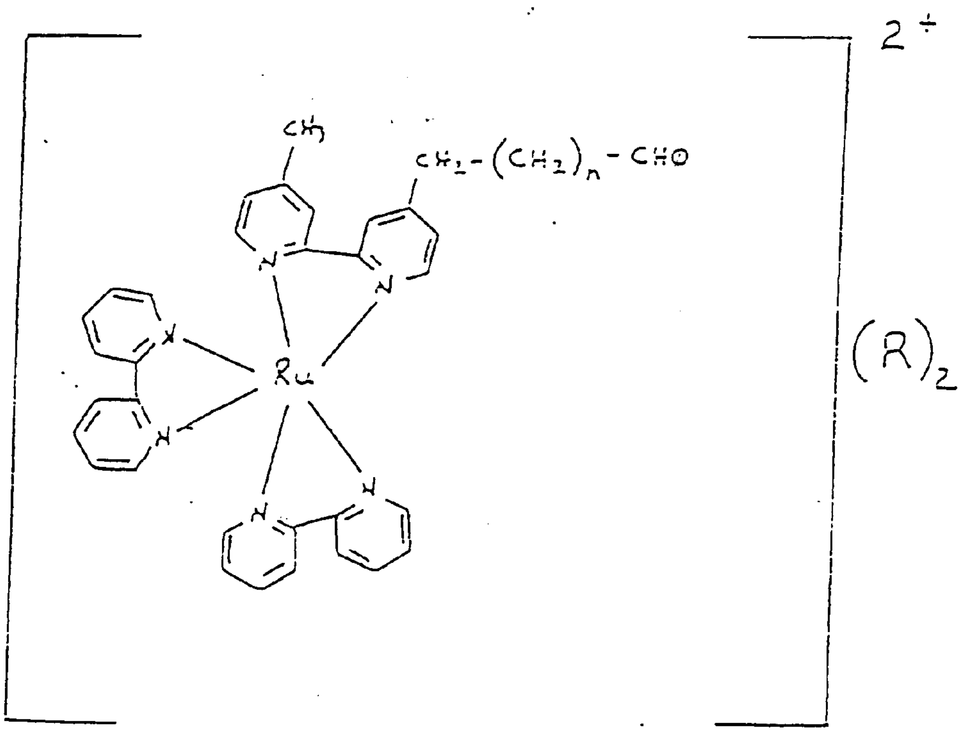

I

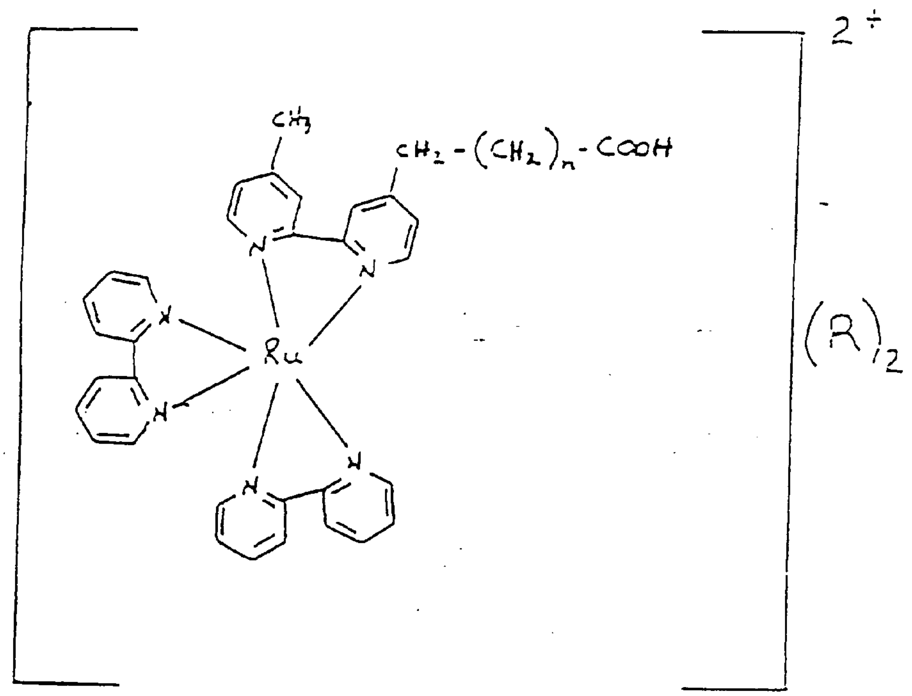

II

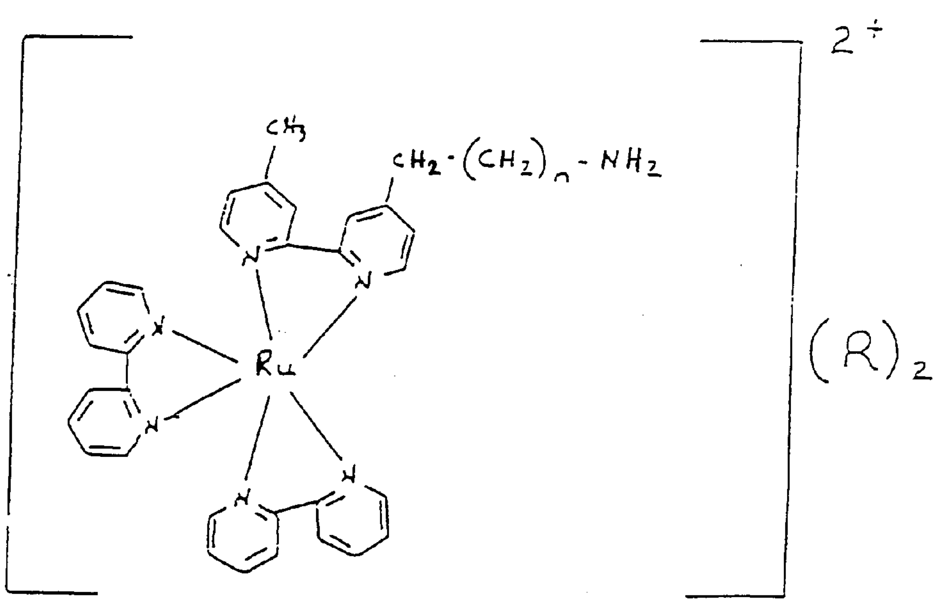

III

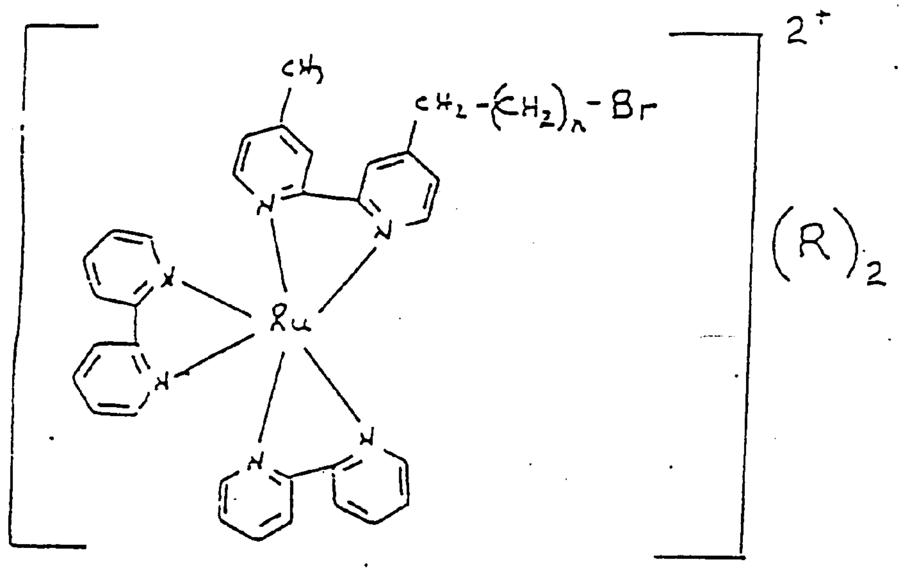

IV

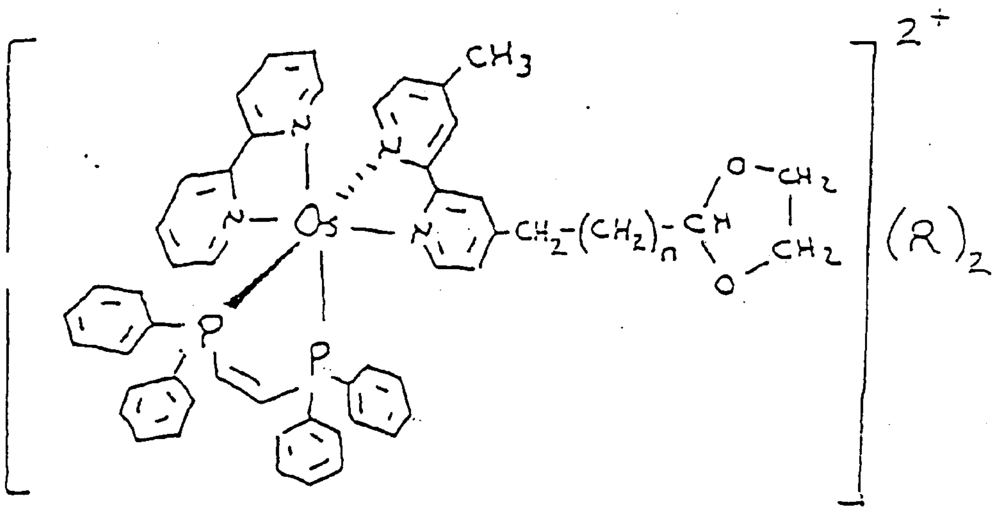

V

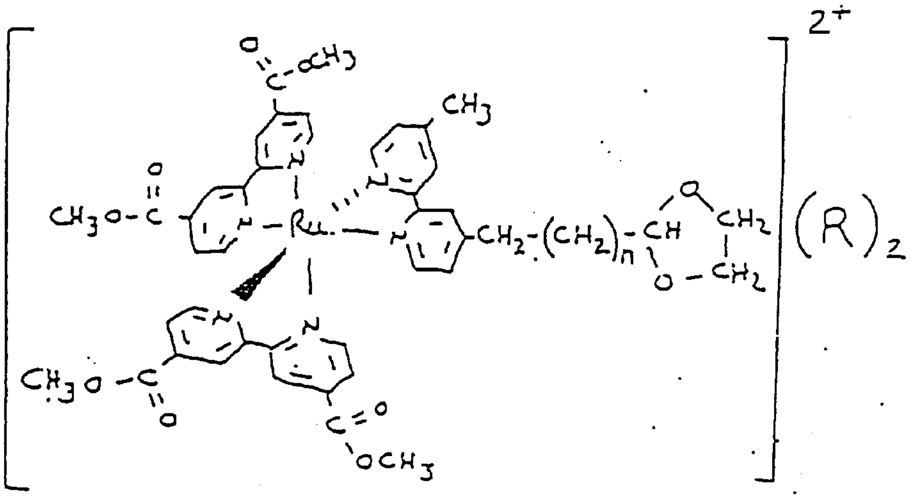

VI

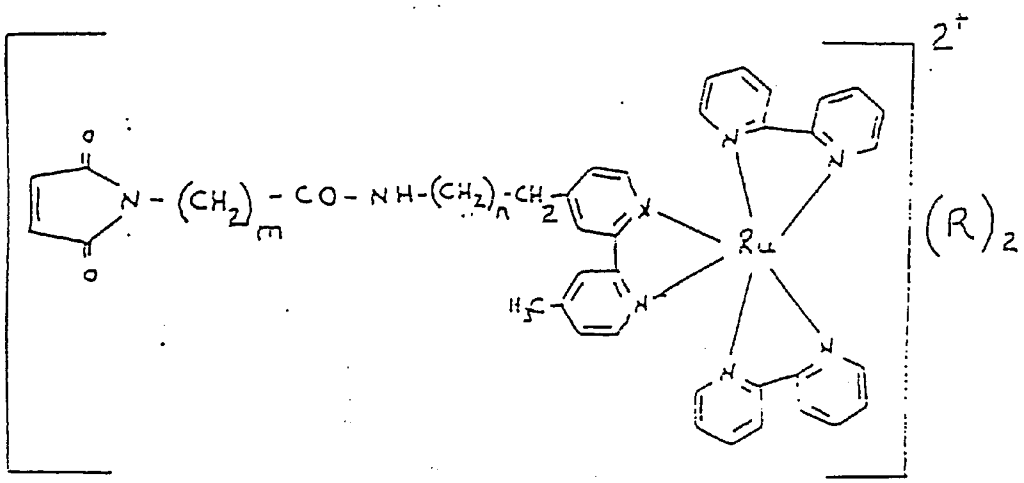

VII

wobei in jeder der Formeln I bis VI ein R Anion und n eine ganze Zahl ist und in der Formel VII R ein Anion ist und m und n jeweils ganze Zahlen sind, die gleich oder verschieden sein können.

**15.** Verfahren nach Anspruch 14, bei dem n in jeder der Formeln I, II, V und VI gleich 2 ist.

**16.** Verfahren nach Anspruch 14, bei dem n in jeder der Formeln III und IV gleich 3 ist.

**17.** Verfahren nach Anspruch 14, bei dem in der Formel VII m 5 und n 3 ist.

**18.** Verfahren nach einem der Ansprüche 14 bis 17, bei dem X aus Theophyllin, Digoxigenin und einem vom menschlichen chorialen Gonadotropin (HCG) abgeleiteten Peptid ausgewählt ist.

**19.** Verfahren nach einem der Ansprüche 1 bis 12, bei dem das Reagenz folgende Struktur hat

$$X\text{-}CH{=}CH\text{-}CO\text{-}NH\text{-}(CH_2)_n\text{-}NH\text{-}CO\text{-}(CH_2)_m\text{-}Z,$$

in der X einen oder mehrere Nucleotiden, die gleich oder verschieden sein können,
Z eine metallhaltige organische elektrochemische lumineszierende Komponente,
n eine ganze Zahl, die größer als oder gleich 1 ist, und
m eine ganze Zahl, die größer als oder gleich 1 ist, bedeutet.

**20.** Verfahren nach Anspruch 19, bei dem X ein beim Kohlenstoff 5 an CH hängendes Thymidin, n 7 und m 3 ist.

**21.** Verfahren nach Anspruch 20, bei dem Z (Bis(2,2′-bipyridin)-[4-(butan-1-al)-4-methyl-1-2,2′-bipyridin]ruthenium(II) ist.

**22.** Verfahren nach Anspruch 21, bei dem das Thymidinnucleotid ein 3′ endständiges Nucleotid ist, das an der Nucleotidsequenz

TCACCAATAAACCGCAAACACCATCCCGTCCTGCCAG

angefügt ist.

**23.** Verfahren nach einem der Ansprüche 1 bis 12, bei dem das Reagenz die Struktur

$$[T\text{-}Y\text{-}Z]^{2+}(R)_2$$

hat, in der
T Theophyllin,
Y eine Verkettungsgruppe, die T mit Z verbindet,
Z Bis(2,2′-bipyridin)-[4-methyl-2,2′-biypridin-4′-yl]ruthenium(II) und
R ein Anion bedeutet.

**24.** Verfahren nach Anspruch 23, bei dem Y am Kohlenstoff in Stellung 8 von T angefügt ist.

**25.** Verfahren nach Anspruch 24, bei dem Y die Struktur

$$(CH_2)_m\text{-CO-NH-}(CH_2)_n$$

aufweist, wobei m und n jeweils eine ganze Zahl darstellen, die gleich oder verschieden und größer als oder gleich 1 sein können.

**26.** Verfahren nach Anspruch 25, bei dem m 3 und n 4 ist.

**27.** Verfahren nach Anspruch 25, bei dem m und n beide 3 bedeuten.

**28.** Verfahren nach Anspruch 24, bei dem Y die Struktur

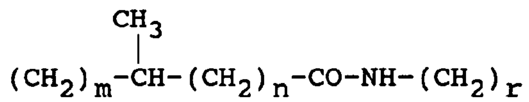

hat, wobei m, n und r jeweils ganze Zahlen bedeuten, die jeweils gleich oder verschieden und größer als oder gleich 1 sein können.

**29.** Verfahren nach Anspruch 28, bei dem m 1, n 1 und r 4 ist.

**30.** Verfahren nach Anspruch 24, bei dem Y die Struktur

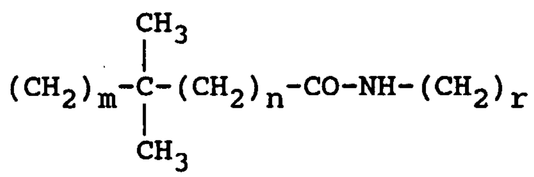

hat, wobei m, n und r jeweils ganze Zahlen bedeuten, die jeweils gleich oder verschieden und größer als oder gleich 1 sein können.

**31.** Verfahren nach Anspruch 30, bei dem m 1, n 1 und r 4 ist.

32. Verfahren nach Anspruch 23, bei dem Y am Stickstoff in Stellung 7 von T hängt.

33. Verfahren nach Anspruch 32, bei dem Y die Struktur

$$(CH_2)_n$$

hat, in der n eine ganze Zahl größer als oder gleich 1 ist.

34. Verfahren nach Anspruch 33, bei dem n 4 ist.

35. Verfahren nach einem der Ansprüche 1 bis 12, bei dem das Reagenz die Struktur

X-Z

hat, in der
X eine oder mehrere Aminosäuren bedeutet, die gleich oder verschieden sein können und mindestens eine Aminosäure umfassen, bei der es- sich um Cystein oder Methionin handelt, und
Z Bis(2,2′-bipyridin)maleinimidhexansäure,4-methyl-2,2-bipyridin-4′-butylamidruthenium(II) bedeutet, das durch den Kohlenstoff an der Stellung 3 oder 4 des Maleinimids an einen Schwefelsubstituenten des Cysteins oder Methionins von X gebunden ist.

36. Verfahren nach einem der Ansprüche 1 bis 35, bei dem die Probe von einem Feststoff, einer Emulsion, Suspension, Flüssigkeit oder einem Gas abgeleitet ist.

37. Verfahren nach einem der Ansprüche 1 bis 36, bei dem die Probe von Wasser, Lebensmitteln, Blut, Serum, Urin, Kot, Gewebe, Speichel, Ölen, organischen Lösungsmitteln oder Luft abgeleitet ist.

38. Verfahren nach einem der Ansprüche 1 bis 37, bei dem die Probe Dimethylsulfoxid, Dimethylformamid, n-Methylpyrrolidon oder tert-Butylalkohol enthält.

39. Verfahren nach Anspruch 38, bei dem die Probe darüber hinaus ein Reduktionsmittel enthält.

40. Verfahren nach Anspruch 38, bei dem die Probe darüber hinaus ein Oxidationsmittel enthält.

41. Verfahren zum Nachweis des Vorhanden- oder Nicht- vorhandenseins eines relevanten Analyten in einem festgelegten Volumen einer flüssigen Multikomponentenprobe, welcher bei Vorhandensein in der Probe eine Konzentration von unter $10^{-3}$ Mol aufweist, bei dem man

a) die Probe mit einem normalerweise nicht darin enthaltenden Komplementärmaterial und einem Reagenz in Kontakt bringt, welches (i) eine metallhaltige organische Verbindung umfaßt, die mit einer ganzen Zelle, einem subzellulären Teilchen, einem Virus, einem Prion, Viroid, Lipid, einer Fettsäure, einer Nukleinsäure, einem Polysaccharid außer einem Zucker, einem Protein, Lipoprotein, Lipopolysaccharid, Glykoprotein, Peptid, zellulären Metaboliten, Hormon, pharmakologischen Wirkstoff, Beruhigungsmittel, Barbiturat, Alkaloid, Steroid, Vitamin, einer Aminosäure, einem Zucker, einem nichtbiologischen Polymeren, einem organometallischen Molekül, synthetischen organischen Molekül oder anorganischen Molekül konjugiert ist,

(ii) induziert werden kann, wiederholt elektrochemische Lumineszenz auszustrahlen, wenn es dem Einfluß einer Menge an elektrochemischer Energie aus einer geeigneten Quelle ausgesetzt wird, die die Induktion einer wiederholten Ausstrahlung elektrochemischer Lumineszenz durch das Reagenz bewirkt, und

(iii) mit dem relevanten Analyten um Bindungsstellen auf dem normalerweise in der Probe nicht vorhandenen Komplementärmaterial in Wettbewerb treten kann, wobei der Kontakt unter geeigneten Bedingungen durchgeführt wird, daß sich der relevante Analyt und das Konjugat kompetitiv an das Komplementärmaterial binden;

b) die entstehende Probe der Einwirkung einer Menge an elektrochemischer Energie aus einer geeigneten Quelle aussetzt, die das Reagenz anregen kann, wiederholt elektrochemische Lumineszenz auszusenden, wobei diese Exposition unter solchen Bedingungen erfolgt, daß das Reagenz induziert wird, wiederholt elektrochemische Lumineszenz auszustrahlen, und

c) die ausgestrahlte Lumineszenz nachweist, um festzustellen, ob der relevante Analyt in der Probe vorhanden ist.

42. Verfahren zum quantitativen Nachweis der Menge eines relevanten Analyten in einem festgelegten Volumen einer flüssigen Multikomponentenprobe, welcher bei Vorhandensein in der Probe eine Konzentra-

tion von unter $10^{-3}$ Mol aufweist, bei dem man

a) die Probe mit einer bekannten Menge eines Komplementärmaterials und einer bekannten Menge eines Reagenz in Kontakt bringt, welches (i) eine metallhaltige organische Verbindung umfaßt, die mit einer ganzen Zelle, einem subzellulären Teilchen, einem Virus, einem Prion, Viroid, Lipid, einer Fettsäure, einer Nukleinsäure, einem Polysaccharid außer einem Zucker, einem Protein, Lipoprotein, Lipopolysaccharid, Glykoprotein, Peptid, zellulären Metaboliten, Hormon, pharmakologischen Wirkstoff, Beruhigungsmittel, Barbiturat, Alkaloid, Steroid, Vitamin, einer Aminosäure, einem Zucker, einem nichtbiologischen Polymeren, einem organometallischen Molekül, synthetischen organischen Molekül oder anorganischen Molekül konjugiert ist,

(ii) induziert werden kann, wiederholt elektrochemische Lumineszenz auszustrahlen, wenn es dem Einfluß einer Menge an elektrochemischer Energie aus einer geeigneten Quelle ausgesetzt wird, die die Induktion einer wiederholten Ausstrahlung elektrochemischer Lumineszenz durch das Reagenz bewirkt, und

(iii) mit dem relevanten Analyten in Wettbewerb um Bindungsstellen auf dem normalerweise nicht in der Probe vorhandenen Komplementärmaterial treten kann, wobei der Kontakt unter solchen Bedingungen durchgeführt wird, daß sich der relevante Analyt und das Konjugat kompetitiv an das Komplementärmaterial binden;

b) die entstehende Probe der Einwirkung einer Menge an elektrochemischer Energie aus einer geeigneten Quelle aussetzt, die das Reagenz anregen kann, wiederholt elektrochemische Lumineszenz auszusenden, wobei diese Exposition unter solchen Bedingungen erfolgt, daß das Reagenz induziert wird, wiederholt elektrochemische Lumineszenz auszustrahlen und

c) die ausgestrahlte Lumineszenz quantitativ nachweist, um festzustellen, ob und in welcher Menge der relevante Analyt in der Probe vorhanden ist.

**43.** Verfahren nach Anspruch 41 oder 42, bei dem der relvante Analyt Theophyllin, Digoxin oder menschliches choriales Gonadotropin (HCG) ist.

**44.** Verfahren nach einem der Ansprüche 41 bis 43, bei dem das Reagenz der relevante Analyt ist, der mit einer metallhaltigen organischen Verbindung konjugiert ist.

**45.** Verfahren nach einem der Ansprüche 41 bis 43, bei dem das Reagenz ein Analogon des relevanten Analyten ist, der mit einer metallhaltigen organischen Verbindung konjugiert ist.

**46.** Verfahren nach einem der Ansprüche 1 bis 45, bei dem die metallhaltige organische Verbindung aus den in einem der Ansprüche 13 bis 35 spezifizierten Verbindungen ausgewählt ist.

**47.** Verfahren nach einem der Ansprüche 41 bis 46, bei dem das Komplementärmaterial eine ganze Zelle, ein subzelluläres Teilchen, ein Virus, Prion, Viroid, Lipid, eine Fettsäure, eine Nukleinsäure, ein Polysaccharid, ein Protein, Lipoprotein, Lipopolysaccharid, Glykoprotein, Peptid, zellulärer Metabolit, Hormon, pharmakologischer Wirkstoff, Beruhigungsmittel, Barbiturat, Alkaloid, Steroid, Vitamin, eine Aminosäure, ein Zucker, ein nichtbiologisches Polymer, ein synthetisches organisches Molekül, ein organometallisches Molekül oder anorganisches Molekül ist.

## Revendications

**1.** Méthode pour détecter dans un volume prédéterminé d'un échantillon liquide à composants multiples, la présence ou l'absence d'un analyte intéressant, qui, s'il est présent dans l'échantillon, est à une concentration inférieure à $10^{-3}$ molaire, ladite méthode comprenant :

(a) la mise en contact de l'échantillon avec un réactif

- (i) comprenant un composé organique contenant un métal, conjugué à une cellule entière, une particule subcellulaire, un virus, un prion, un viroïde, un lipide, un acide gras, un acide nucléique, un polysaccharide autre qu'un sucre, une protéine, une lipoprotéine, un lipopolysaccharide, une glycoprotéine, un peptide, un métabolite cellulaire, une hormone, un agent pharmacologique, un tranquillisant, un barbirurate, un alcaloïde, un stéroïde, une vitamine, un acide aminé, un sucre, un polymère non biologique, une molécule organométallique, une molécule organique de synthèse, ou une molécule minérale,
- (ii) capable d'être induit à émettre de façon répétée, une électrochimiluminescence, par exposition à une quantité d'énergie électrochimique provenant d'une source appropriée, efficace pour induire le réactif à émettre une électrochimiluminescence de façon répétée, et

- (iii) capable de se combiner avec l'analyte intéressant, le contact étant effectué dans des conditions telles, que l'analyte et le conjugué se lient;

(b) l'exposition de l'échantillon obtenu à une quantité d'énergie électrochimique provenant d'une source appropriée, efficace pour induire le réactif, à émettre une électrochimiluminescence de façon répétée, l'exposition étant effectuée dans des conditions adéquates, pour amener le réactif à émettre une électrochimiluminescence de façon répétée; et

(c) la détection de la luminescence émise, pour déterminer si l'analyte intéressant est présent dans l'échantillon.

2. Méthode pour la détermination quantitative dans un volume prédéterminé d'un échantillon liquide à composants multiples, de la présence ou de l'absence d'un analyte intéressant, qui, s'il est présent dans l'échantillon, est à une concentration inférieure à $10^{-3}$ molaire, ladite méthode comprenant :

(a) la mise en contact de l'échantillon avec une quantité connue d'un réactif

- (i) comprenant un composé organique contenant un métal, conjugué à une cellule entière, une particule subcellulaire, un virus, un prion, un viroïde, un lipide, un acide gras, un acide nucléique, un polysaccharide autre qu'un sucre, une protéine, une lipoprotéine, un lipopolysaccharide, une glycoprotéine, un peptide, un métabolite cellulaire, une hormone, un agent pharmacologique, un tranquillisant, un barbirurate, un alcaloïde, un stéroïde, une vitamine, un acide aminé, un sucre, un polymère non biologique, une molécule organométallique, une molécule organique de synthèse, ou une molécule minérale,
- (ii) capable d'être induit à émettre de façon répétée, une électrochimiluminescence, par exposition à une quantité d'énergie électrochimique provenant d'une source appropriée, efficace pour induire le réactif, à émettre une électrochimiluminescence de façon répétée, et
- (iii) capable de se combiner avec l'analyte intéressant, le contact étant effectué dans des conditions telles, que l'analyte et le conjugué se lient;

(b) l'exposition de l'échantillon obtenu à une quantité d'énergie électrochimique provenant d'une source appropriée, efficace pour induire le réactif, à émettre une électrochimiluminescence de façon répétée, l'exposition étant effectuée dans des conditions adéquates, pour amener le réactif à émettre une électrochimiluminescence de façon répétée; et

(c) la détermination quantitative de la quantité de luminescence émise, pour déterminer si l'analyte intéressant est présent dans l'échantillon, et en quelle quantité.

3. Méthode selon la revendication 1, ou la revendication 2, dans laquelle, avant l'étape (b), tout réactif qui n'est pas combiné avec l'analyte intéressant, est séparé de l'échantillon obtenu à l'étape (a).

4. Méthode selon la revendication 1, ou la revendication 2, dans laquelle, avant la mise en contact de l'échantillon avec le réactif à l'étape (a), l'échantillon est traité de façon à immobiliser l'analyte intéressant.

5. Méthode selon l'une quelconque des revendications 1 à 4, dans laquelle l'analyte intéressant est une cellule entière, une particule subcellulaire, un virus, un prion, un viroïde, un lipide, un acide gras, un acide nucléique, un polysaccharide autre qu'un sucre, une protéine, une lipoprotéine, un lipopolysaccharide, une glycoprotéine, un peptide, un métabolite cellulaire, une hormone, un agent pharmacologique, un tranquillisant, un barbirurate, un alcaloïde, un stéroïde, une vitamine, un acide aminé, un sucre, un polymère non biologique, une molécule organique de synthèse, une molécule organométallique, ou une molécule minérale.

6. Méthode selon la revendication 5, dans laquelle l'agent pharmacologique est la théophylline.

7. Méthode selon la revendication 5, dans laquelle l'agent pharmacologique est la digoxine.

8. Méthode selon la revendication 5, dans laquelle l'hormone est la gonadotrophine chorionique humaine.

9. Méthode selon l'une quelconque des revendications 5 à 8, dans laquelle l'analyte intéressant est une cellule entière, une particule subcellulaire, un virus, un prion, un viroïde, un acide nucléique, une protéine, une lipoprotéine, un lipopolysaccharide, une glycoprotéine, un peptide, une hormone, un agent pharmacologique, un polymère non biologique, une molécule organique de synthèse, une molécule organométallique, ou une molécule minérale, et est présent dans l'échantillon à une concentration inférieure à environ $10^{-12}$ molaire.

**10.** Méthode selon la revendication 9, dans laquelle l'analyte intéressant est une cellule entière, une particule subcellulaire, un virus, un prion, un viroïde, ou un acide nucléique, et est présent dans l'échantillon à une concentration inférieure à environ $10^{-15}$ molaire.

**11.** Méthode selon l'une quelconque des revendications 1 à 10, dans laquelle le réactif comprend un composé organique contenant un métal, conjugué à un anticorps, à un antigène, à un haptène, à un ligand, à la biotine, à l'avidine ou à la streptavidine.

**12.** Méthode selon l'une quelconque des revendications 1 à 11, dans laquelle le métal du composé organique contenant un métal est choisi parmi le ruthénium, l'osmium, le rhénium, l'iridium, le rhodium; le platine, le palladium, le molybdène, et le technétium.

**13.** Méthode selon la revendication 12, dans laquelle le composé organique contenant un métal est choisi parmi :

- le bis[(4,4′-carbométhoxy)-2,2′-bipyridine]-2-[3-(4-méthyl-2,2′-bipyridin-4-yl)propyl]-1,3-dioxolane ruthénium (II);
- le bis(2,2′-bipyridine)-[4-(butan-1-al)-4′-méthyl-2,2′-bipyridine] ruthénium (II);
- le bis(2,2′-bipyridine)-[acide 4-(4-méthyl-2,2′-bipyridin-4-yl)-butyrique] ruthénium (II);
- le bis(2,2′-bipyridine)-[cis-bis(1,2-diphénylphosphino)éthylène]{2-[3-(4-méthyl-2,2′-bipyridin-4′-yl)propyl]-1,3-dioxolane} osmium (II);
- le bis(2,2′-bipyridine)-[4-(4′-méthyl-2,2′-bipyridine)butylamine] ruthénium (II);
- le bis(2,2′-bipyridine)-[1-bromo-4-(4′-méthyl-2,2′-bipyridin-4-yl)butane] ruthénium (II);
- le bis[(2,2′-bipyridine)-maléimidohexanoïque acide, 4-méthyl-2,2′-bipyridine-4′-butylamide ruthénium (II) diperchlorate; et
- le bis[(2,2′-bipyridine)-maléimidohexanoïque acide, 4-méthyl-2,2′-bipyridine-4′-butylamide ruthénium (II);

**14.** Méthode selon l'une quelconque des revendications 1 à 12. dans laquelle le réactif comprend un composé Z contenant un métal, attaché à une entité biologique X, X étant choisi parmi un ou plusieurs nucléotides, qui peuvent être identiques ou différents, un ou plusieurs acides aminés, qui peuvent être identiques ou différents, un anticorps, un analyte intéressant ou un analogue d'un analyte intéressant, X pouvant éventuellement être attaché à Z par un agent de liaison Y comportant au moins un atome de carbone, et Z étant choisi parmi les composés ayant les structures suivantes :

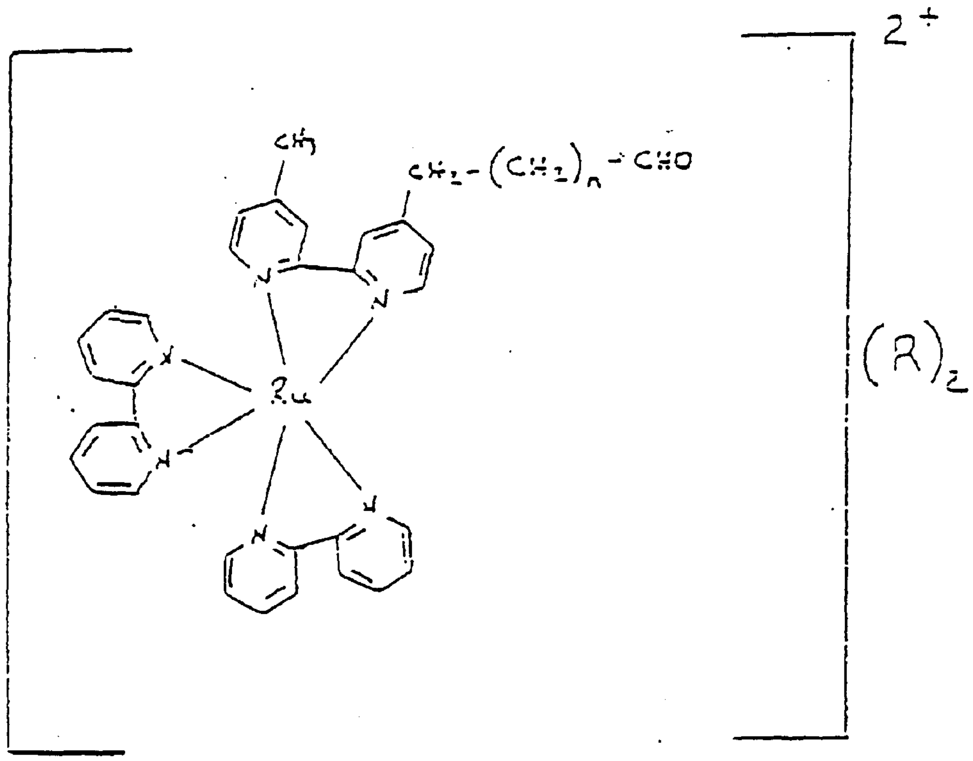

I

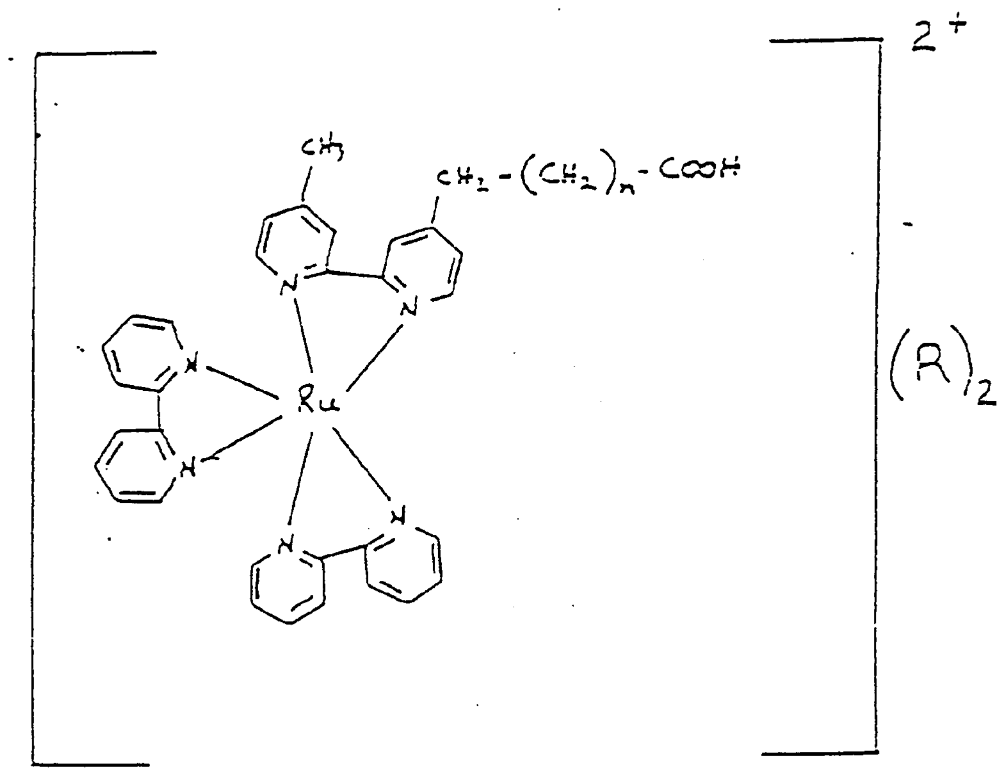

II

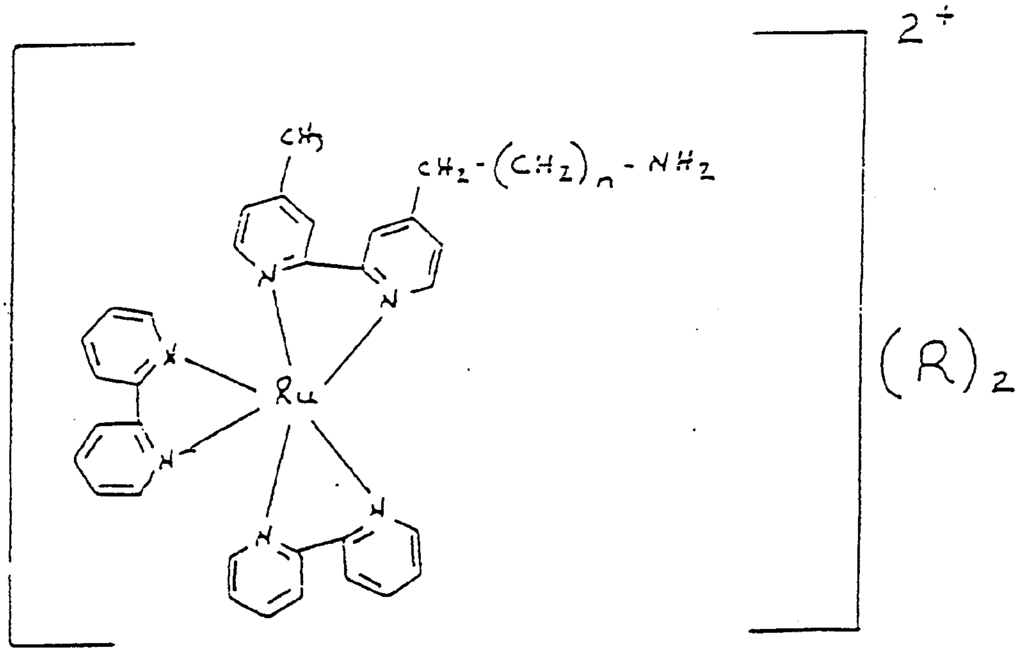

III

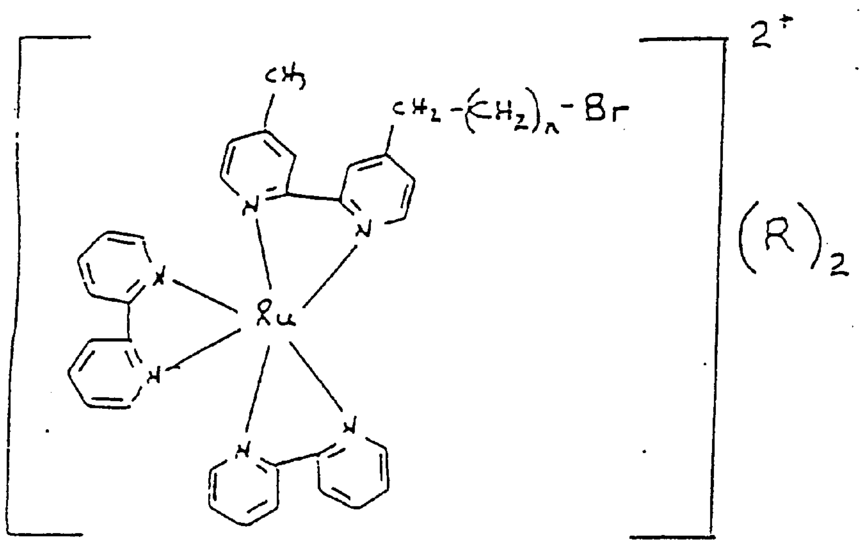

IV

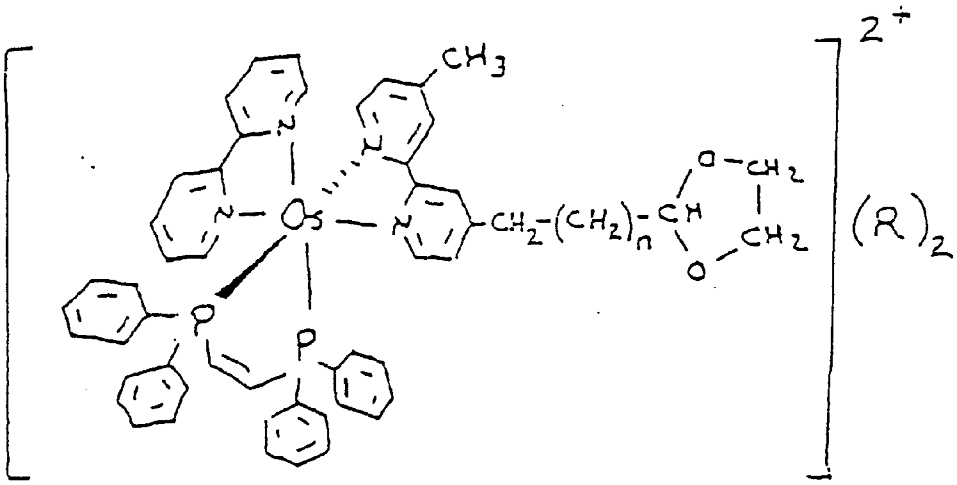

V

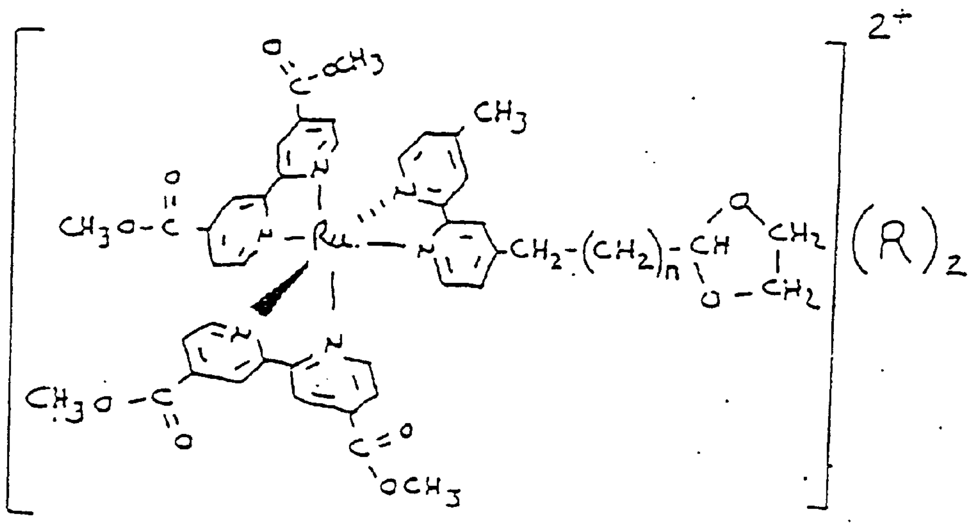

VI

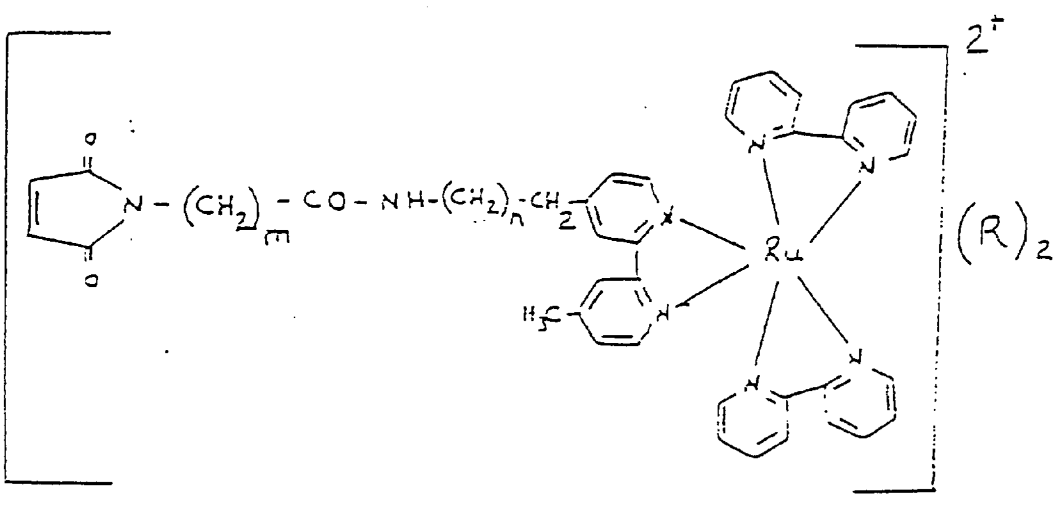

V

dans lesquelles, pour chacune des formules I à VI, R est un anion et n est un entier, et dans lesquelles pour la formule VII, R est un anion, et m et n qui sont chacun un entier, peuvent être identiques ou différents.

**15.** Méthode selon la revendication 14, dans laquelle dans chacune des formules I, II, V, et VI, n est égal à 2.

**16.** Méthode selon la revendication 14, dans laquelle dans chacune des formules III, et IV, n est égal à 3.

**17.** Méthode selon la revendication 14, dans laquelle dans la formule VII, m est égal à 5 et n est égal à 3.

**18.** Méthode selon l'une quelconque des revendications 14 à 17, dans laquelle X est choisi parmi la théophylline, la digoxigénine, et un peptide dérivé de la gonadotrophine chorionique humaine.

**19.** Méthode selon l'une quelconque des revendications 1 à 12, dans laquelle le réactif a la structure :

$$X\text{-}CH{=}CH\text{-}CO\text{-}NH\text{-}(CH_2)_n\text{-}NH\text{-}CO\text{-}(CH_2)_m\text{-}Z$$

dans laquelle :

- X représente un ou plusieurs nucléotides qui peuvent être identiques ou différents;
- Z représente une entité organique électrochimiluminescente contenant un métal;
- n représente un entier supérieur ou égal à 1; et
- m représente un entier supérieur ou égal à 1.

**20.** Méthode selon la revendication 19, dans laquelle X est la thymidine attachée au CH sur le carbone 5, n est égal à 7, et m est égal à 3.

**21.** Méthode selon la revendication 20, dans laquelle Z est le bis(2,2′-bipyridine)-[4-(butan-1-al)-4-méthyl-1,2,2′-bipyridine] ruthénium (II).

**22.** Méthode selon la revendication 21, dans laquelle le nucléotide de la thymidine est un nucléotide 3′ terminal attaché à la séquence :

TCACCAATAAACCGCAAACACCATCCCGTCCTGCCAG.

**23.** Méthode selon l'une quelconque des revendications 1 à 12, dans laquelle le réactif a la structure :

$$[T\text{-}Y\text{-}Z]^{2+}(R)_2$$

dans laquelle :

- T représente la théophylline;
- Y représente un agent de liaison attachant T à Z;
- Z représente le bis(2,2′-bipyridine)-[4-méthyl-2,2′-bipyridin-4′-yl] ruthénium (II); et
- R représente un anion.

**24.** Méthode selon la revendication 23, dans laquelle Y est attaché à l'atome de carbone en position 8, de T.

**25.** Méthode selon la revendication 24, dans laquelle Y a la structure :

$$(CH_2)_m\text{-}CO\text{-}NH\text{-}(CH_2)_n$$

dans laquelle m et n représentent chacun des entiers qui peuvent être identiques ou différents, supérieurs ou égaux à 1.

**26.** Méthode selon la revendication 25, dans laquelle m est égal à 3, et n est égal à 4.

**27.** Méthode selon la revendication 25, dans laquelle m et n sont tous deux égaux à 3.

**28.** Méthode selon la revendication 24, dans laquelle Y a la structure :

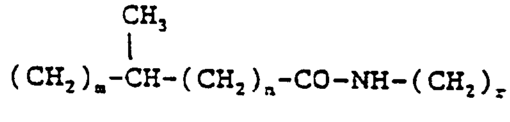

dans laquelle m, n, et r représentent chacun des entiers qui peuvent être identiques ou différents, supérieurs ou égaux à 1.

**29.** Méthode selon la revendication 28, dans laquelle m est égal à 1, n est égal à 1, et r est égal à 4.

**30.** Méthode selon la revendication 24, dans laquelle Y a la structure :

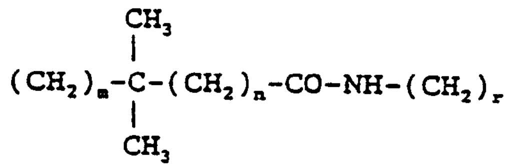

dans laquelle n, m, et r représentent chacun des entiers pouvant être identiques ou différents, supérieurs ou égaux à 1.

**31.** Méthode selon la revendication 30, dans laquelle m est égal à 1, n est égal à 1, et r est égal à 4.

**32.** Méthode selon la revendication 23, dans laquelle Y est attaché à l'atome d'azote en position 7, de T.

**33.** Méthode selon la revendication 32, dans laquelle Y a la structure :

$$(CH_2)_n$$

dans laquelle n est un entier supérieur ou égal à 1.

**34.** Méthode selon la revendication 33, dans laquelle n est égal à 4.

**35.** Méthode selon l'une quelconque des revendications 1 à 12, dans laquelle le réactif a la structure :

X-Z

dans laquelle :

- X représente un ou plusieurs acides aminés qui peuvent être identiques ou différents, comprenant au moins un acide aminé qui est la cystéine ou la méthionine; et
- Z est le bis(2,2′-bipyridine)maléimidohexanoïque acide, 4-méthyl-2,2′-bipyridine-4′-butylamide ruthénium (II) attaché par l'atome de carbone en position 3 ou 4 du maléimide, à un substituant soufré de la cystéine ou de la méthionine, de X.

**36.** Méthode selon l'une quelconque des revendications 1 à 35, dans laquelle l'échantillon est dérivé d'un solide, d'une émulsion, d'une suspension, d'un liquide, ou d'un gaz.

**37.** Méthode selon l'une quelconque des revendications 1 à 36, dans laquelle l'échantillon est dérivé de l'eau, de produits alimentaires, du sang, du sérum, de l'urine, de matières fécales, de tissu, de la salive, d'huiles, de solvants organiques, ou de l'air.

**38.** Méthode selon l'une quelconque des revendications 1 à 37, dans laquelle l'échantillon comprend du diméthylsulfoxyde, du diméthylformamide, de la N-méthylpyrrolidinone, ou de l'alcool terbutylique.

**39.** Méthode selon la revendication 38, dans laquelle l'échantillon comprend en plus, un agent réducteur.

**40.** Méthode selon la revendication 38, dans laquelle l'échantillon comprend en plus, un agent oxydant.

**41.** Méthode pour la détection dans un volume prédéterminé d'un échantillon liquide à composants multiples, de la présence ou de l'absence d'un analyte intéressant, qui, s'il est présent dans l'échantil-lon, est à une concentration inférieure à $10^{-3}$ molaire, ladite méthode comprenant :

(a) la mise en contact de l'échantillon avec un matériau complémentaire, normalement non présent dans l'échantillon, et avec un réactif

- (i) comprenant un composé organique contenant un métal, conjugué à une cellule entière, une particule subcellulaire, un virus, un prion, un viroïde, un lipide, un acide gras, un acide nucléique, un polysaccharide autre qu'un sucre, une protéine, une lipoprotéine, un lipopolysaccharide, une

glycoprotéine, un peptide, un métabolite cellulaire, une hormone, un agent pharmacologique, un tranquillisant, un barbirurate un alcaloïde, un stéroïde, une vitamine, un acide aminé, un sucre, un polymère non biologique, une molécule organométallique, une molécule organique de synthèse, ou une molécule minérale,

- (ii) capable d'être induit à émettre de façon répétée, une électrochimiluminescence, par exposition à une quantité d'énergie électrochimique provenant d'une source appropriée, efficace pour induire le réactif à émettre une électrochimiluminescence de façon répétée, et
- (iii) capable de concurrencer l'analyte intéressant, pour les sites de liaison sur le matériau complémentaire normalement non présent dans l'échantillon, le contact étant effectué dans des conditions appropriées, de façon à ce que l'analyte intéressant, et le conjugué se lient concurrentiellement au matériau complémentaire;

(b) l'exposition de l'échantillon obtenu à une quantité d'énergie électrochimique provenant d'une source appropriée, efficace pour induire le réactif à émettre une électrochimiluminescence de façon répétée, l'exposition étant effectuée dans des conditions adéquates, pour amener le réactif, à émettre une électrochimiluminescence de façon répétée; et

(c) la détection de la luminescence émise, pour déterminer si l'analyte intéressant est présent dans l'échantillon.

**42.** Méthode pour la détermination quantitative dans un volume prédéterminé d'un échantillon liquide à composants multiples, de la quantité présente dans l'échantillon, d'un analyte intéressant, qui, s'il est présent dans l'échantillon, est à une concentration inférieure à $10^{-3}$ molaire, ladite méthode comprenant :

(a) la mise en contact de l'échantillon avec une quantité connue d'un matériau complémentaire, et d'une quantité connue de réactif

- (i) comprenant un composé organique contenant un métal, conjugué à une cellule entière, une particule subcellulaire, un virus, un prion, un viroïde, un lipide, un acide gras, un acide nucléique, un polysaccharide autre qu'un sucre, une protéine, une lipoprotéine, un lipopolysaccharide, une glycoprotéine, un peptide, un métabolite cellulaire, une hormone, un agent pharmacologique, un tranquillisant, un barbirurate, un alcaloïde, un stéroïde, une vitamine, un acide aminé, un sucre, un polymère non biologique, une molécule organométallique, une molécule organique de synthèse, ou une molécule minérale,
- (ii) capable d'être induit à émettre de façon répétée, une électrochimiluminescence, par exposition à une quantité d'énergie électrochimique provenant d'une source appropriée, efficace pour induire le réactif, à émettre une électrochimiluminescence de façon répétée, et
- (iii) capable de concurrencer l'analyte intéressant, pour les sites de liaison sur le matériau complémentaire normalement non présent dans l'échantillon, le contact étant effectué dans des conditions appropriées, de façon à ce que l'analyte intéressant, et le conjugué se lient concurrentiellement au matériau complémentaire;

(b) l'exposition de l'échantillon obtenu à une quantité d'énergie électrochimique provenant d'une source appropriée, efficace pour induire le réactif, à émettre une électrochimiluminescence de façon répétée, l'exposition étant effectuée dans des conditions adéquates, pour amener le réactif à émettre une électrochimiluminescence de façon répétée; et

(c) la détermination quantitative de la quantité de luminescence émise, pour déterminer si l'analyte intéressant est présent dans l'échantillon, et en quelle quantité.

**43.** Méthode selon la revendication 41 ou la revendication 42, dans laquelle l'analyte intéressant est la théophylline, la digoxine, ou la gonadotrophine chorionique humaine.

**44.** Méthode selon l'une quelconque des revendications 41 à 43, dans laquelle le réactif est l'analyte intéressant conjugué à un composé organique contenant un métal.

**45.** Méthode selon l'une quelconque des revendications 41 à 43, dans laquelle le réactif est un analogue de l'analyte intéressant, conjugué à un composé organique contenant un métal.

**46.** Méthode selon l'une quelconque des revendications 41 à 45, dans laquelle le composé organique contenant un métal est choisi parmi ceux spécifiés dans l'une quelconque des revendications 13 à 35.

**47.** Méthode selon l'une quelconque des revendications 41 à 46, dans laquelle le matériau complémentaire est une cellule entière, une particule subcellulaire, un virus, un prion, un viroïde, un lipide, un acide gras,

un acide nucléique, un polysaccharide, une protéine, une lipoprotéine, un lipopolysaccharide, une glycoprotéine, un peptide, un métabolite cellulaire, une hormone, un agent pharmacologique, un tranquillisant, un barbirurate, un alcaloïde, un stéroïde, une vitamine, un acide aminé, un sucre, un polymère non biologique, une molécule organique de synthèse, une molécule organométallique, ou une molécule minérale.

Figure 1

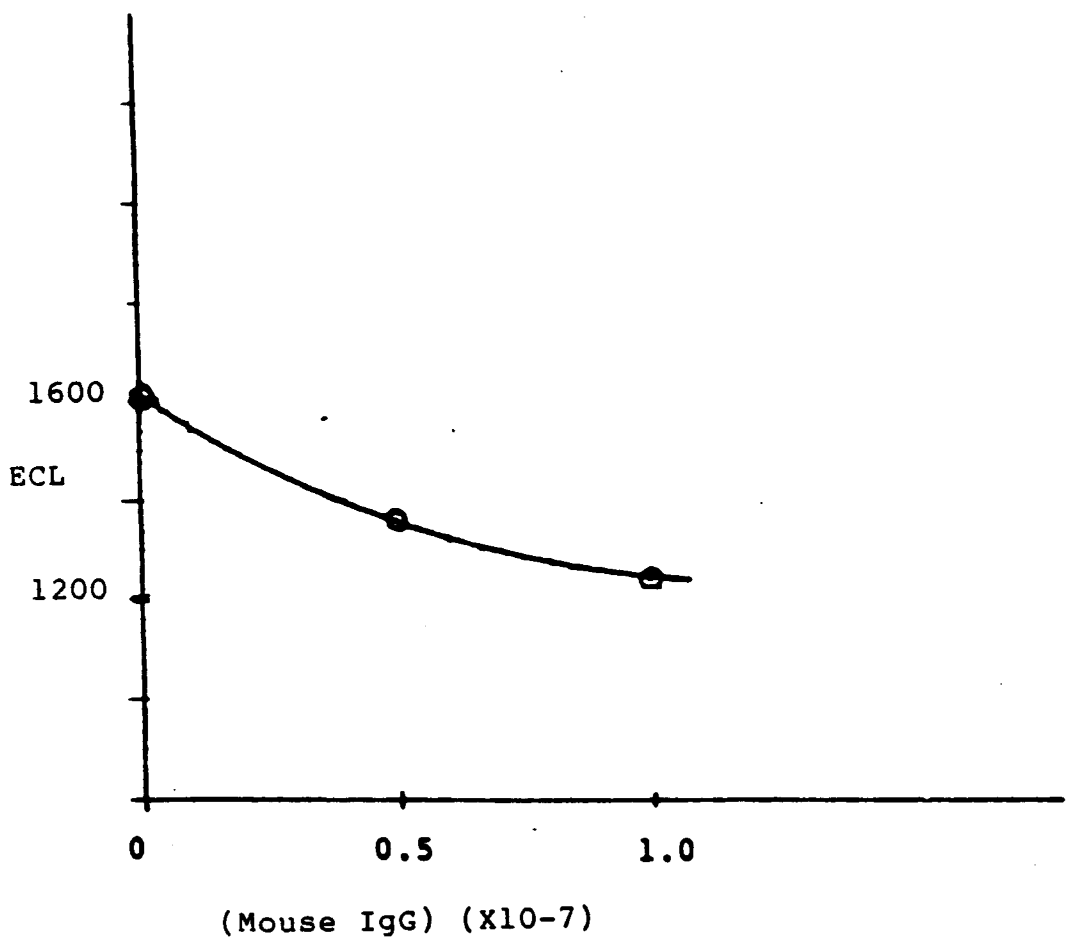

Figure 2

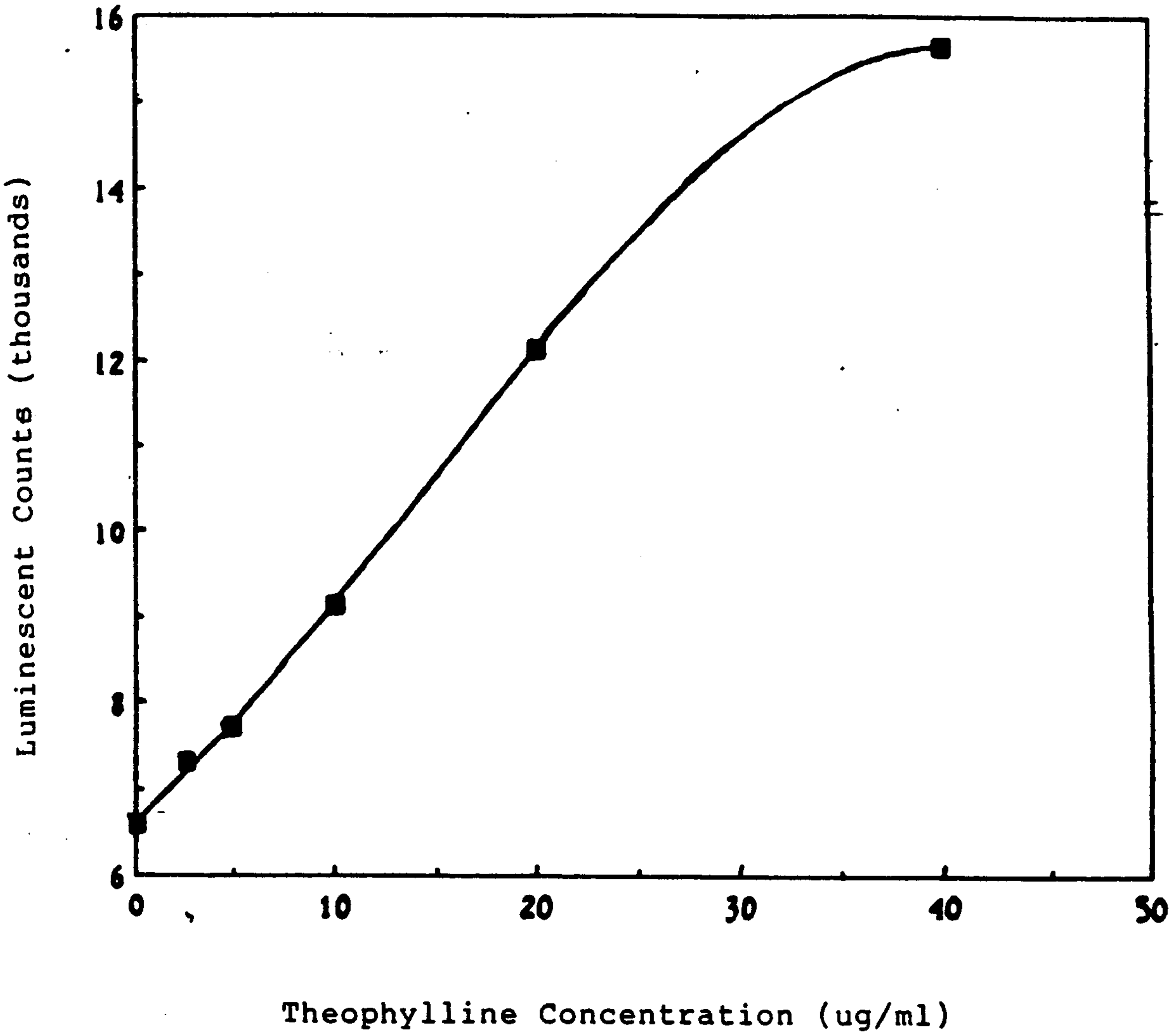
16
14
12
10
8
6
0
10
20
30
40
50
Luminescent Counts (thousands)
Theophylline Concentration (ug/ml)

Figure 3

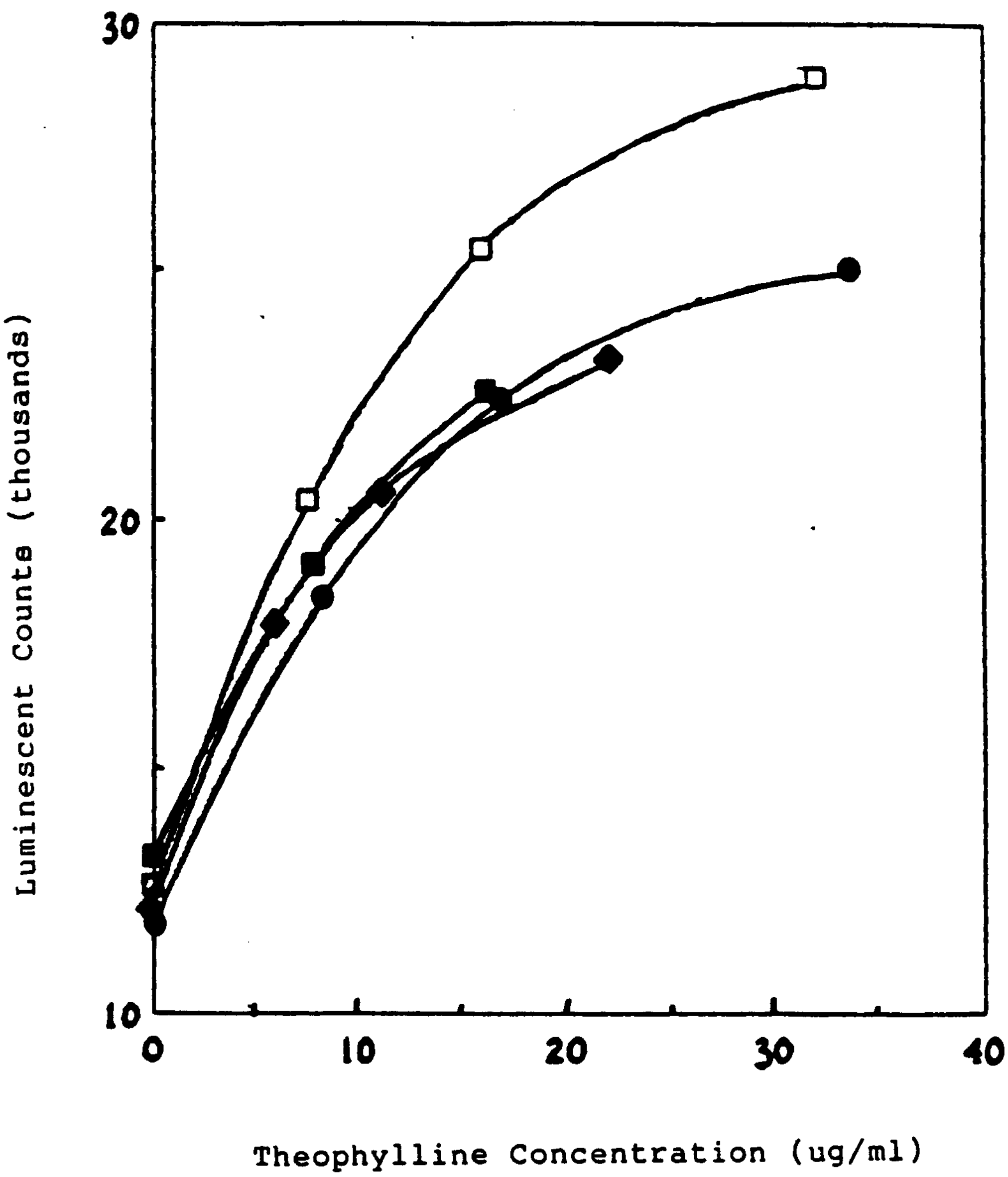
30
20
10
0
10
20
30
40
Luminescent Counts (thousands)
Theophylline Concentration (ug/ml)

Figure 4A

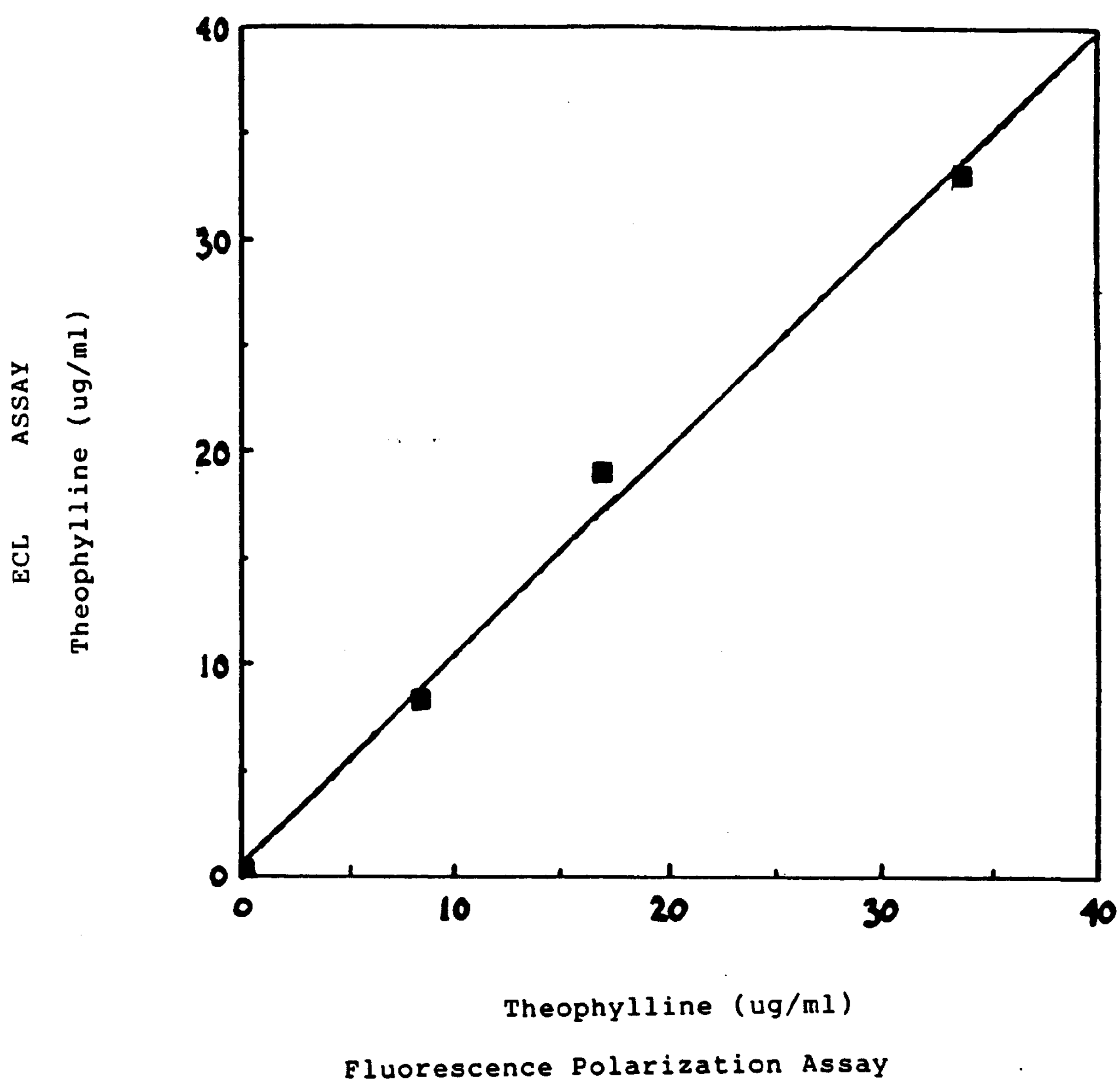
ECL ASSAY
Theophylline (ug/ml)
40
30
20
10
0
0
10
20
30
40
Theophylline (ug/ml)
Fluorescence Polarization Assay

Figure 4B

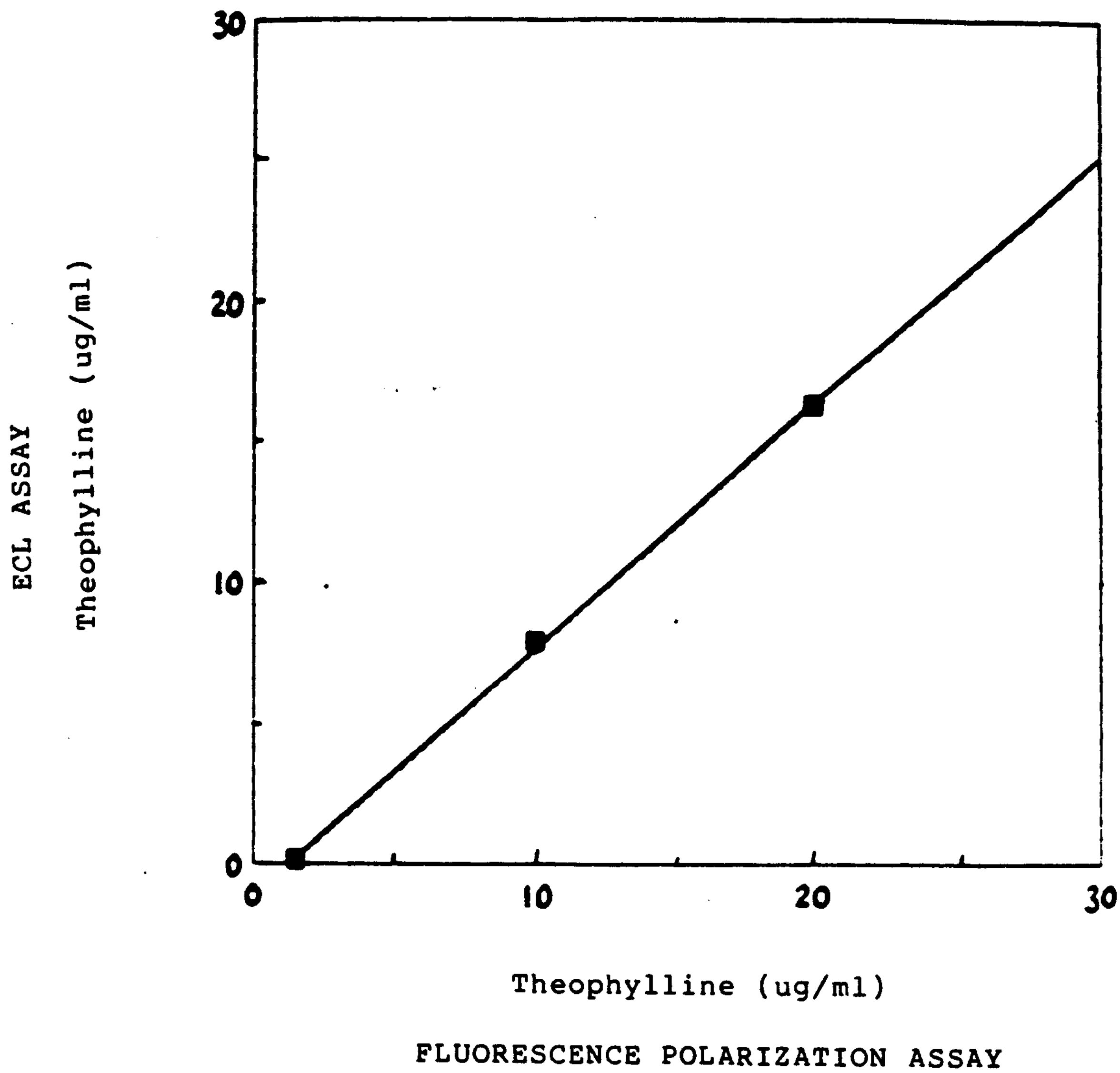
30
20
10
0
0
10
20
30
ECL ASSAY
Theophylline (ug/ml)
Theophylline (ug/ml)
FLUORESCENCE POLARIZATION ASSAY

Figure 4C

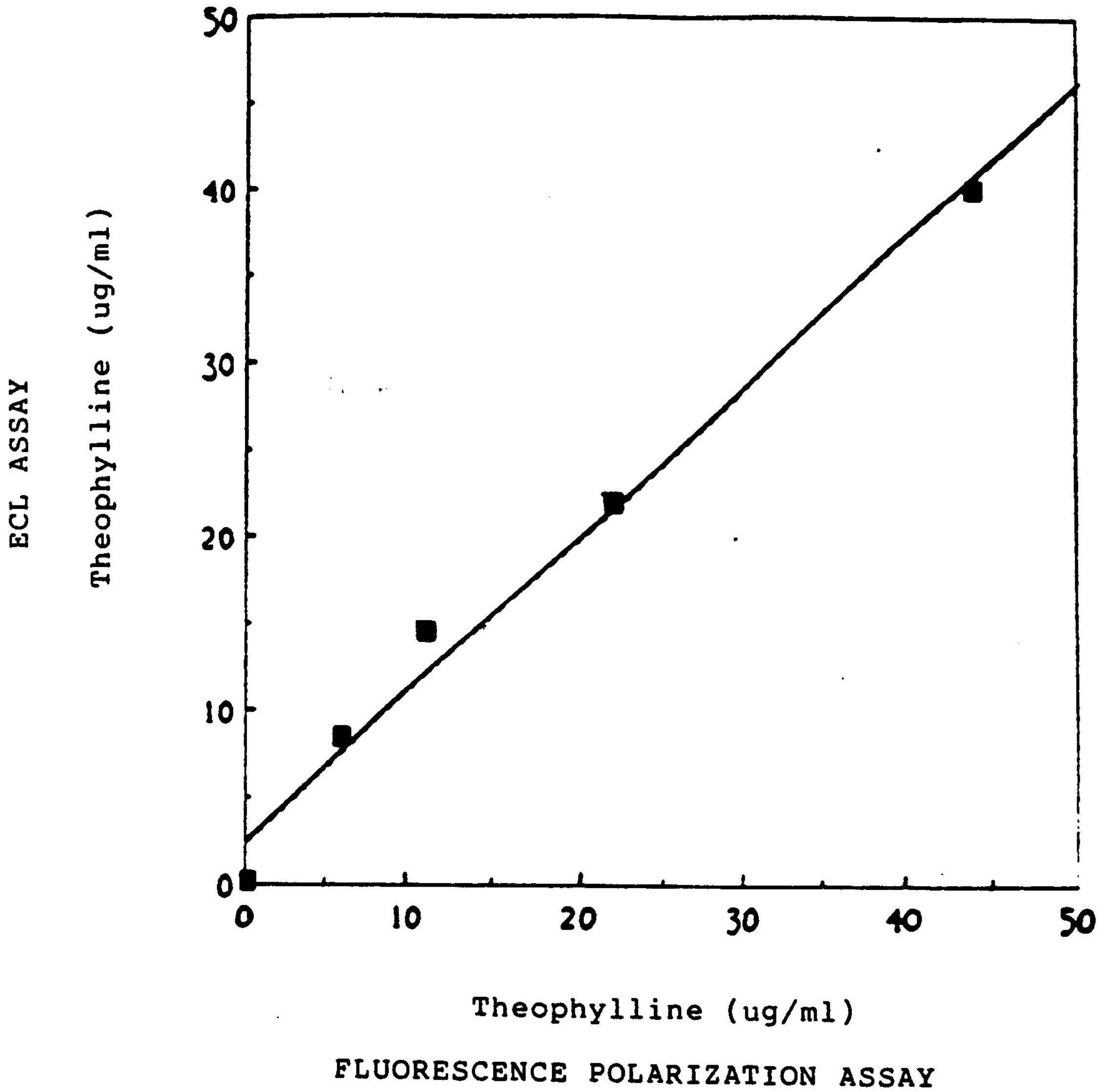
ECL ASSAY
Theophylline (ug/ml)
50
40
30
20
10
0
0
10
20
30
40
50
Theophylline (ug/ml)
FLUORESCENCE POLARIZATION ASSAY

Figure 4D

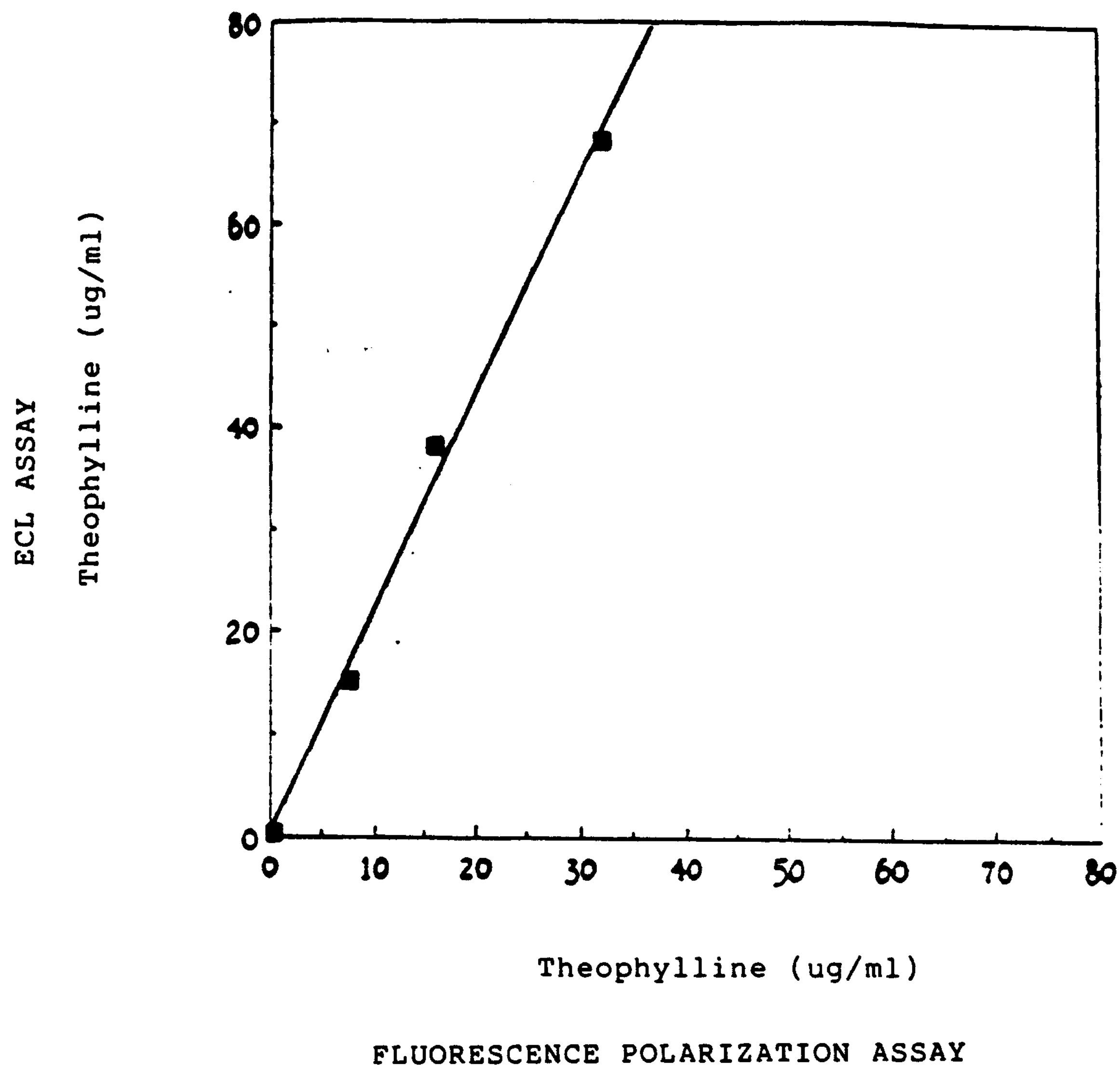
ECL ASSAY
Theophylline (ug/ml)
80
60
40
20
0
0
10
20
30
40
50
60
70
80
Theophylline (ug/ml)
FLUORESCENCE POLARIZATION ASSAY

Figure 5

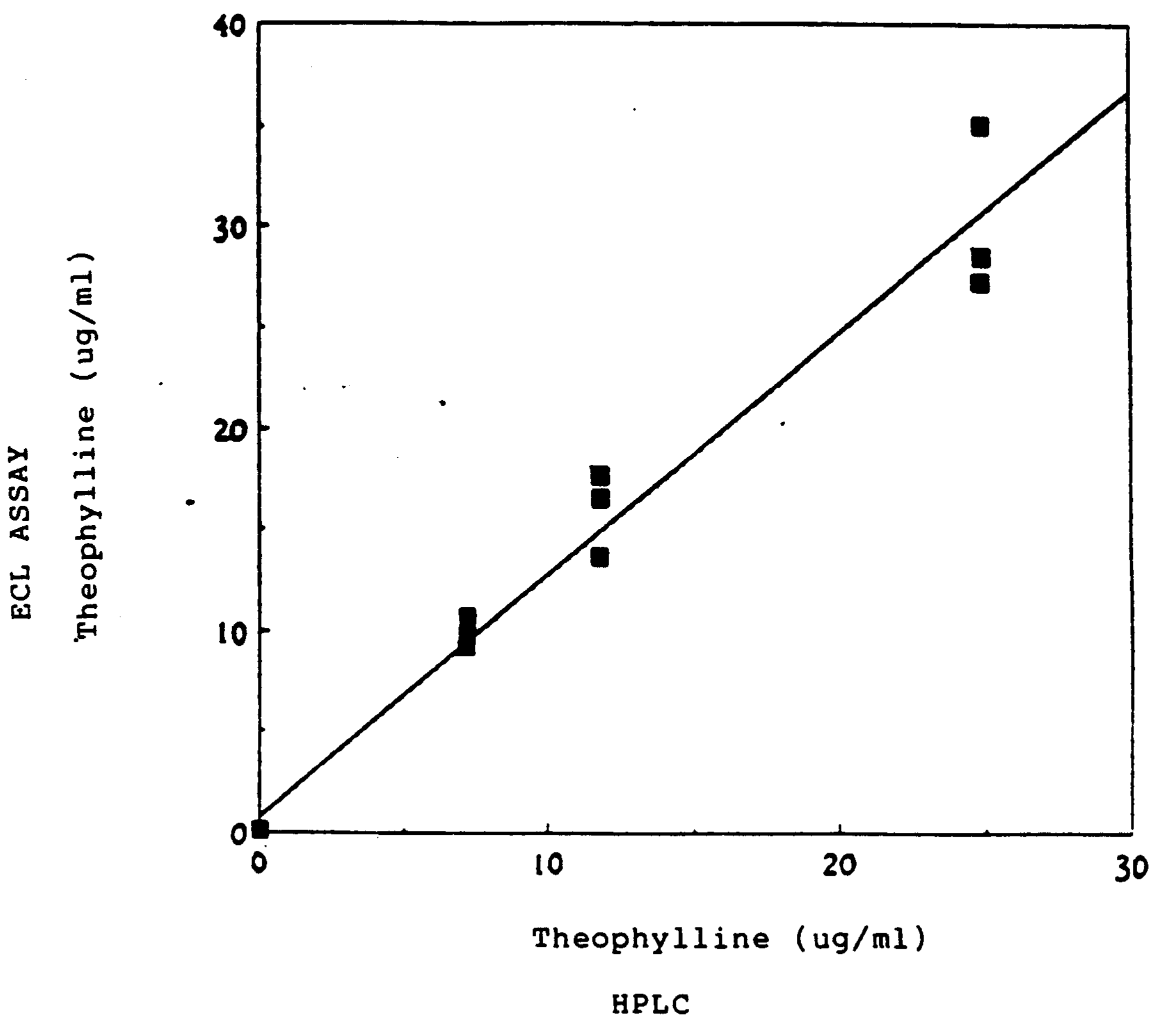
ECL ASSAY
Theophylline (ug/ml)
0
10
20
30
40
0
10
20
30
Theophylline (ug/ml)
HPLC

Figure 6

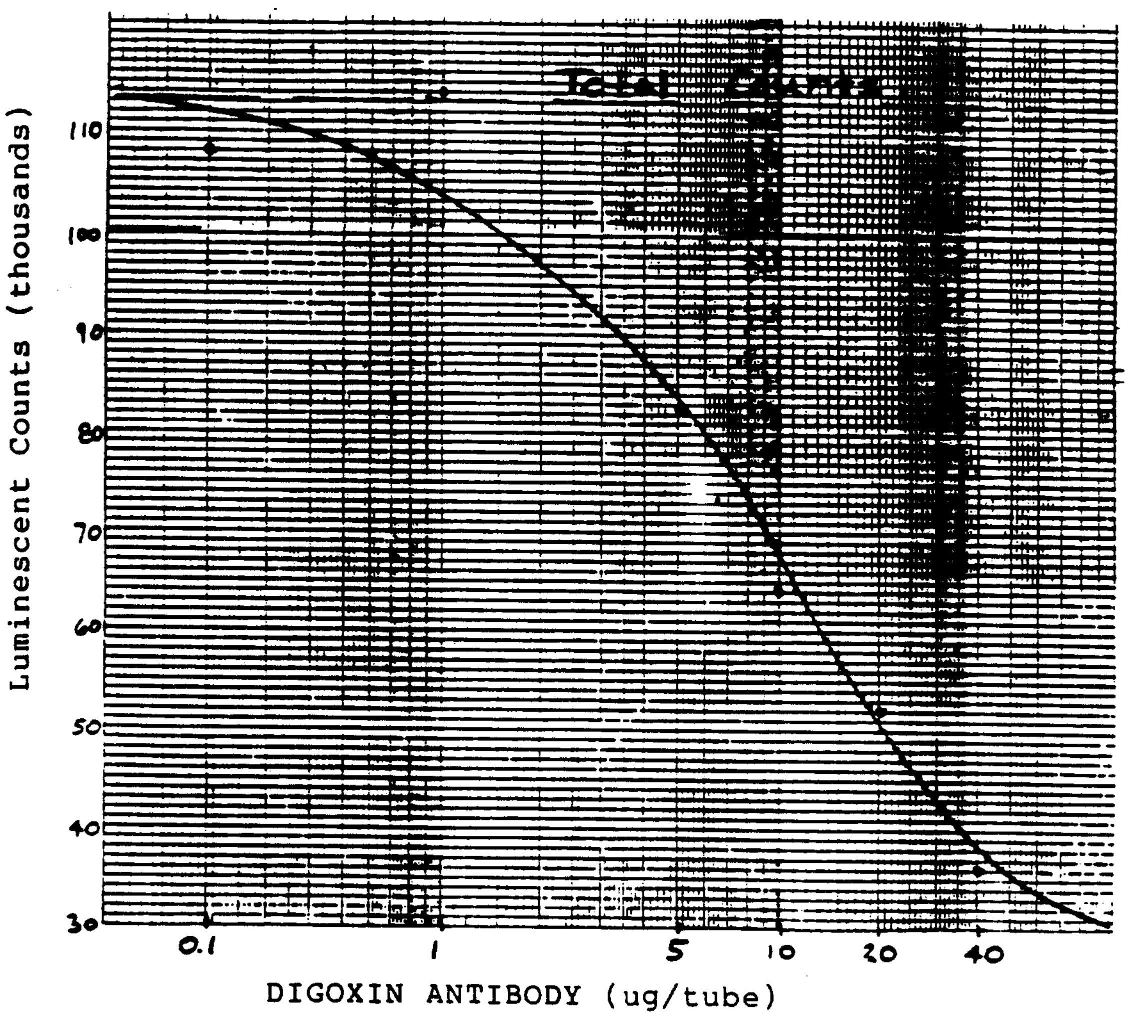
Total Counts
110
100
90
80
70
60
50
40
30
0.1
1
5
10
20
40
Luminescent Counts (thousands)
DIGOXIN ANTIBODY (ug/tube)

Figure 7

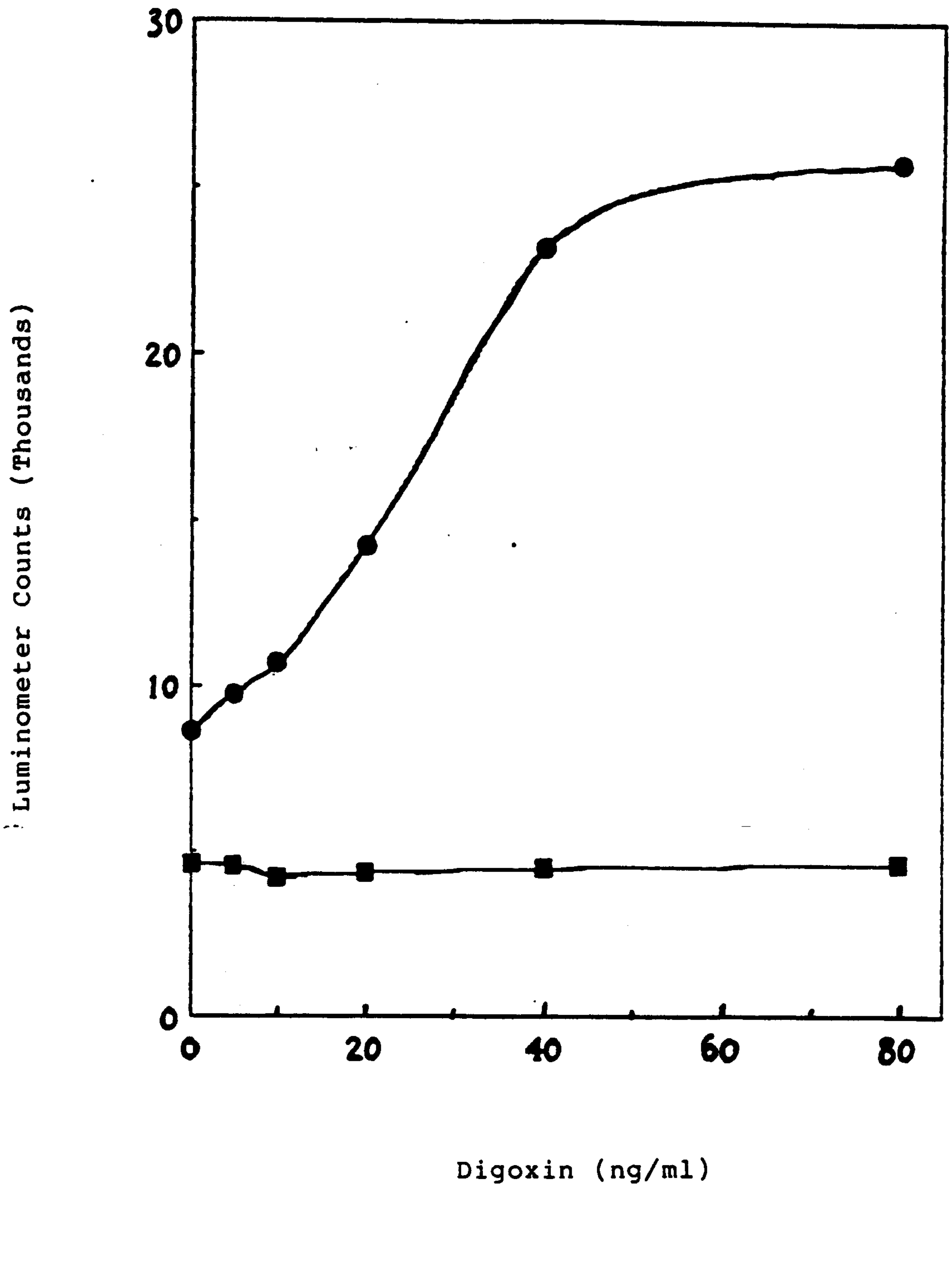
30
20
10
0
Luminometer Counts (Thousands)
0
20
40
60
80
Digoxin (ng/ml)

Figure 8

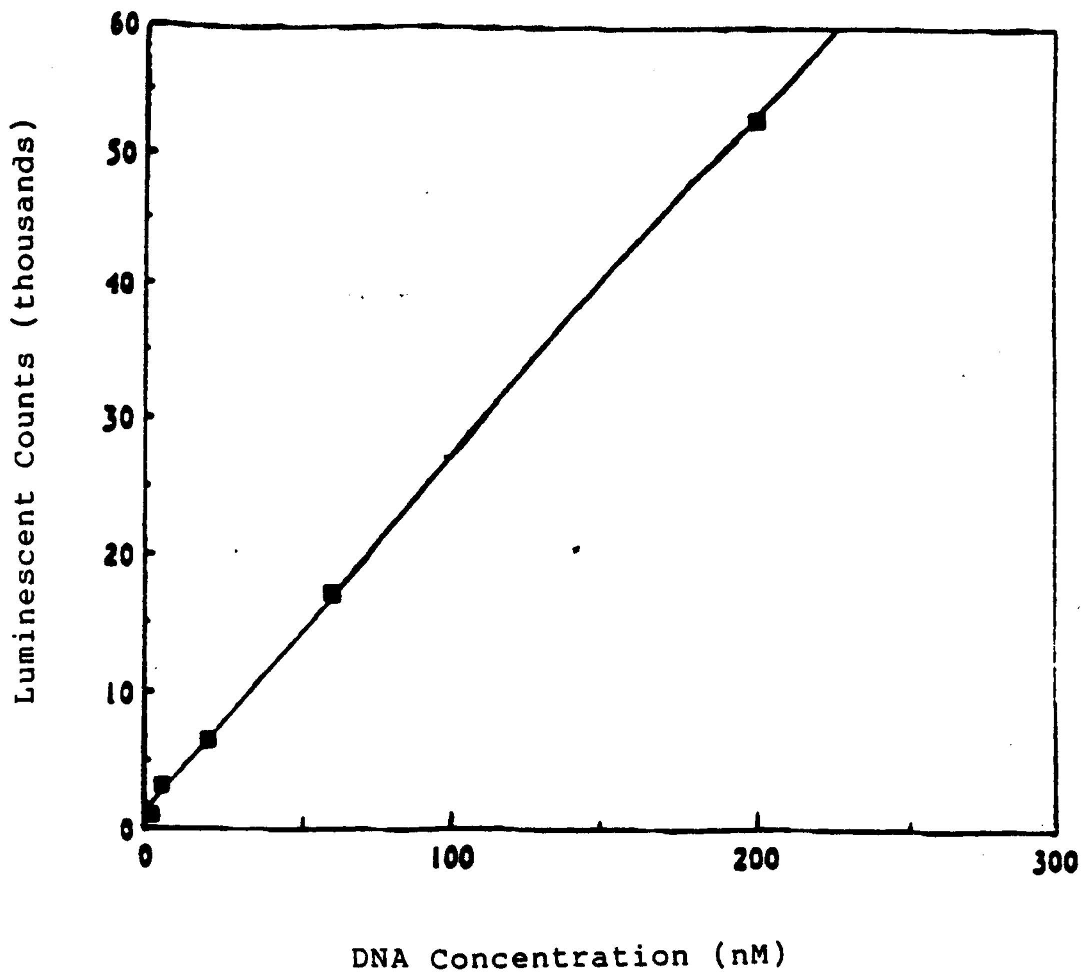
Luminescent Counts (thousands)
60
50
40
30
20
10
0
0
100
200
300
DNA Concentration (nM)

Fig.9.

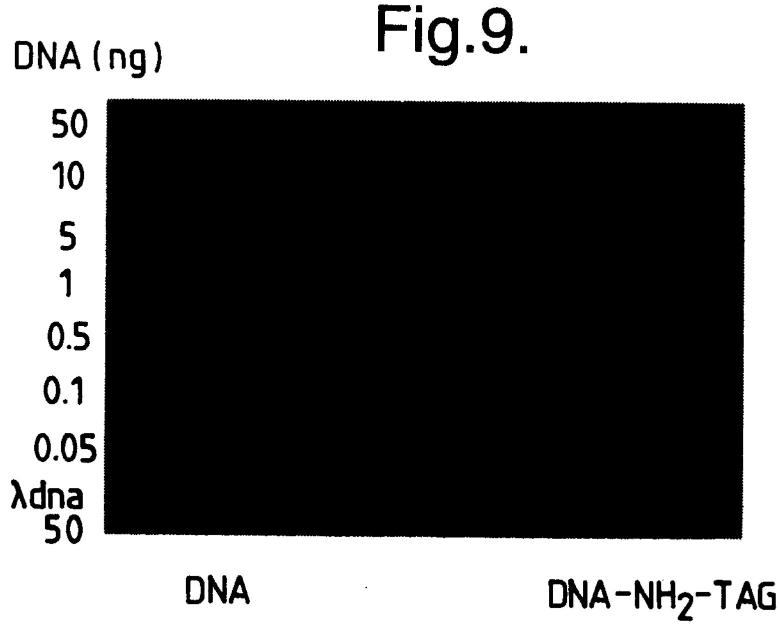
DNA (ng)
50
10
5
1
0.5
0.1
0.05
λdna
50
DNA
DNA-$NH_2$-TAG

Fig.10.

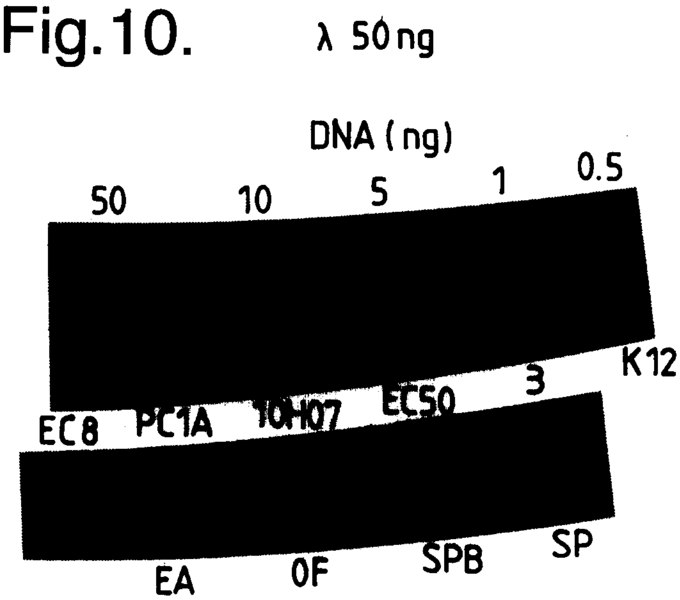
λ 50ng
DNA (ng)
50
10
5
1
0.5
EC8
PC1A
10H07
EC50
3
K12
EA
0F
SPB
SP